# EUROPEAN PATENT SPECIFICATION

(11) **EP 2 932 981 B1**
(45) Date of publication and mention of the grant of the patent: **16.06.2021**
(21) Application number: 15152442.8
(22) Date of filing: 17.09.2004
(51) Int. Cl.: A61K 47/54, C07K 14/605, A61P 3/10, A61P 1/04, A61P 1/14, A61P 3/04, A61P 3/06, A61P 9/10, A61P 9/12, A61P 25/00

(54) **Albumin-binding derivatives of GLP-1**
Albumin-bindende Derivative von GLP-1
Dérivés du GLP-1 se liant à l'albumine

(30) Priority: 19.09.2003 DK 200301367; 25.09.2003 US 505739 P; 04.12.2003 DK 200301789; 04.12.2003 US 526847 P
(43) Date of publication of application: 21.10.2015
(62) Divisional of application: 04762844.1
(73) Proprietor: NOVO NORDISK A/S, 2880 Bagsværd (DK)
(72) Inventor: Johansen, Nils Langeland, 2880 Bagsværd (DK)

(56) References cited:
- WO-A-01/51071
- WO-A-98/08872
- WO-A-99/43341
- WO-A-99/43361
- WO-A-99/43705
- WO-A-99/43707
- WO-A-03/002136
- WO-A-03/087139
- WO-A1-01/04156
- WO-A1-90/11296
- KNUDSEN LOTTE B ET AL: "Potent derivatives of glucagon-like peptide-1 with pharmacokinetic properties suitable for once daily administration", JOURNAL OF MEDICINAL CHEMISTRY, vol. 43, no. 9, 4 May 2000 (2000-05-04), pages 1664-1669, XP002201014, ISSN: 0022-2623
- OSTROVSKII YU M: "COMPARATIVE CHARACTERISTICS OF THE HYDROPHOBIC NATURE OF CERTAIN PROTEINS BY THEIR INTERACTION WITH 2-P TOLUIDINO NAPHTHALENE-6-SULFONATE", UKRAYINS'KYI BIOKHIMICHNYI ZHURNAL, vol. 47, no. 6, 1975, pages 701-707, XP008039733,
- PICO G: "USE OF 1 ANILINO-8-NAPHTHALENESULFONATE AS A REPORTER MOLECULE TO STUDY THE BILE SALTS BOVINE SERUM ALBUMIN BINDING", STUDIA BIOPHYSICA, vol. 136, no. 1, 1990, pages 21-26, XP008039734, ISSN: 0081-6337
- HOLZ G G ET AL: "GLUCAGON-LIKE PEPTIDE-1 SYNTHETIC ANALOGS: NEW THERAPEUTIC AGENTS FOR USE IN THE TREATMENT OF DIABETES MELLITUS", CURRENT MEDICINAL CHEMISTRY, vol. 10, no. 22, November 2003 (2003-11), pages 2471-2483, XP009035149, ISSN: 0929-8673
- KNUDSEN L B ET AL: "GLP-1 DERIVATIVES AS NOVEL COMPOUNDS FOR THE TREATMENT OF TYPE 2 DIABETES: SELECTION OF NN2211 FOR CLINICAL DEVELOPMENT", DRUGS OF THE FUTURE, vol. 26, no. 7, July 2001 (2001-07), pages 677-685, XP009071531, ISSN: 0377-8282, DOI: 10.1358/DOF.2001.026.07.628723

## Description

### FIELD OF THE INVENTION

The present invention relates to novel derivatives of glucagon-like-peptide-1 (GLP-1), fragments thereof and analogues of such fragments, which have a protracted profile of action, and methods of making and using them. Also disclosed are novel derivatives of exendin and the use of such derivatives.

### BACKGROUND OF THE INVENTION

Peptides are widely used in medical practice, and since they can be produced by recombinant DNA technology it can be expected that their importance will increase also in the years to come. When native peptides or analogues thereof are used in therapy it is generally found that they have a high clearance. A high clearance of a therapeutic agent is inconvenient in cases where it is desired to maintain a high blood level thereof over a prolonged period of time since repeated administrations will then be necessary. Examples of peptides which have a high clearance are: ACTH, corticotropin-releasing factor, angiotensin, calcitonin, insulin, glucagon, glucagon-like peptide-1, glucagon-like peptide-2, insulin-like growth factor-1, insulin-like growth factor-2, gastric inhibitory peptide, growth hormone-releasing factor, pituitary adenylate cyclase activating peptide, secretin, enterogastrin, somatostatin, somatotropin, somatomedin, parathyroid hormone, thrombopoietin, erythropoietin, hypothalamic releasing factors, prolactin, thyroid stimulating hormones, endorphins, enkephalins, vasopressin, oxytocin, opiods and analogues thereof, superoxide dismutase, interferon, asparaginase, arginase, arginine deaminase, adenosine deaminase and ribonuclease. In some cases it is possible to influence the release profile of peptides by applying suitable pharmaceutical compositions, but this approach has various shortcomings and is not generally applicable.

The number of known endogenous peptides and proteins with interesting biological activities is growing rapidly, also as a result of the ongoing exploration of the human genome. Due to their biological activities, many of these polypeptides could in principle be used as therapeutic agents. Endogenous peptides are, however, not always suitable as drug candidates because these peptides often have half-lives of few minutes due to rapid degradation by peptidases and/or due to renal filtration and excretion in the urine. The half-life of polypeptides in human plasma varies strongly (from a few minutes to more than one week). Similarly, the half-life of small molecule drugs is also highly variable. The reason for this strong variability of plasma half-lives of peptides, proteins, or other compounds is, however, not well understood. Thus, there is a need to modify therapeutic compounds to provide longer duration of action in vivo while maintaining low toxicity and therapeutic advantages. Serum albumin has a half-life of more than one week, and one approach to increasing the plasma half-life of peptides has been to derivatize the peptides with a chemical entity that binds to serum albumin.

Zobel et al. (Bioorg. Med. Chem. Lett. 2003, 13, 1513-1515) have shown that the plasma half-life of an anticoagulant peptide in rabbits increased by 10-50 fold on derivatization of the amino terminus with phosphate ester based small molecules binding to serum albumin.

WO 99/043341 A discloses GLP-1 derivatives that form partially helical, micelle-like aggregates in water. D1 does not disclose a GLP-1 derivative having the claimed spacer-linker structure, WBY, and does not disclose GLP-1 derivatives that bind to albumin in any way.

WO 99/043707 A discloses N-terminally modified GLP-1 derivatives that may contain a spacer but not a spacer-linker structure as disclosed herein.

WO 99/043705 A discloses N-terminally truncated GLP-1 derivatives that may contain a spacer but not a spacer-linker structure as disclosed herein.

WO 98/008872 A relates to GLP-2 derivatives, not GLP-1 derivatives as disclosed herein.

Diabetes 2003, vol. 52, p. 751-759 discloses CJC-1131, a DPP-IV resistant drug affinity complex (DAC) GLP-1 compound having a reactive chemical linker that binds covalently to human albumin.

Knudsen et al. (J. Med. Chem. 2000, 43, 1664-1669) discloses several specific GLP-1 compounds that are structurally close to the alkyl carboxylic acid derivatives of the present invention. These derivatives may contain a spacer but not the spacer-linker structure as disclosed herein.

WO 01/051071 A discloses GLP-1 derivatives that are suitable for pulmonary delivery. The derivatives may contain a spacer but not the spacer-linker structure as disclosed herein.

WO 01/004156 A relates to peptide conjugates that lower blood glucose levels, such as GLP-1 derivatives with a lipophilic substituent. However, these GLP-1 derivatives do not contain a spacer-linker structure as disclosed herein.

WO 03/002136 A relates to stable formulations of modified GLP-1. The derivatives do not contain a spacer-linker structure as disclosed herein.

Deacon et al. (Diabetologia 1998, 41, 271-278) discloses DPP-IV resistant analogues of GLP-1. The derivatives do not contain a spacer-linker structure as disclosed herein.

Sheffield (Current Drug Targets - Cardiovascular and Haematological Disorders 2001, 1(1), 1-22) is a general review article relating to retardation of the clearance and catabolism of injected therapeutic peptides.

Ostrovskii (Ukrayins' KYI Biokhimichnyi Zhurnal 1975, 47(6), 701-707) reported studies of the hydrophobicity of various proteins. GLP-1 derivatives as disclosed herein are not disclosed.

Pico (Study Biophysica 1990, 136, 21-26) reported studies of the binding of bile salts to albumin using 1-anilino-8-naphthalene sulfonate as a reporter molecule. GLP-1 derivatives as disclosed herein are not disclosed.

Kurtzhals et al. (Biochem. Jour. 1995, 32, 725-731) relates to insulin derivatives.

### SUMMARY OF THE INVENTION

Disclosed herein is a compound which comprises a therapeutic polypeptide linked to an albumin binding residue via a hydrophilic spacer.

The present invention relates to a compound of formula (I):

A-W-B-Y-therapeutic polypeptide (I)

wherein
said polypeptide is a GLP-1 peptide comprising the amino acid sequence of formula (V):
   Xaa₇-Xaa₈-Glu-Gly-Thr-Phe-Thr-Ser-Asp-Val-Ser-Xaa₁₈-Tyr-Leu-Glu-Xaa₂₂-Xaa₂₃-Ala-Ala-Xaa₂₆-Glu-Phe-Ile-Xaa₃₀-Trp-Leu-Val-Xaa₃₄-Xaa₃₅-Xaa₃₆-Xaa₃₇-Xaa₃₈
   Formula (V) (SEQ ID No: 3)
   wherein
   Xaa₇ is L-histidine, D-histidine, desamino-histidine, 2-amino-histidine, β-hydroxy-histidine, homohistidine, N^{α}-acetyl-histidine, α-fluoromethyl-histidine, α-methyl-histidine, 3-pyridylalanine, 2-pyridylalanine or 4-pyridylalanine;
      Xaa₈ is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid;
      Xaa₁₈ is Ser, Lys or Arg;
      Xaa₂₂ is Gly, Glu or Aib;
      Xaa₂₃ is Gin, Glu, Lys or Arg;
      Xaa₂₆ is Lys, Glu or Arg;
      Xaa₃₀ is Ala, Glu or Arg;
      Xaa₃₄ is Lys, Glu or Arg;
      Xaa₃₅ is Gly or Aib;
      Xaa₃₆ is Arg or Lys;
      Xaa₃₇ is Gly, Ala, Glu or Lys;
      Xaa₃₈ is Lys, amide or is absent;
   wherein A is an albumin binding residue selected from the group consisting of 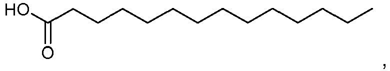 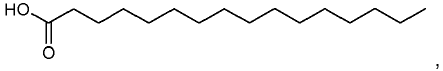 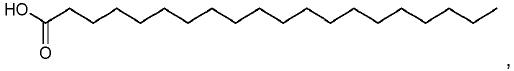 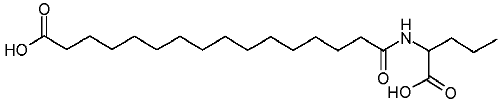 wherein the chiral carbon atom is either R or S, 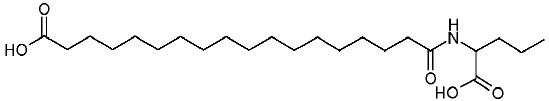 wherein the chiral carbon atom is either R or S, 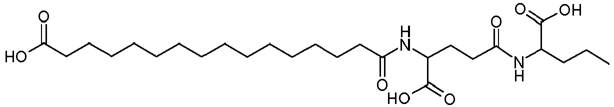 wherein the two chiral carbon atoms independently are either R or S, 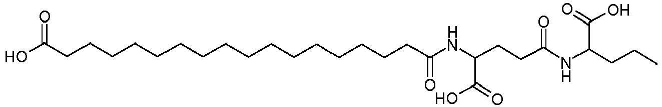 wherein the two chiral carbon atoms independently are either R or S, 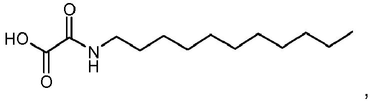 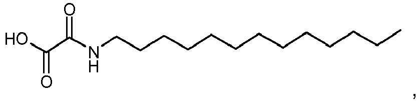 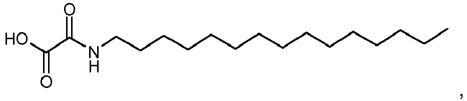 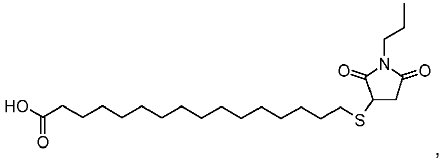 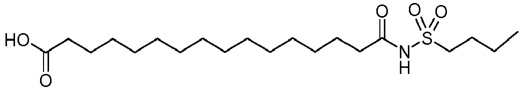 , and 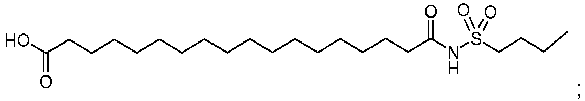
   wherein B is -(CH₂)ₗ-O-[(CH₂)ₙ-O]ₘ-(CH₂)ₚ-[C(O)NH-(CH₂)ₗ-O-[(CH₂)ₙ-O]ₘ-(CH₂)ₚ]_{q}-, where I, m, n, and p independently are 1-5, and q is 0-5;
   wherein Y is a chemical group linking B and the therapeutic agent, selected from the group consisting of -C(O)NH-, -NHC(O)-, -C(O)NHCH₂-, -CH₂NHC(O)-, -OC(O)NH -, -NHC(O)O-, -C(O)NHCH₂-, CH₂NHC(O)-, -C(O)CH₂-, -CH₂C(O)-, -C(O)CH=CH-, -CH=CHC(O)-, -(CH₂)ₛ-, -C(O)-, -C(O)O-, -OC(O)-, -NHC(O)- and -C(O)NH-, wherein s is 0 or 1;
      and
   wherein W is a chemical group linking A and B, selected from the group consisting of - C(O)NH-, -NHC(O)-, -C(O)NHCH₂-, -CH₂NHC(O)-, -OC(O)NH -, -NHC(O)O-, -C(O)CH₂-, - CH₂C(O)-, -C(O)CH=CH-, -CH=CHC(O)-, -(CH₂)ₛ-, -C(O)-, -C(O)O-, -OC(O)-, -NHC(O)- and -C(O)NH-, wherein s is 0 or 1;
   or a pharmaceutically acceptable salt thereof.

The present invention also relates to a pharmaceutical composition comprising the above-mentioned compound and a pharmaceutically acceptable excipient.

The present invention also relates to the above-mentioned compound for use as a medicament. Specifically, the present invention relates to use of the above-mentioned compound for the preparation of a medicament; preferably i) for the treatment or prevention of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, stroke, inflammatory bowel syndrome, dyspepsia and gastric ulcers; ii) for the preparation of a medicament for delaying or preventing disease progression in type 2 diabetes; and/or iii) for the preparation of a medicament for decreasing food intake, decreasing 3-cell apoptosis, increasing β-cell funtion and β-cell mass, and/or for restoring glucose sensitivity to β-cells.

### DEFINITIONS

In the present specification, the following terms have the indicated meaning:
The term "albumin binding residue" as used herein means a residue which binds non-covalently to human serum albumin. The albumin binding residue attached to the therapeutic polypeptide typically has an affinity below 10 µM to human serum albumin and preferably below 1 µM. A range of albumin binding residues are known among linear and branched lipohophillic moieties containing 4-40 carbon atoms, compounds with a cyclopentanophenanthrene skeleton, peptides having 10-30 amino acid residues etc.

The term "hydrophilic spacer" as used herein means a spacer that separates a peptide and an albumin binding residue with a chemical moiety which comprises at least 5 non-hydrogen atoms where 30-50% of these are either N or O.

The term "therapeutic polypeptide" as used herein means a polypeptide which is being developed for therapeutic use, or which has been developed for therapeutic use.

The term "polypeptide" and "peptide" as used herein means a compound composed of at least five constituent amino acids connected by peptide bonds. The constituent amino acids may be from the group of the amino acids encoded by the genetic code and they may be natural amino acids which are not encoded by the genetic code, as well as synthetic amino acids. Natural amino acids which are not encoded by the genetic code are e.g. hydroxyproline, γ-carboxyglutamate, ornithine, phosphoserine, D-alanine and D-glutamine. Synthetic amino acids comprise amino acids manufactured by chemical synthesis, i.e. D-isomers of the amino acids encoded by the genetic code such as D-alanine and D-leucine, Aib (a-aminoisobutyric acid), Abu (a-aminobutyric acid), Tle (tert-butylglycine), β-alanine, 3-aminomethyl benzoic acid, anthranilic acid.

The term "analogue" as used herein referring to a polypeptide means a modified peptide wherein one or more amino acid residues of the peptide have been substituted by other amino acid residues and/or wherein one or more amino acid residues have been deleted from the peptide and/or wherein one or more amino acid residues have been deleted from the peptide and or wherein one or more amino acid residues have been added to the peptide. Such addition or deletion of amino acid residues can take place at the N-terminal of the peptide and/or at the C-terminal of the peptide. A simple system is used to describe analogues : For example [Arg³⁴]GLP-1(7-37)Lys designates a GLP-1 analogue wherein the naturally occuring lysine at position 34 has been substituted with arginine and a lysine residue has been added to the C-terminal (position 38). Formulae of peptide analogs and derivatives thereof are drawn using standard single letter abbreviation for amino acids used according to IUPAC-IUB nomenclature.

The term "derivative" as used herein in relation to a peptide means a chemically modified peptide or an analogue thereof, wherein at least one substituent is not present in the unmodified peptide or an analogue thereof, i.e. a peptide which has been covalently modified. Typical modifications are amides, carbohydrates, alkyl groups, acyl groups, esters and the like. An example of a derivative of GLP-1(7-37) is N^{ε26}-(γ-Glu(N^{α}-hexadecanoyl)))-[Arg³⁴, Lys²⁶])GLP-1 (7-37).

The term "GLP-1 peptide" as used herein means GLP-1(7-37) (SEQ ID No. 1), a GLP-1 analogue, a GLP-1 derivative or a derivative of a GLP-1 analogue. In one embodiment the GLP-1 peptide is an insulinotropic agent.

The term "insulinotropic agent" as used herein means a compound which is an agonist of the human GLP-1 receptor, i.e. a compound which stimulates the formation of cAMP in a suitable medium containing the human GLP-1 receptor. The potency of an insulinotropic agent is determined by calculating the EC₅₀ value from the dose-response curve as described below.

Purified plasma membranes from a stable transfected cell line, BHK467-12A (tk-ts13), expressing the human GLP-1 receptor was stimulated with GLP-1 and peptide analogues, and the potency of cAMP production was measured using the AlphaScreen™ cAMP Assay Kit from Perkin Elmer Life Sciences.

A stable transfected cell line has been prepared at NN and a high expressing clone was selected for screening. The cells were grown at 5% CO₂ in DMEM, 5% FCS, 1% Pen/Strep and 0.5 mg/ml G418.

Cells at approximate 80% confluence were washed 2X with PBS and harvested with Versene, centrifuged 5 min at 1000 rpm and the supernatant removed. The additional steps were all made on ice. The cell pellet was homogenized by the Ultrathurax for 20-30 sec. in 10 ml of Buffer 1 (20 mM Na-HEPES, 10 mM EDTA, pH=7.4), centrifuged 15 min at 20.000 rpm and the pellet resuspended in 10 ml of Buffer 2 (20 mM Na-HEPES, 0.1 mM EDTA, pH=7.4). The suspension was homogenized for 20-30 sec and centrifuged 15 min at 20.000 rpm. Suspension in Buffer 2, homogenization and centrifugation was repeated once and the membranes were resuspended in Buffer 2 and ready for further analysis or stored at -80°C.
The functional receptor assay was carried out by measurering the peptide induced cAMP production by The AlphaScreen Technology. The basic principle of The AlphaScreen Technology is a competition between endogenous cAMP and exogenously added biotin-cAMP. The capture of cAMP is achieved by using a specific antibody conjugated to acceptor beads. Formed cAMP was counted and measured at a AlphaFusion Microplate Analyzer. The EC₅₀ values was calculated using the Graph-Pad Prisme software.

The term "GLP-2 peptide" as used herein means GLP-2(1-33), a GLP-2 analogue, a GLP-2 derivative or a derivative of a GLP-2 analogue.
The term "exendin-4 peptide" as used herein means exendin-4(1-39), an exendin-4 analogue, an exendin-4 derivative or a derivative of an exendin-4 analogue. In one embodiment the exendin-4 peptide is an insulinotropic agent.

The terms "stable exendin-4 peptide" and "stable GLP-1 peptides" as used herein means chemically modified peptides derived from exendin-4(1-39) or GLP-1(7-37), i.e. an analogue or a derivative which exhibits an in vivo plasma elimination half-life of at least 10 hours in man, as determined by the following method. The method for determination of plasma elimination half-life of an exendin-4 peptide or a GLP-1 peptide in man is : The peptide is dissolved in an isotonic buffer, pH 7.4, PBS or any other suitable buffer. The dose is injected peripherally, preferably in the abdominal or upper thigh. Blood samples for determination of active peptide are taken at frequent intervals, and for a sufficient duration to cover the terminal elimination part (e.g. Pre-dose, 1, 2, 3, 4, 5, 6, 7, 8, 10, 12, 24 (day 2), 36 (day 2), 48 (day 3), 60 (day 3), 72 (day 4) and 84 (day 4) hours post dose). Determination of the concentration of active peptide is performed as described in Wilken et al., Diabetologia 43(51):A143, 2000. Derived pharmacokinetic parameteres are calculated from the concentration-time data for each individual subject by use of non-compartmental methods, using the commercially available software WinNonlin Version 2.1 (Pharsight, Cary, NC, USA). The terminal elimination rate constant is estimated by log-linear regression on the terminal log-linear part of the concentration-time curve, and used for calculating the elimination half-life.

The term "DPP-IV protected" as used herein referring to a polypeptide means a polypeptide which has been chemically modified in order to render said compound resistant to the plasma peptidase dipeptidyl aminopeptidase-4 (DPP-IV). The DPP-IV enzyme in plasma is known to be involved in the degradation of several peptide hormones, e.g. GLP-1, GLP-2, Exendin-4 etc. Thus, a considerable effort is being made to develop analogues and derivatives of the polypeptides susceptible to DPP-IV mediated hydrolysis in order to reduce the rate of degradation by DPP-IV.

Resistance of a peptide to degradation by dipeptidyl aminopeptidase IV is determined by the following degradation assay :
Aliquots of the peptides are incubated at 37 °C with an aliquot of purified dipeptidyl aminopeptidase IV for 4-22 hours in an appropriate buffer at pH 7-8 (buffer not being albumin). Enzymatic reactions are terminated by the addition of trifluoroacetic acid, and the peptide degradation products are separated and quantified using HPLC or LC-MS analysis. One method for performing this analysis is: The mixtures are applied onto a Zorbax 300SB-C18 (30 nm pores, 5 µm particles) 150 x 2.1 mm column and eluted at a flow rate of 0.5 ml/min with a linear gradient of acetonitrile in 0.1% trifluoroacetic acid (0% -100% acetonitrile over 30 min). Peptides and their degradation products may be monitored by their absorbance at 214 nm (peptide bonds) or 280 nm (aromatic amino acids), and are quantified by integration of their peak areas. The degradation pattern can be determined by using LC-MS where MS spectra of the separated peak can be determined. Percentage intact/degraded compound at a given time is used for estimation of the peptides DPPIV stability.
A peptide is defined as DPPIV stabilised when it is 10 times more stable than the natural peptide based on percentage intact compound at a given time. Thus, a DPPIV stabilised GLP-1 compound is at least 10 times more stable than GLP-1(7-37).

The term "C₁₋₆-alkyl" as used herein means a saturated, branched, straight or cyclic hydrocarbon group having from 1 to 6 carbon atoms. Representative examples include, but are not limited to, methyl, ethyl, n-propyl, isopropyl, butyl, isobutyl, sec-butyl, *tert*-butyl, n-pentyl, isopentyl, neopentyl, *tert*-pentyl, n-hexyl, isohexyl, cyclohexane and the like
The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

### DETAILED DESCRIPTION OF THE INVENTION

Disclosed herein is a compound which comprises a therapeutic polypeptide linked to an albumin binding residue via a hydrophilic spacer.

Disclosed herein is a compound which comprises a therapeutic polypeptide linked to an albumin binding residue via a hydrophilic spacer that separates the polypeptide and the albumin binding residue with a chemical moiety comprising at least 5 non-hydrogen atoms where 30-50% of these atoms are either N or O.
In one embodiment, the spacer is defined as

-(CH₂)ₗD[(CH₂)ₙE]ₘ(CH₂)ₚQ_{q}-,

wherein
I, m and n independently are 1-20 and p is 0-10,
Q is -Z-(CH₂)ₗD[(CH₂)ₙG]ₘ(CH₂)ₚ-,
q is an integer in the range from 0 to 5,
each D, E, and G independently are selected from -O-, -NR³-, -N(COR⁴)-, -PR⁵(O)-, and -P(OR⁶)(O)-, wherein R³, R⁴, R⁵, and R⁶ independently represent hydrogen or C₁₋₆-alkyl,
Z is selected from -C(O)NH-, -C(O)NHCH₂-, -OC(O)NH -, -C(O)NHCH₂CH₂-, -C(O)CH₂-, -C(O)CH=CH-, -(CH₂)ₛ-, -C(O)-, -C(O)O- or -NHC(O)-, wherein s is 0 or 1
or a pharmaceutically acceptable salt or prodrug thereof.

The present invention relates to a compound which has the formula (I) :

A-W-B-Y-therapeutic polypeptide (I)

wherein
A is an albumin binding residue,
B is a hydrophilic spacer being -(CH₂)ₗD[(CH₂)ₙE]ₘ(CH₂)ₚQ_{q}-, wherein
   I, m and n independently are 1-20 and p is 0-10,
   Q is -Z-(CH₂)ₗD[(CH₂)ₙG]ₘ(CH₂)ₚ-,
   q is an integer in the range from 0 to 5,
   each D, E, and G independently are selected from -O-, -NR³-, -N(COR⁴)-, -PR⁵(O)-, and -P(OR⁶)(O)-, wherein R³, R⁴, R⁵, and R⁶ independently represent hydrogen or C₁₋₆-alkyl,
   Z is selected from -C(O)NH-, -C(O)NHCH₂-, -OC(O)NH -, -C(O)NHCH₂CH₂-, -C(O)CH₂-, -C(O)CH=CH-, -(CH₂)ₛ-, -C(O)-, -C(O)O- or -NHC(O)-, wherein s is 0 or 1,
Y is a chemical group linking B and the therapeutic agent, and
W is a chemical group linking A and B.

Also disclosed herein is a compound which has the formula (II)

A-W-B-Y-therapeutic polypeptide -Y'-B'-W'-A' (II)

wherein
A and A' are albumin binding residues,
B and B' are hydrophilic spacers independently selected from -(CH₂)ₗD [(CH₂)ₙE]ₘ(CH₂)ₚ-Q_{q}-, wherein
   I, m and n independently are 1-20 and p is 0-10,
   Q is -Z-(CH₂)ₗD[(CH₂)ₙG]ₘ(CH₂)ₚ-,
   q is an integer in the range from 0 to 5,
   each D, E, and G independently are selected from -O-, -NR³-, -N(COR⁴)-, -PR⁵(O)-, and -P(OR⁶)(O)-, wherein R³, R⁴, R⁵, and R⁶ independently represent hydrogen or C₁₋₆-alkyl,
   Z is selected from -C(O)NH-, -C(O)NHCH₂-, -OC(O)NH -, -C(O)NHCH₂CH₂-, -C(O)CH₂-, -C(O)CH=CH-, -(CH₂)ₛ-, -C(O)-, -C(O)O- or -NHC(O)-, wherein s is 0 or 1,
Y is a chemical group linking B and the therapeutic agent, and
Y' is a chemical group linking B' and the therapeutic agent, and
W is a chemical group linking A and B, and
W' is a chemical group linking A' and B'.

In one embodiment, Y' is selected from the group consisting of -C(O)NH-, -NHC(O)-, - C(O)NHCH₂-, -CH₂NHC(O)-, -OC(O)NH -, -NHC(O)O-, -C(O)NHCH₂-, CH₂NHC(O)-, -C(O)CH₂-, -CH₂C(O)-, -C(O)CH=CH-, -CH=CHC(O)-, -(CH₂)ₛ-, -C(O)-, -C(O)O-, -OC(O)-, -NHC(O)- and -C(O)NH-, wherein s is 0 or 1.

In a further embodiment, W' is selected from the group consisting of -C(O)NH-, -NHC(O)-, - C(O)NHCH₂-, -CH₂NHC(O)-, -OC(O)NH -, -NHC(O)O-, -C(O)CH₂-, -CH₂C(O)-, -C(O)CH=CH-, -CH=CHC(O)-, -(CH₂)ₛ-, -C(O)-, -C(O)O-, -OC(O)-, -NHC(O)- and -C(O)NH-, wherein s is 0 or 1.

Also disclosed herein is a compound which has the formula (III) 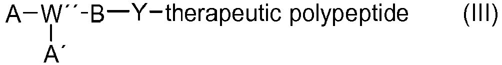 wherein
A and A' are albumin binding residues,
B is a hydrophilic spacer selected from -(CH₂)ₗD[(CH₂)ₙE]ₘ(CH₂)ₚ-Q_{q}- wherein
   I, m and n independently are 1-20 and p is 0-10,
   Q is -Z-(CH₂)ₗD[(CH₂)ₙG]ₘ(CH₂)ₚ-,
   q is an integer in the range from 0 to 5,
   each D, E, and G are independently selected from -O-, -NR³-, -N(COR⁴)-, -PR⁵(O)-, and -P(OR⁶)(O)-, wherein R³, R⁴, R⁵, and R⁶ independently represent hydrogen or C₁₋₆-alkyl,
   Z is selected from -C(O)NH-, -C(O)NHCH₂-, -OC(O)NH -, -C(O)NHCH₂CH₂-, -C(O)CH₂-, -C(O)CH=CH-, -(CH₂)ₛ-, -C(O)-, -C(O)O- or -NHC(O)-, wherein s is 0 or 1,
Y is a chemical group linking B and the therapeutic agent, and
W" is a chemical group linking B with A and A'.

Also disclosed herein is a compound comprising a hydrophilic spacer between a therapeutic peptide and one or more albumin binding residue(s), said compound having a protracted profile of action relative to the therapeutic polypeptide, where the albumin binding fraction as well as the free fraction of said compound are both able to bind to the receptor mediating the effect of the therapeutic polypeptide.

In one embodiment, the hydrophilic spacer is an unbranched oligo ethylene glycol moiety with appropiate funtional groups at both terminals that forms a bridge between an amino group of the therapeutic polypeptide and a funtional group of the albumin binding residue.

In one embodiment, Y is selected from the group consisting of -C(O)NH-, -NHC(O)-, -C(O)NHCH₂-, -CH₂NHC(O)-, -OC(O)NH -, -NHC(O)O-, -C(O)NHCH₂-, CH₂NHC(O)-, -C(O)CH₂-, -CH₂C(O)-, -C(O)CH=CH-, -CH=CHC(O)-, -(CH₂)ₛ-, -C(O)-, -C(O)O-, -OC(O)-, -NHC(O)- and -C(O)NH-, wherein s is 0 or 1.

In another embodiment, W is selected from the group consisting of of -C(O)NH-, - NHC(O)-, -C(O)NHCH₂-, -CH₂NHC(O)-, -OC(O)NH -, -NHC(O)O-, -C(O)CH₂-, -CH₂C(O)-, -C(O)CH=CH-, -CH=CHC(O)-, -(CH₂)ₛ-, -C(O)-, -C(O)O-, -OC(O)-, -NHC(O)- and -C(O)NH-, wherein s is 0 or 1.

In another embodiment, W" is selected from the group consisting of 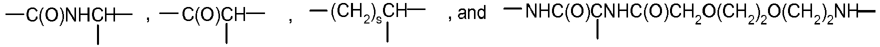 , wherein s is 0, 1 or 2.

In another embodiment, I is 1 or 2, n and m are independently 1-10 and p is 0-10.

In another embodiment D is -O-.

In another embodiment of the invention, E is -O-.

In yet another embodiment of the invention the hydrophilic spacer is -CH₂O[(CH₂)₂O]ₘ(CH₂)ₚQ_{q}-, where m is 1-10, p is 1-3, and Q is -Z-CH₂O[(CH₂)₂O]ₘ(CH₂)ₚ-.

In another embodiment q is 1.

In another embodiment G is -O-.

In yet another embodiment of the invention Z is selected from the group consisting of - C(O)NH-, -C(O)NHCH₂-, and -OC(O)NH-.

In yet another embodiment q is 0.

In another embodiment I is 2.

In another embodiment n is 2.

In yet another embodiment the hydrophilic spacer B is -[CH₂CH₂O]ₘ₊₁(CH₂)ₚQ_{q}-.

In yet another embodiment the hydrophilic spacer B is -(CH₂)ₗ-O-[(CH₂)ₙ-O]ₘ-(CH₂)ₚ-[C(O)NH-(CH₂)ₗ-O-[(CH₂)ₙ-O]ₘ-(CH₂)ₚ]_{q}-,
where I, m, n, and p independently are 1-5, and q is 0-5.

In yet another embodiment, -W-B-Y- is selected from the group consisting of 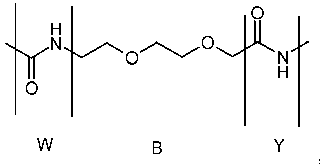 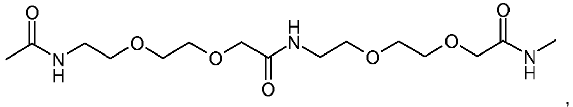 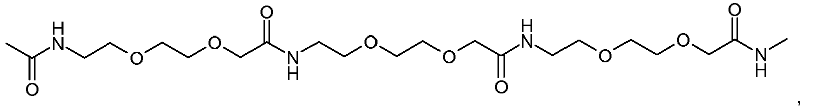 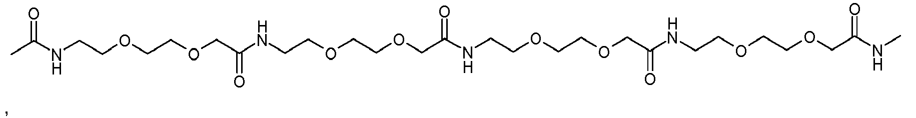 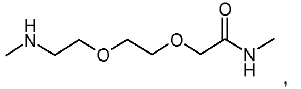 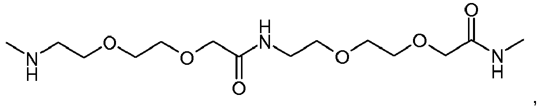 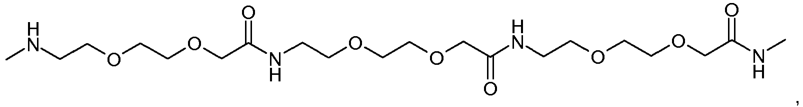 and

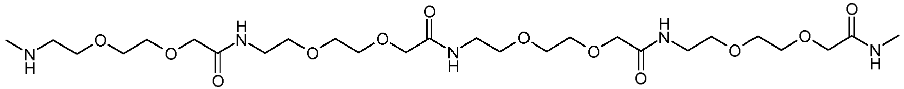

In yet another embodiment, >W"-B-Y- is

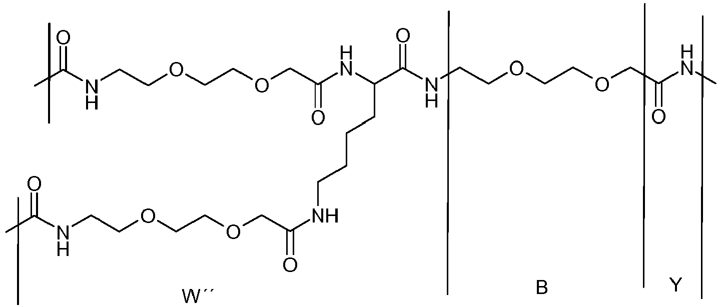

In yet another embodiment, the albumin binding residue A is selected from the group consisting of 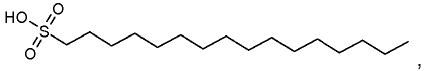 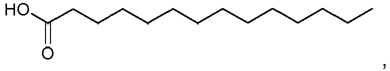 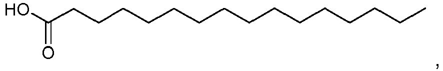 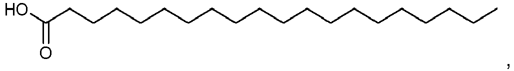 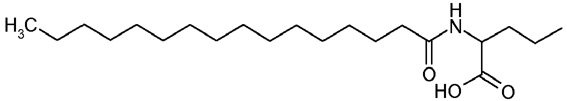 where the chiral carbon atom is either R or S, 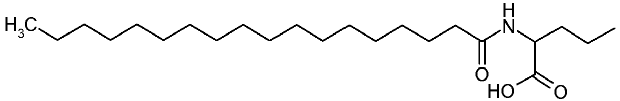 where the chiral carbon atom is either R or S, 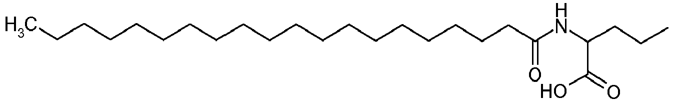 where the chiral carbon atom is either R or S, 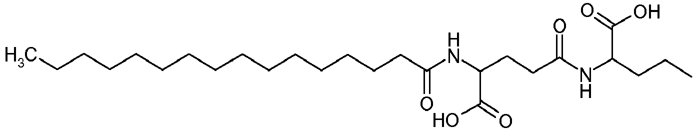 where the two chiral carbon atoms independently are either R or S, 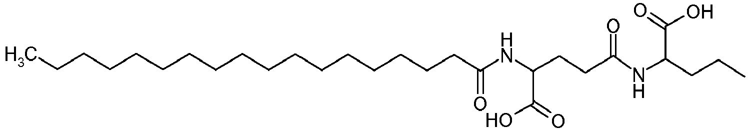 where the two chiral carbon atoms independently are either R or S, 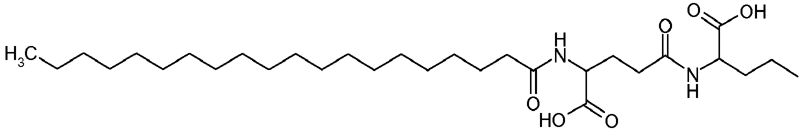 where the two chiral carbon atoms independently are either L or D, 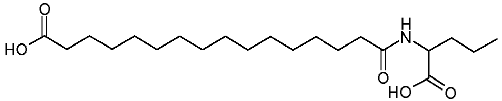 where the chiral carbon atom is either R or S, 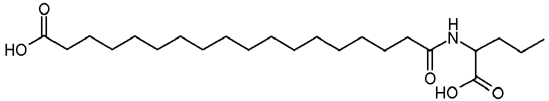 where the chiral carbon atom is either R or S, 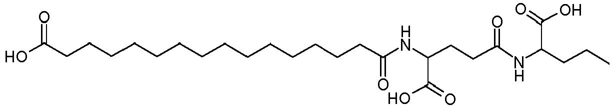 where the two chiral carbon atoms independently are either R or S, 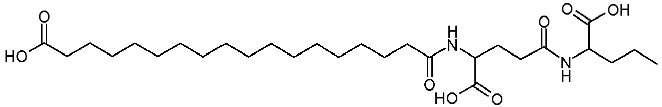 where the two chiral carbon atoms independently are either R or S, 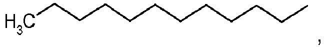 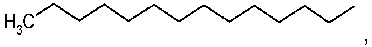 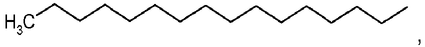 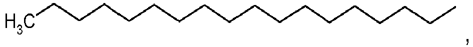 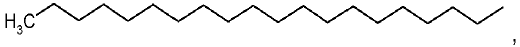 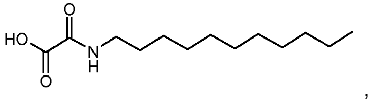 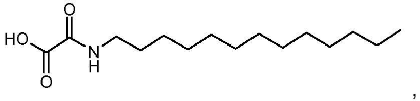 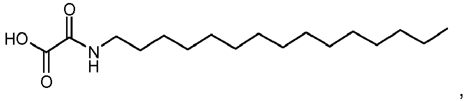 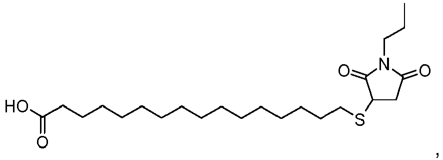 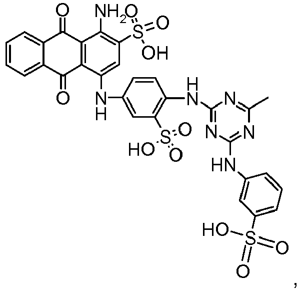 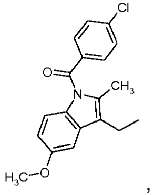 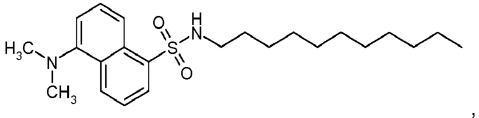 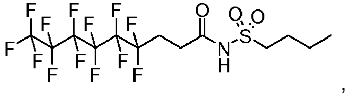 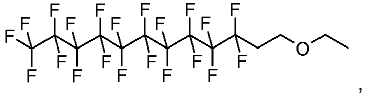 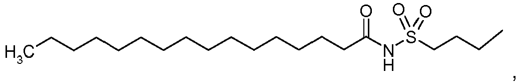 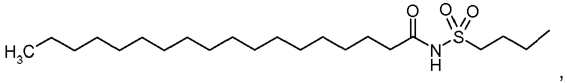 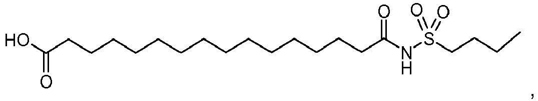 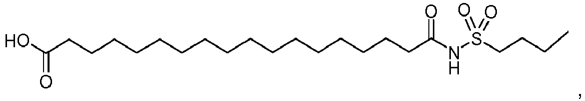 and

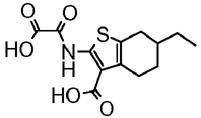

In yet another embodiment the molar weight of the hydrophilic spacer is in the range from 80D to 1000D or in the range from 80D to 300D.

In another embodiment, the albumin binding residue is a lipophilic residue.

In another embodiment the albumin binding residue is negatively charged at physiological pH. In another embodiment the albumin binding residue comprises a group which can be negatively charged. One preferred group which can be negatively charged is a carboxylic acid group.

In another embodiment, the albumin binding residue binds non-covalently to albumin. In another embodiment the albumin binding residue has a binding affinity towards human serum albumin that is below about 10 µM or below about 1µM.

In yet another embodiment the albumin binding residue is selected from a straight chain alkyl group, a branched alkyl group, a group which has an ω-carboxylic acid group, a partially or completely hydrogenated cyclopentanophenanthrene skeleton.

In another embodiment the albumin binding residue is a cibacronyl residue.

In another embodiment the albumin binding residue has from 6 to 40 carbon atoms, from 8 to 26 carbon atoms or from 8 to 20 carbon atoms.

In another embodiment the albumin binding residue is an acyl group selected from the group comprising CH₃(CH₂)ᵣCO-, wherein r is an integer from 4 to 38, preferably an integer from 4 to 24, more preferred selected from the group comprising CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

In another embodiment the albumin binding residue is an acyl group of a straight-chain or branched alkane α,ω-dicarboxylic acid.

In another embodiment the albumin binding residue is an acyl group selected from the group comprising HOOC(CH₂)ₛCO-, wherein s is an integer from 4 to 38, preferably an integer from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

In another embodiment the albumin binding residue is a group of the formula CH₃(CH₂)ᵥCO-NHCH(COOH)(CH₂)₂CO-, wherein v is an integer of from 10 to 24.

In another embodiment the albumin binding residue is a group of the formula CH₃(CH₂)_{w}CO-NHCH((CH₂)₂COOH)CO-, wherein w is an integer of from 8 to 24.

In another embodiment the albumin binding residue is a group of the formula COOH(CH₂)ₓCO- wherein x is an integer of from 8 to 24.

In another embodiment the albumin binding residue is a group of the formula -NHCH(COOH)(CH₂)₄NH-CO(CH₂)_{y}CH₃, wherein y is an integer of from 8 to 18.

In another embodiment the albumin binding residue is a peptide, such as a peptide comprising less than 40 amino acid residues. A number of small peptides which are albumin binding residues as well as a method for their identification is found in J. Biol Chem. 277, 38 (2002) 35035-35043.

In another embodiment of the invention the albumin binding residue via spacer and linkers is attached to said therapeutic polypeptide via the ε-amino group of a lysine residue.

In another embodiment the albumin binding residue via spacer and linkers is attached to said therapeutic polypeptide via an amino acid residue selected from cysteine, glutamate and aspartate.

In one embodiment of the present invention the therapeutic polypeptide is a GLP-1 peptide.

In another embodiment of the invention the therapeutic polypeptide is a GLP-1 peptide comprising the amino acid sequence of the formula (IV):
Xaa₇-Xaa₈-Glu-Gly-Thr-Phe-Thr-Ser-Asp-Xaa₁₆-Ser-Xaa₁₈-Xaa₁₉-Xaa₂₀-Glu-Xaa₂₂-Xaa₂₃-Ala-Xaa₂₅-Xaa₂₆-Xaa₂₇-Phe-lIe-Xaa₃₀-Trp-Leu-Xaa₃₃-Xaa₃₄-Xaa₃₅-Xaa₃₆-Xaa₃₇-Xaa₃₈-Xaa₃₉-Xaa₄₀-Xaa₄₁-Xaa₄₂-Xaa₄₃-Xaa₄₄-Xaa₄₅-Xaa₄₆
Formula (IV) (SEQ ID No: 2)
wherein
Xaa₇ is L-histidine, D-histidine, desamino-histidine, 2-amino-histidine, β-hydroxy-histidine, homohistidine, N^{α}-acetyl-histidine, α-fluoromethyl-histidine, α-methyl-histidine, 3-pyridylalanine, 2-pyridylalanine or 4-pyridylalanine;
Xaa₈ is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid;
Xaa₁₆ is Val or Leu;
Xaa₁₈ is Ser, Lys or Arg;
Xaa₁₉ is Tyr or Gln;
Xaa₂₀ is Leu or Met;
Xaa₂₂ is Gly, Glu or Aib;
Xaa₂₃ is Gln, Glu, Lys or Arg;
Xaa₂₅ is Ala or Val;
Xaa₂₆ is Lys, Glu or Arg;
Xaa₂₇ is Glu or Leu;
Xaa₃₀ is Ala, Glu or Arg;
Xaa₃₃ is Val or Lys;
Xaa₃₄ is Lys, Glu, Asn or Arg;
Xaa₃₅ is Gly or Aib;
Xaa₃₆ is Arg, Gly or Lys;
Xaa₃₇ is Gly, Ala, Glu, Pro, Lys, amide or is absent;
Xaa₃₈ is Lys, Ser, amide or is absent.
Xaa₃₉ is Ser, Lys, amide or is absent;
Xaa₄₀ is Gly, amide or is absent;
Xaa₄₁ is Ala, amide or is absent;
Xaa₄₂ is Pro, amide or is absent;
Xaa₄₃ is Pro, amide or is absent;
Xaa₄₄ is Pro, amide or is absent;
Xaa₄₅ is Ser, amide or is absent;
Xaa₄₆ is amide or is absent;
provided that if Xaa₃₈, Xaa₃₉, Xaa₄₀, Xaa₄₁, Xaa₄₂, Xaa₄₃, Xaa₄₄, Xaa₄₅ or Xaa₄₆ is absent then each amino acid residue downstream is also absent.

In another embodiment of the invention the polypeptide is a GLP-1 peptide comprising the amino acid sequence of formula (V):
Xaa₇-Xaa₈-Glu-Gly-Thr-Phe-Thr-Ser-Asp-Val-Ser-Xaa₁₈-Tyr-Leu-Glu-Xaa₂₂-Xaa₂₃-Ala-Ala-Xaa₂₆-Glu-Phe-Ile-Xaa₃₀-Trp-Leu-Val-Xaa₃₄-Xaa₃₅-Xaa₃₆-Xaa₃₇-Xaa₃₈
Formula (V) (SEQ ID No: 3)
wherein
Xaa₇ is L-histidine, D-histidine, desamino-histidine, 2-amino-histidine, β-hydroxy-histidine, homohistidine, N^{α}-acetyl-histidine, α-fluoromethyl-histidine, α-methyl-histidine, 3-pyridylalanine, 2-pyridylalanine or 4-pyridylalanine;
Xaa₈ is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid;
Xaa₁₈ is Ser, Lys or Arg;
Xaa₂₂ is Gly, Glu or Aib;
Xaa₂₃ is Gin, Glu, Lys or Arg;
Xaa₂₆ is Lys, Glu or Arg;
Xaa₃₀ is Ala, Glu or Arg;
Xaa₃₄ is Lys, Glu or Arg;
Xaa₃₅ is Gly or Aib;
Xaa₃₆ is Arg or Lys;
Xaa₃₇ is Gly, Ala, Glu or Lys;
Xaa₃₈ is Lys, amide or is absent.

In yet another embodiment of the invention the GLP-1 peptide is selected from GLP-1(7-35), GLP-1(7-36), GLP-1(7-36)-amide, GLP-1 (7-37), GLP-1(7-38), GLP-1 (7-39), GLP-1(7-40), GLP-1(7-41) or an analogue thereof.

In another embodiment the GLP-1 peptide is a fragment of a peptide selected from the group comprising GLP-1(7-35), GLP-1(7-36), GLP-1(7-36)amide, GLP-1(7-37), GLP-1(7-38), GLP-1(7-39), GLP-1(7-40) and GLP-1 (7-41) or an analogue thereof.

In another embodiment of the invention the GLP-1 peptide is GLP-1 (A-B) wherein A is an integer from 1 to 7 and B is an integer from 38 to 45 or an analogue thereof comprising one albumin binding residue attached via a hydrophilic spacer to the C-terminal amino acid residue and, optionally, a second albumin binding residue attached to one of the other amino acid residues.

In another embodiment the GLP-1 peptide comprises no more than fifteen amino acid residues which have been exchanged, added or deleted as compared to GLP-1(7-37) (SEQ ID No. 1), or no more than ten amino acid residues which have been exchanged, added or deleted as compared to GLP-1(7-37) (SEQ ID No. 1).

In another embodiment the GLP-1 peptide comprises no more than six amino acid residues which have been exchanged, added or deleted as compared to GLP-1(7-37) (SEQ ID No. 1).

In another embodiment the GLP-1 peptide comprises no more than 4 amino acid residues which are not encoded by the genetic code.

In another embodiment the GLP-1 peptide is a DPPIV protected GLP-1 peptide.

In another embodiment the compound according to this invention is DPPIV stabilised.

In another embodiment the GLP-1 peptide comprises an Aib residue in position 8.

In another embodiment the amino acid residue in position 7 of said GLP-1 peptide is selected from the group consisting of D-histidine, desamino-histidine, 2-amino-histidine, β-hydroxy-histidine, homohistidine, N^{α}-acetyl-histidine , α-fluoromethyl-histidine, α-methyl-histidine, 3-pyridylalanine, 2-pyridylalanine and 4-pyridylalanine.

In another embodiment the GLP-1 peptide is selected from the group consisting of Arg³⁴GLP-1(7-37),
Lys³⁸Arg^{26,34}GLP-1(7-38), Lys³⁸Arg^{26,34}GLP-1(7-38)-OH, Lys³⁶Arg^{26,34}GLP-1(7-36),
Aib^{8,22,35} GLP-1 (7-37), Aib^{8,35} GLP-1(7-37), Aib^{8,22} GLP-1 (7-37),
Aib^{8,22,35} Arg^{26,34}LyS³⁸GLP-1(7-38), Aib^{8,35} Arg^{26,34}LyS³⁸GLP-1(7-38),
Aib^{8,22} Arg^{26,34}Lys³⁸GLP-1(7-38), Aib^{8,22,35} Arg^{26,34}LyS³⁸GLP-1(7-38),
Aib^{8,35} Arg^{26,34}Lys³⁸GLP-1(7-38), Aib^{8,22,35} Arg²⁶Lys³⁸GLP-1(7-38),
Aib^{8,35} Arg²⁶Lys³⁸GLP-1(7-38), Aib^{8,22} Arg²⁶Lys³⁸GLP-1(7-38),
Aib^{8,22,35} Arg³⁴Lys³⁸GLP-1(7-38), Aib^{8,35}Arg³⁴LyS³⁸GLP-1(7-38), Aib^{8,22}Arg³⁴Lys³⁸GLP-1(7-38), Aib^{8,22,35}Ala³⁷Lys³⁸GLP-1(7-38), Aib^{8,35}Ala³⁷Lys³⁸GLP-1(7-38), Aib^{8,22}Ala³⁷Lys³⁸GLP-1(7-38), Aib^{8,22,35} Lys³⁷GLP-1(7-37), Aib^{8,35}LyS³⁷GLP-1(7-37) and Aib^{8,22}Lys³⁷GLP-1(7-38).

In another embodiment the GLP-1 peptide is attached to said hydrophilic spacer via the amino acid residue in position 23, 26, 34, 36 or 38 relative to the amino acid sequence SEQ ID No:1.

In another embodiment the GLP-1 peptide is exendin-4 (SEQ ID NO 4).

In another embodiment the GLP-1 peptide is ZP-10, i.e. HGEGTFTSDLSKQMEEEAVRLFIEWLKNGGPSSGAPPSKKKKKK-amide (SEQ ID NO 5).

In another embodiment the GLP-1 peptide is HGEGTFTSDLSKQMEEEAVRLFIEWLKNGGX, wherein X = P or Y, or a fragment or an analogue thereof.

In another embodiment, the GLP-1 peptide is Arg¹⁸, Leu²⁰, Gln³⁴, Lys³³ (N^{ε}-(γ-aminobutyroyl(N^{α}-hexadecanoyl))) Exendin-4-(7-45)-amide or Arg³³, Leu²⁰, Gln³⁴, Lys¹⁸ (N^{ε}-(γ-aminobutyroyl(N^{α}-hexadecanoyl))) Exendin-4-(7-45)-amide.

In another embodiment of the invention one albumin binding residue is attached to the C-terminal amino acid residue of the GLP-1 peptide via the hydrophilic spacer.

In another embodiment of the invention a second albumin binding residue is attached to an amino acid residue which is not the C-terminal amino acid residue of the GLP-1 peptide.

In another embodiment, the lipophilic substituent is attached to the GLP-1 peptide by means of a hydrophilic spacer in such a way that a carboxyl group of the spacer forms an amide bond with an amino group of the GLP-1 peptide.

The compound may be selected from the group consisting of
N^{ε37}-(2-(2-(2-(dodecylamino)ethoxy)ethoxy)acetyl)-[Aib^{8,22,35}Lys³⁷]GLP-1(7-37)amide 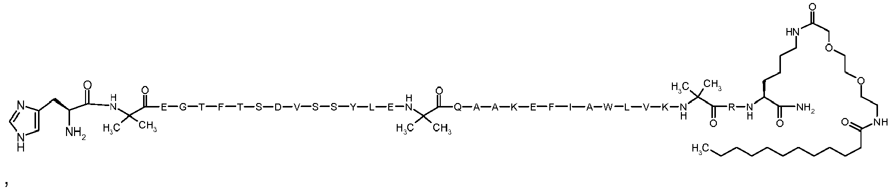
N^{ε37}-(2-(2-(2-(17-sulphohexadecanoylamino)ethoxy)ethoxy)acetyl)-[Aib^{8,22,35},Lys³⁷] GLP-1 (7-37)amide 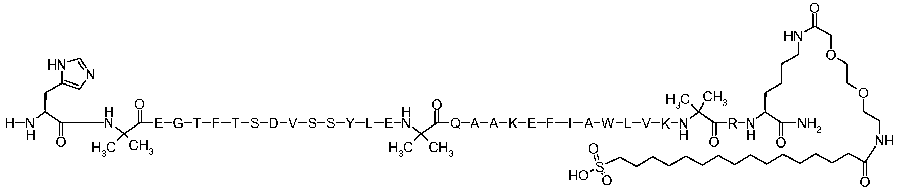 ,
N^{ε37}-{2-[2-(2-(15-carboxypentadecanoylamino)ethoxy)ethoxy]acetyl}-[Aib^{8,22,35},Lys³⁷] GLP-1(7-37)amide 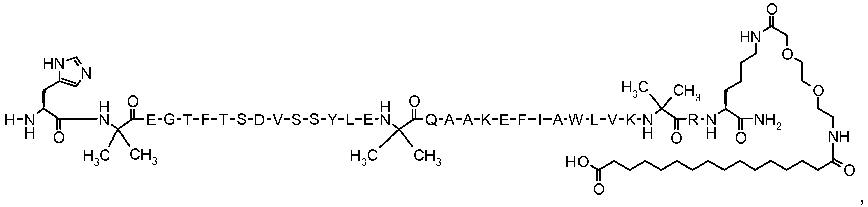
N^{ε37}-(2-(2-(2-(17-carboxyheptadecanoylamino)ethoxy)ethoxy)acetyl)[Aib^{8,22,35},Lys³⁷]GLP-1(7-37)amide 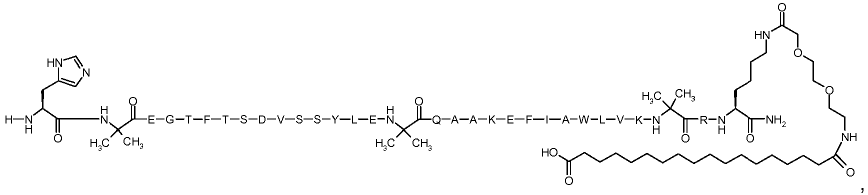
N^{ε37}-(2-(2-(2-(19-carboxynonadecanoylamino)ethoxy)ethoxy)acetyl)[Aib^{8,22,35},Lys³⁷]GLP-1(7-37)amide 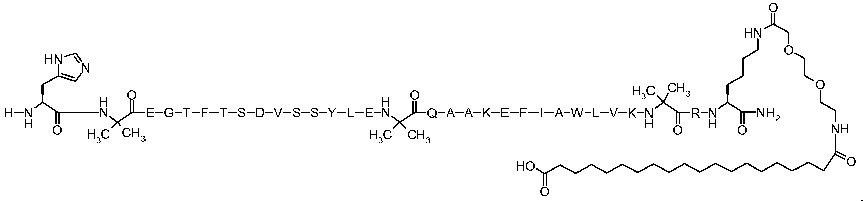
[Aib^{8,22,35},Arg^{26,34}]GLP-1-(7-37)Lys(4-(Hexadecanoylamino)-4(S)-carboxybutyryl)-OH 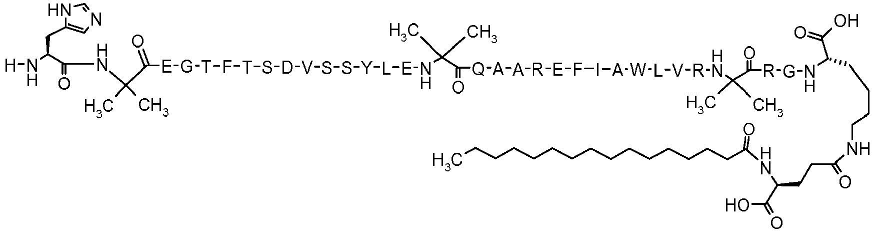
, [Aib^{8,22,35}Arg^{26,34}]GLP-1-(7-37)Lys(2-(2-(2-(hexadecanoylamino)ethoxy)ethoxy)acetyl)-OH 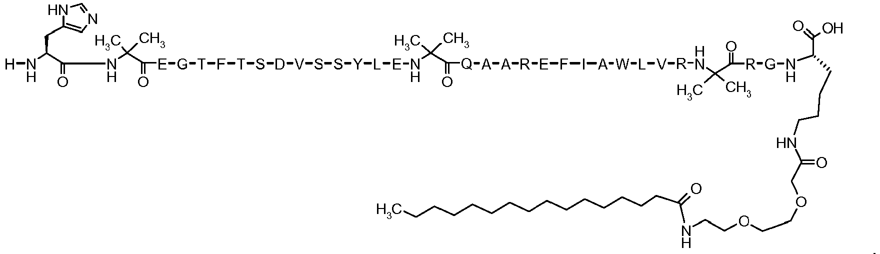
N^{ε37}-(2-[2-(2,6-(S)-Bis-{2-[2-(2-(dodecanoylamino)ethoxy)ethoxy]acetylamino}hexanoylamino)ethoxy]ethoxy}) acetyl-[Aib^{8,22,35}]GLP-1(7-37)amide 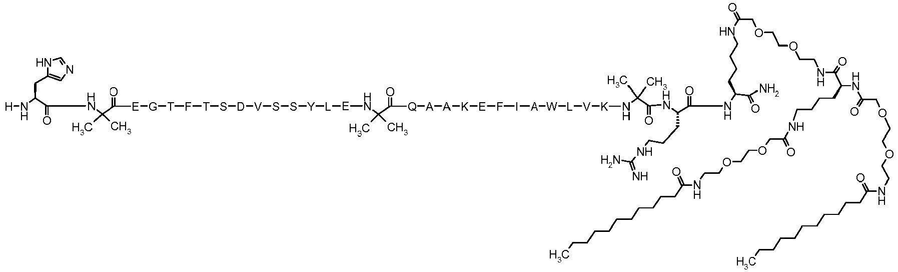 ,
N^{ε37}-(2-[2-(2,6-(S)-Bis-{2-[2-(2-(tetradecanoylamino)ethoxy)ethoxy]acetylamino}hexanoylamino)ethoxy]ethoxy}) acetyl-[Aib^{8,22,35}]GLP-1(7-37)amide 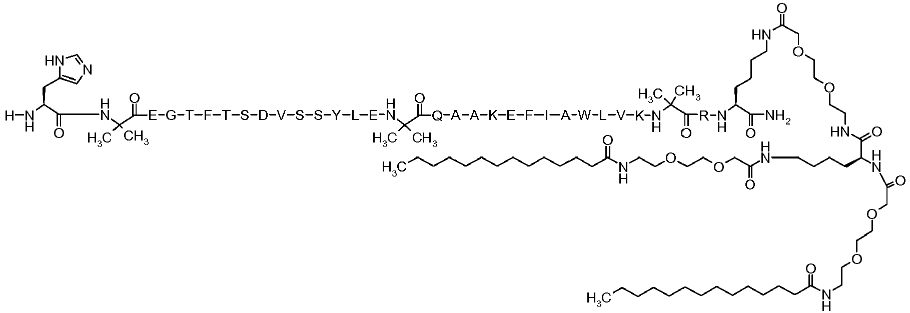
, [Aib^{8,22,35},Arg^{26,34}]GLP-1-(7-37)Lys(2-(2-(2-(4-(Hexadecanoylamino)-4(S)-carboxybutyrylamino)ethoxy)ethoxy)acetyl)-OH 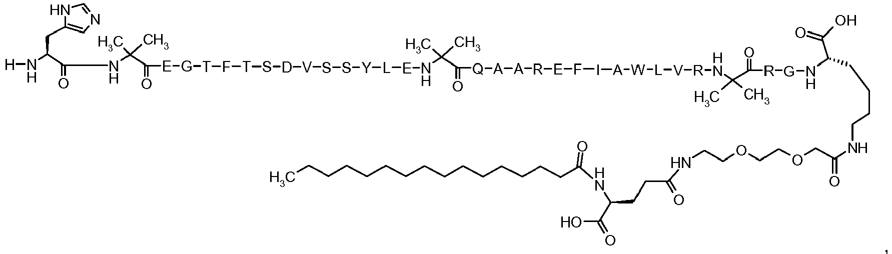
[Aib^{8,22,35}]GLP-1(7-37)Lys((2-{2-[4-[4-(4-Amino-9,10-dioxo-3-sulfo-9,10-dihydro-anthracen-1-ylamino)-2-sulfo-phenylamino]-6-(2-sulfo-phenylamino)-[1,3,5]triazin-2-ylamino]-ethoxy}-ethoxy)-acetyl))amide 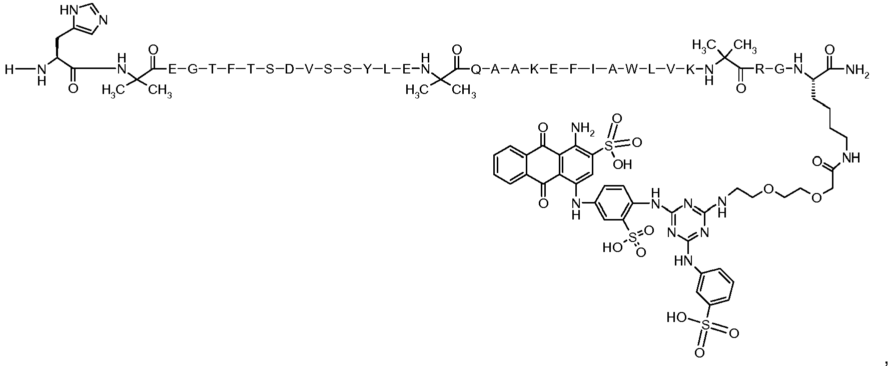
[Aib^{8,22,35}]GLP-1(7-37)Lys(({2-[2-(2-{2-[2-(2-{2-[2-(15-carboxypentadecanoylamino)-ethoxy]ethoxy}acetylamino)ethoxy]ethoxy}acetyl amino)ethoxy]ethoxy}acetyl))amide 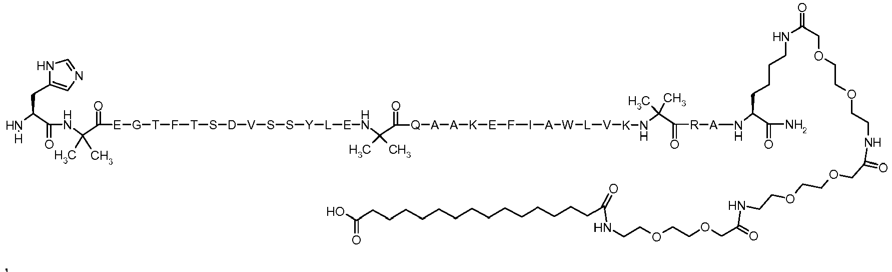
N^{ε37}-([2-(2-13-[2,5-dioxo-3-(15-carboxypentadecylsulfanyl)-pyrrolidin-1-yl]-propionylamino}ethoxy)ethoxy)acetyl-[D-Ala⁸,Lys³⁷]-GLP-1-[7-37]amide 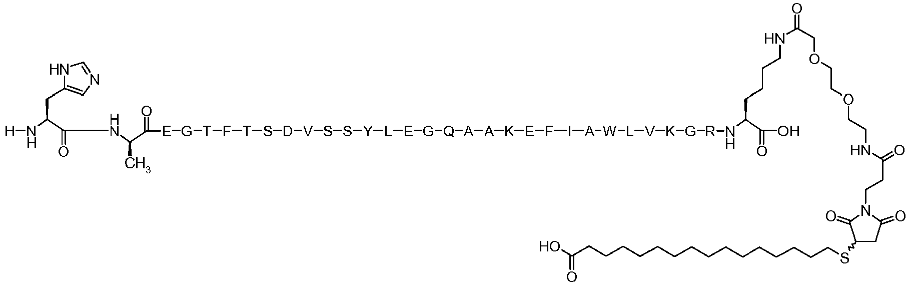
, [Aib^{8,22,35}Ala³⁷]GLP-1(7-37)Lys((2-(2-(2-(11-(oxalylamino)undecanoylamino)ethoxy)ethoxy)acetyl-)))amide 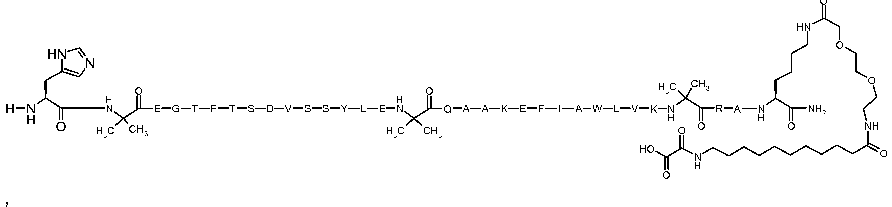
[Aib^{8,22,35},Ala³⁷]-GLP-1(7-37)Lys({2-[2-(2-{2-[2-(2-(15-carboxy-pentadecanoylamino)-ethoxy]ethoxy}acetylamino)ethoxy]ethoxy}acetyl)amide 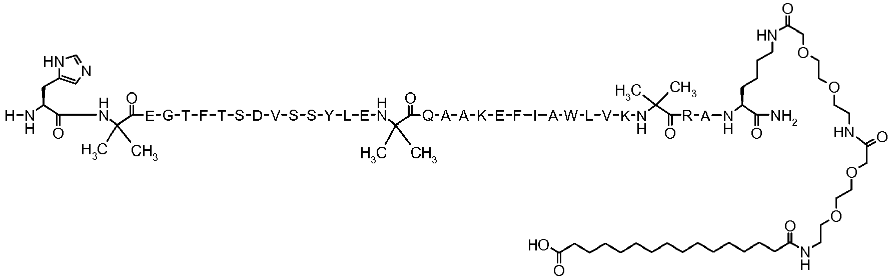 ,
[Aib^{8,22,35},Ala³⁷]-GLP-1(7-37)Lys((2-12-[11-(5-Dimethylaminonaphthalene-1-sulfonylamino)undecanoylamino]ethoxy}ethoxy)acetyl)amide 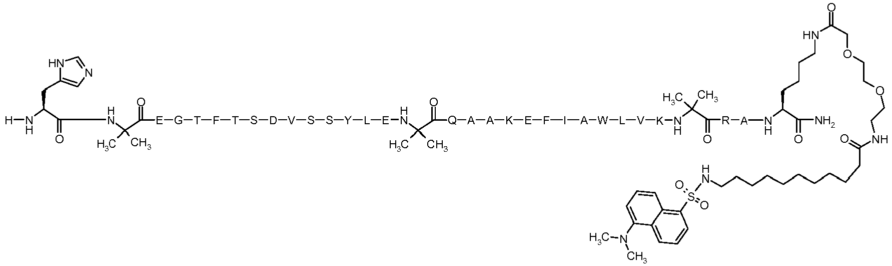 ,
[Aib^{8,22,35},Ala³⁷]-GLP-1(7-37)Lys(([2-(2-{2-[1-(4-Chlorobenzoyl)-5-methoxy-2-methyl-1H-indol-3-yl]acetylamino}ethoxy)ethoxy]acetyl))amide 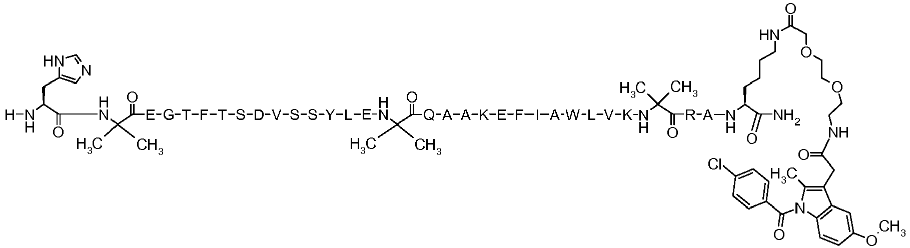
, [Aib⁸,Arg^{26,34},Glu^{22,23,30}]GLP-1 H(7-37)Lys(2-(2-(2-(octadecanoylamino)ethoxy)ethoxy)acetyl)amide 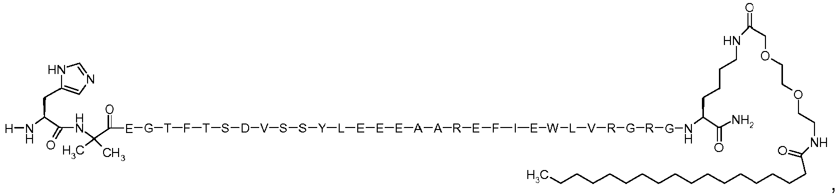
[Aib⁸,Arg^{26,34},Glu^{22,23,30}]GLP-1(7-37)Lys(2-(2-(2-(eicosanoylamino)ethoxy)ethoxy)acetyl)amide 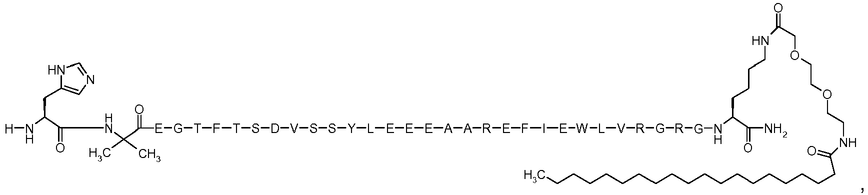
[Gly⁸,Arg^{26,34}] GLP-1 H-(7-37)Lys(2-(2-(2-(2-(2-(2-(4-(octadecanoylamino)-4(S)-carboxybutyrylamino)ethoxy)ethoxy)acetyl)ethoxy)ethoxy)acetyl)-OH 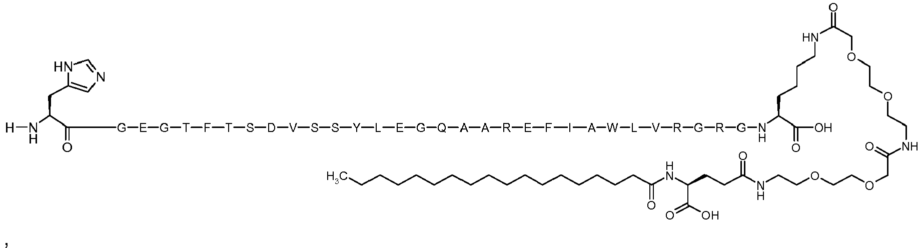
[Aib⁸,Arg^{26,34}]GLP-1 (7-37)Lys{2-(2-(2-(2-[2-(2-(octadecanoylamino)ethoxy)ethoxy]acetyl)ethoxy)ethoxy)acetyl)}-OH 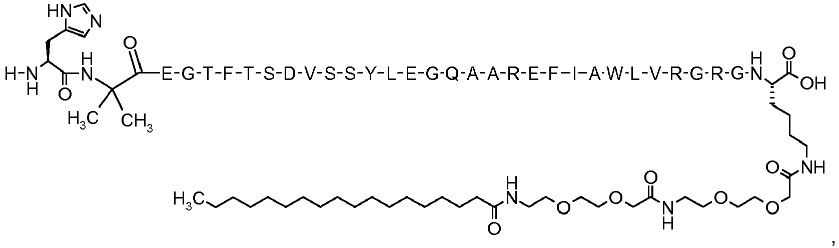
[Aib⁸]-GLP-1-(7-37)Lys (2-(2-(2-(4-(Hexadecanoylamino)-4(S)-carboxybutyrylamino)ethoxy)ethoxy)acetyl)-OH 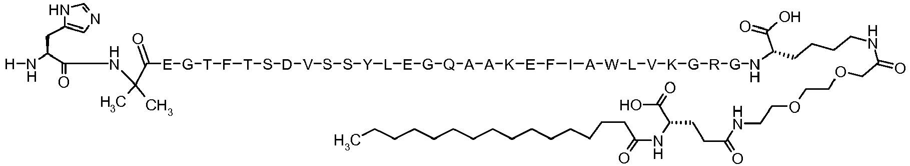 ,
[Aib⁸Arg^{26,14}] GLP-1 (7-37) Lys{2-(2-(2-(2-[2-(2-(4-(octadecanoylamino)-4-carboxybutyrylamino)ethoxy)ethoxy]acetyl)ethoxy)ethoxy)acetyl)}-OH 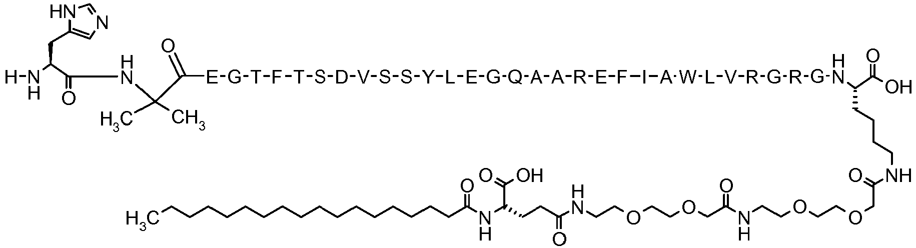 ,
[Aib⁸,Arg^{26,34}] GLP-1 (7-37)Lys{2-(2-(2-(2-[2-(2-(17-carboxyheptanoylamino)ethoxy)ethoxy]acetylamino)ethoxy)ethoxy)acetyl)}-OH 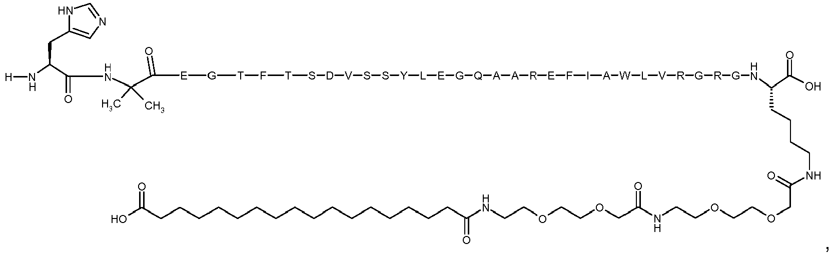
[Gly⁸, Arg^{26,34}] GLP1-(7-37) Lys{2-(2-(2-(2-[2-(2-(17-carboxyheptadecanoylamino)ethoxy)ethoxy]acetyl)ethoxy)ethoxy)acetyl)}-OH 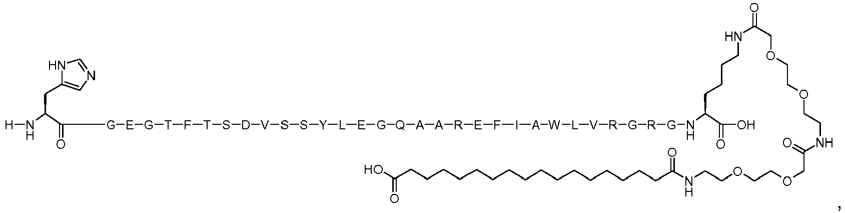
[Aib⁸]GLP-1-(7-37)Lys(2-(2-(2-(2-(2-(2-(4-(Hexadecanoylamino)-4(S)-carboxybutyrylamino)ethoxy)ethoxy)acetylamino) ethoxy)ethoxy)acetyl)-OH 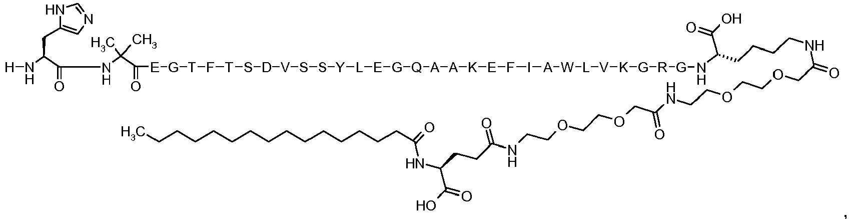
N^{ε37}-(2-(2-(2-(dodecanoylamino)ethoxy)ethoxy)acetyl)-[Aib^{8,22,35}Lys³⁷] GLP-1 H(7-37)-amide 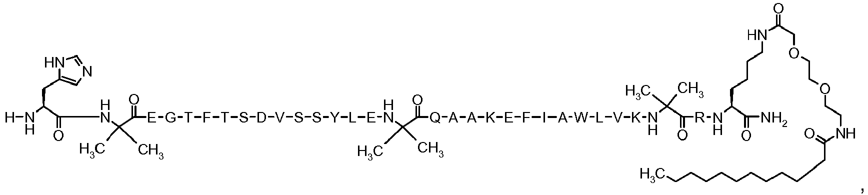
N^{ε37}-(2-(2-(2-(tetradecanoylamino)ethoxy)ethoxy)acetyl)-[Aib^{8,22,35}Lys³⁷] GLP-1 H(7-37)-amide 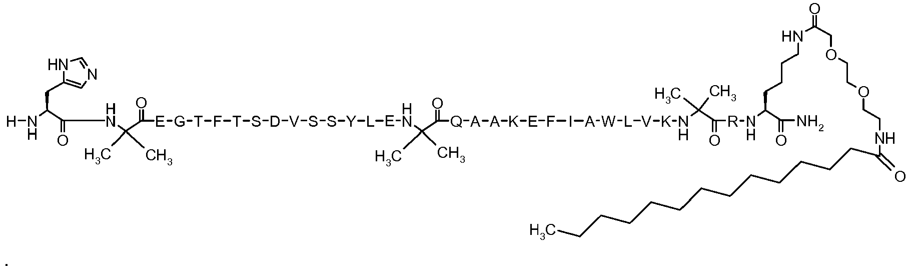
N^{ε37}-(2-(2-(2-(hexadecanoylamino)ethoxy)ethoxy)acetyl)-[Aib^{8,22,35}Lys³⁷] GLP-1 (7-37)-amide 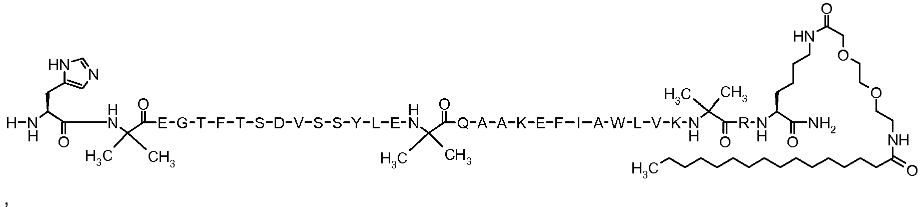
N^{ε37}-(2-(2-(2-(octadecanoylamino)ethoxy)ethoxy)acetyl)-[Aib^{8,22,35}Lys³⁷] GLP-1 (7-37)-amide 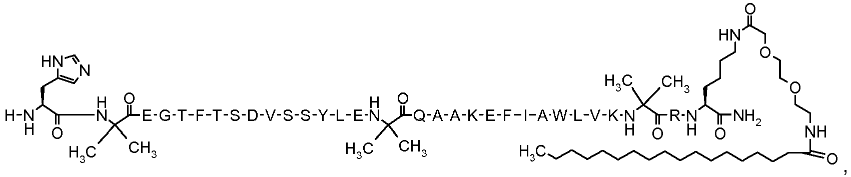
N^{ε37}-(2-(2-(2-(eicosanoylamino)ethoxy)ethoxy)acetyl)-[Aib^{8,22,35}Lys³⁷] GLP-1(7-37)-amide 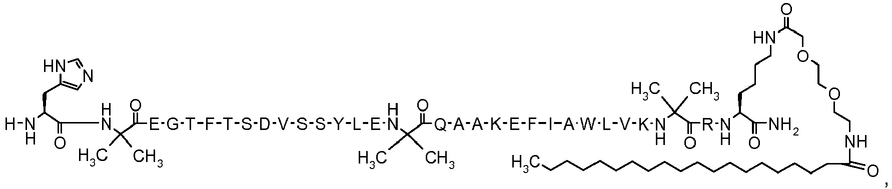
N^{ε36}-(2-(2-(2-(2-(2-(2-(octadecanoylamino)ethoxy)ethoxy)acetylamino)ethoxy)ethoxy)acetyl))-[Aib⁸,Arg^{26,34},Lys³⁶]GLP-1-(7-37)-OH 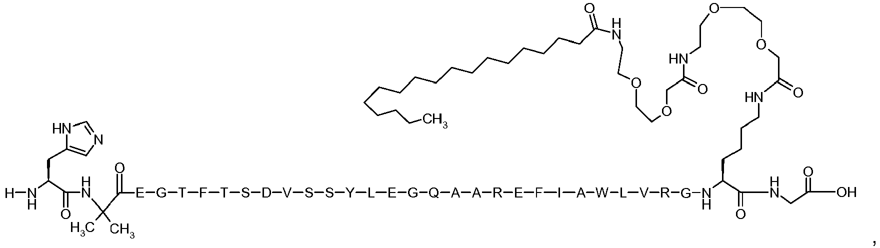
N^{ε36}-(2-(2-(2-(2-(2-(2-(octadecanoylamino)ethoxy)ethoxy)acetylamino)ethoxy)ethoxy)acetyl))[Arg^{26,34},Lys³⁶]GLP-1(7-37)-OH 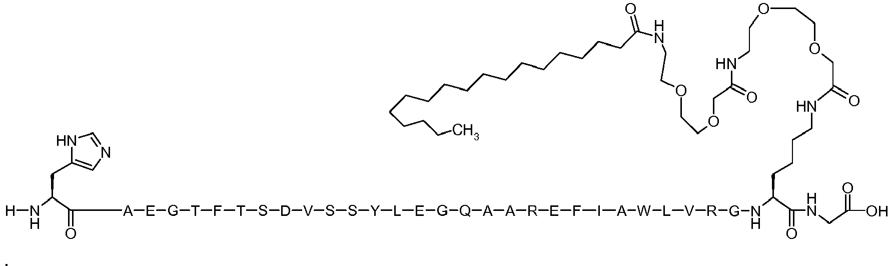
N^{ε36}-{2-(2-(2-(2-[2-(2-(octadecanoylamino)ethoxy)ethoxy)acetylamino)ethoxy)ethoxy)acetyl)}-[Gly⁸,Arg^{26,34},Lys³⁶]GLP-1-(7-37)-OH 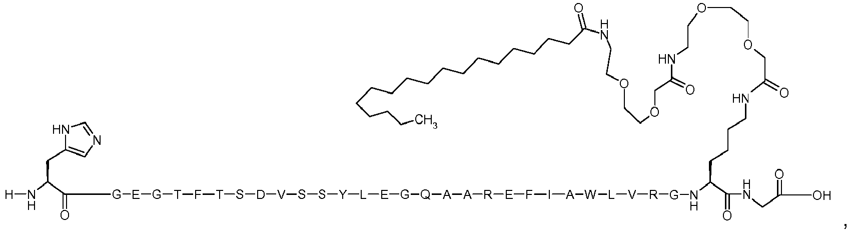
N^{ε37}-(2-(2-(2-(4-4(4,4,5,5,6,6,7,7,8,8,9,9,9-tridecafluorononanoylsulfamoyl-butyrylamino)ethoxy)ethoxy)acetyl))[Aib^{8,22,35},Lys³⁷]GLP-1-(7-37)-OH 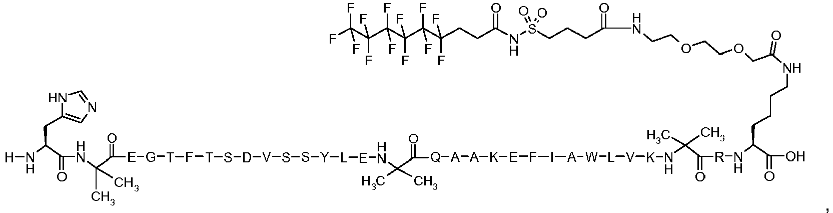
N^{ε37}-(2-(2-(2-(3,3,4,4,5,5,6,6,7,7,8,8,9,9,10,10,11,11,12,12,12-Heneicosafluoro-dodecyloxyacetylamino)ethoxy) ethoxy)acetyl)[Aib^{8,22,35},Lys³⁷]GLP-1-(7-37)-OH 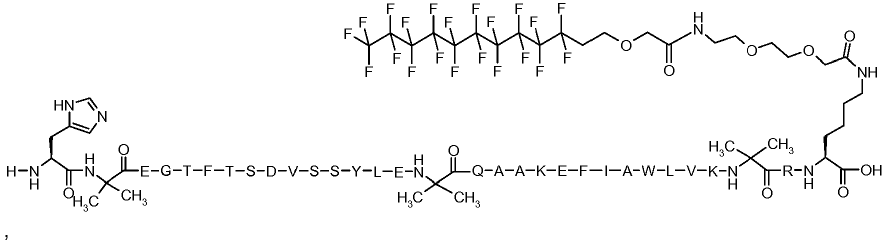
N^{ε37}-(2-(2-(2-(4-(hexadecanoylsulfamoyl)butyrylamino)ethoxy)ethoxy)acetyl)[Aib^{8,22,35},Lys³⁷] GLP-1-(7-37)-OH 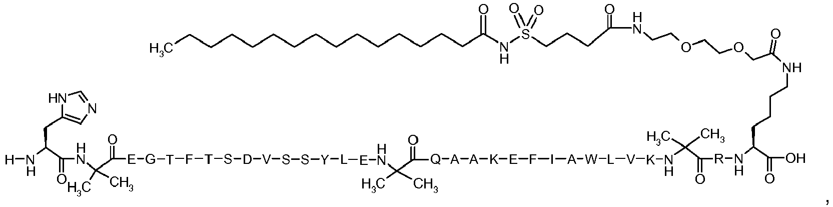
[Arg^{26,34}]GLP-1(7-37)Lys(12-(2-(2-(2-[2-(2-(octadecanoylamino)ethoxy)ethoxy]acetylamino)ethoxy)ethoxy)acetyl)})-OH 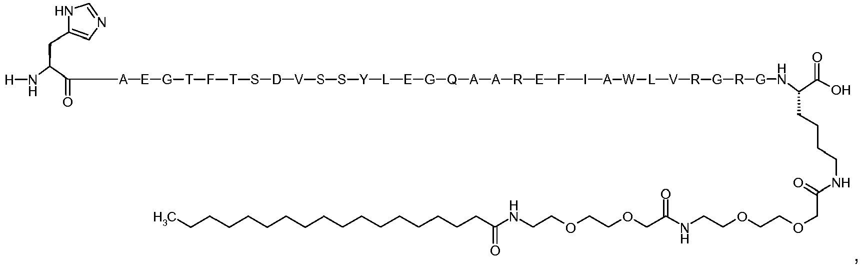
[Arg^{26,34}] GLP-1 (7-37)Lys{2-(2-(2-(2-[2-(2-(4-(octadecanoylamino)-4-carboxybutyrylamino)ethoxy)ethoxy]acetylamino)ethoxy)ethoxy)acetyl)}-OH 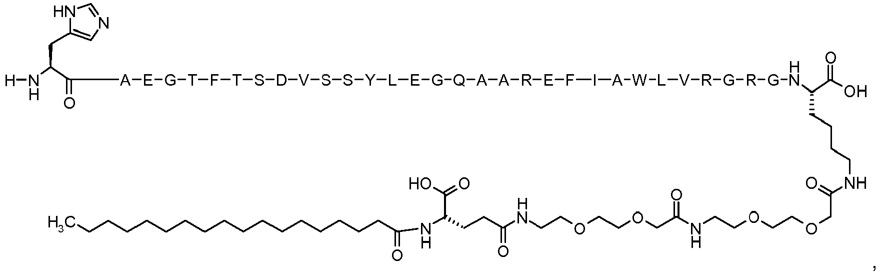
N^{ε20}-12-(2-(2-(2-[2-(2-(4-(hexadecanoylamino)-4-carboxybutyrylamino)ethoxy)ethoxy]acetylamino)ethoxy)ethoxy)acetyl)}-exendin(1-39) 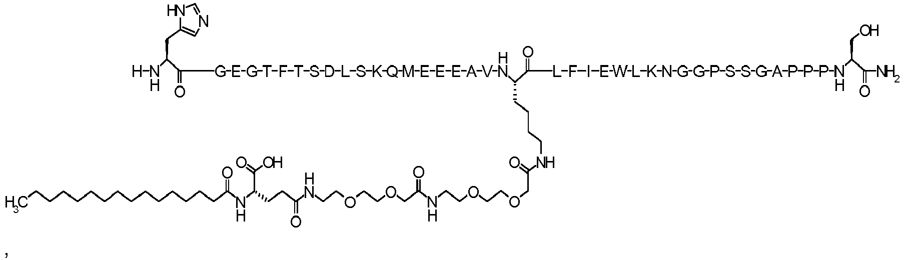
[Ala⁸, Arg^{26,34}]GLP-1(7-37)Lys((2-[2-((2-oxalylamino-3-carboxy-2-4,5,6,7-tetrahydro-benzo[b]thiophen-6-yl-acetylamino))ethoxy]ethoxyacetyl) amide 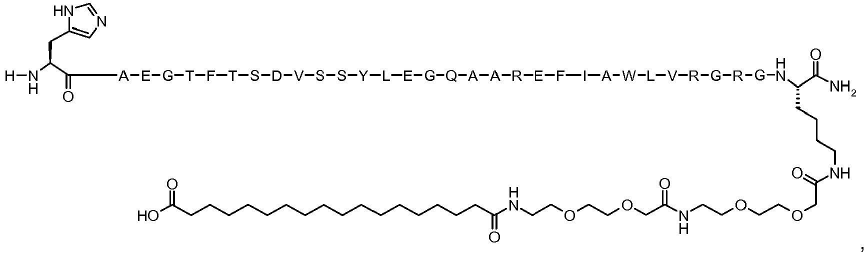
[Aib^{8,22,35}]GLP-1(7-37)Lys((2-[2-((2-oxalylamino-3-carboxy-2-4,5,6,7-tetrahydro-benzo[b]thiophen-6-yl-acetylamino))ethoxy]ethoxyacetyl) amide 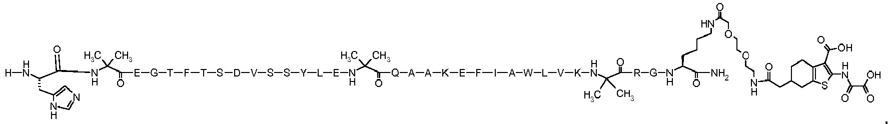
N^{ε36}-(2-(2-(2-(2-(2-(2-(4-(octadecanoylamino)-4(S)-carboxybutyrylamino)ethoxy)ethoxy)acetylamino)ethoxy)ethoxy)acetyl)-[Aib⁸,Arg^{26,34},Lys³⁶]GLP-1-(7-37)-OH 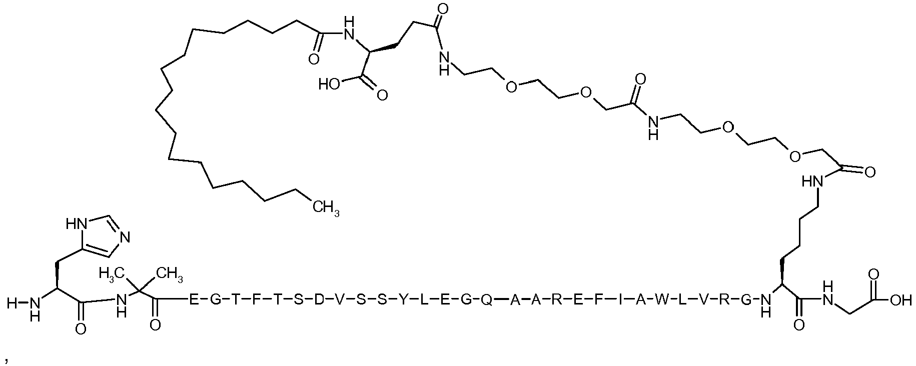
N^{ε36}-(2-(2-(2-(2-(2-(2-(4-(octadecanoylamino)-4(S)-carboxybutyrylamino)ethoxy)ethoxy)acetylamino)ethoxy)ethoxy)acetyl)-[Gly⁸,Arg^{26,34},Lys³⁶]GLP-1-(7-37)-OH 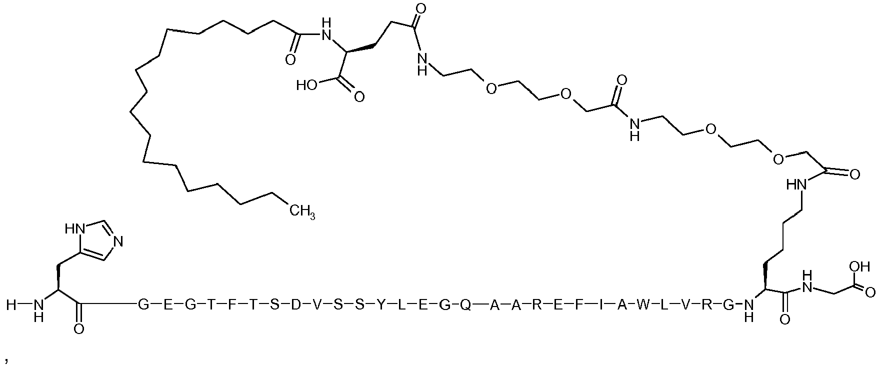
N^{ε37}-2-(2-(2-(4-(4-(Heptadecanoylamino)-4-(S)-carboxybutyrylamino)-4-(S)-carboxybutyrylamino)ethoxy)ethoxy) acetyl-[Aib^{8,22,35}Lys³⁷]GLP-1-(7-37)-NH₂ 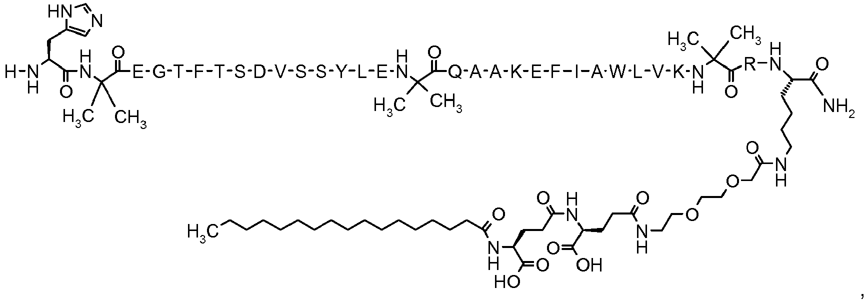
N^{ε37}-2-(2-[2-(2-[2-(4-[4-(Heptadecanoylamino)-4-(S) carboxybutyrylamino]-4-(S)-carboxybutyrylamino)ethoxy] ethoxy)acetylamino)ethoxy]ethoxy)acetyl-[Aib^{8,22,35},Lys³⁷]GLP-1-(7-37)-NH₂ 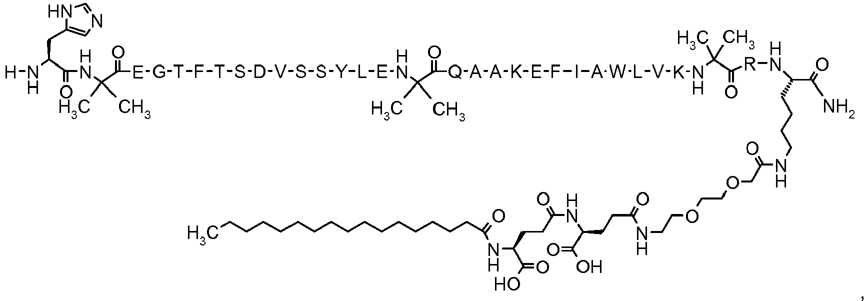
N^{ε26}-(2-(2-(2-(4-(Hexadecanoylamino)-4(S)-carboxybutyrylamino) ethoxy)ethoxy)acetyl)-[Aib⁸,Arg³⁴]GLP-1-(7-37)- -OH 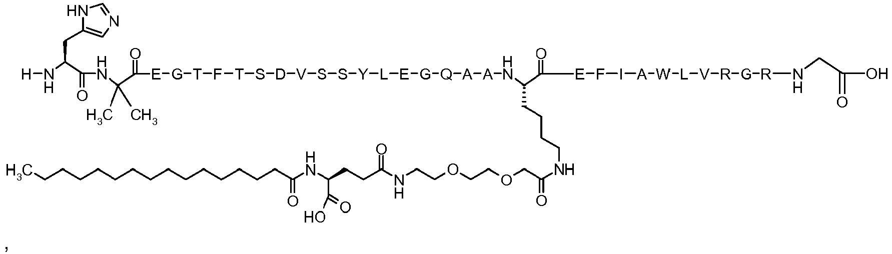
N^{ε26}-2-(2-2-(2-(2-(2-(4-(Octadecanoylamino)-4(S)-carboxybutyrylamino)ethoxy)ethoxy)acetylamino)ethoxy)ethoxy)acetyl-[Aib⁸, Arg³⁴]GLP-1-(7-37)-OH 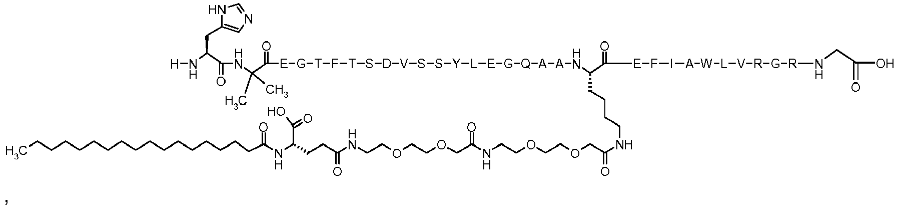
[Gly⁸,Arg^{26,34}]GLP-1(7-37)Lys(2-(2-(19-(carboxy)nonadecanoylamino)ethoxy)ethoxy)acetyl)-OH 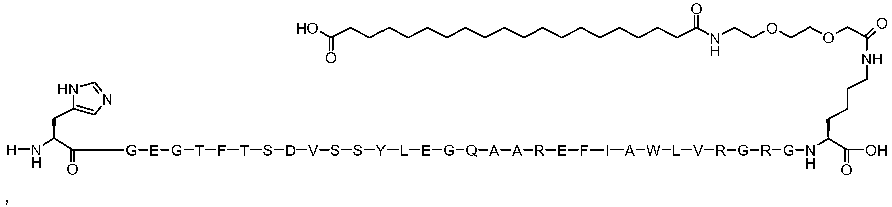
[Gly⁸,Arg^{26,34}]GLP-1(7-37)Lys((2-(2-(17-(carboxy)heptadecanoylamino)ethoxy)ethoxy)acetyl))-OH 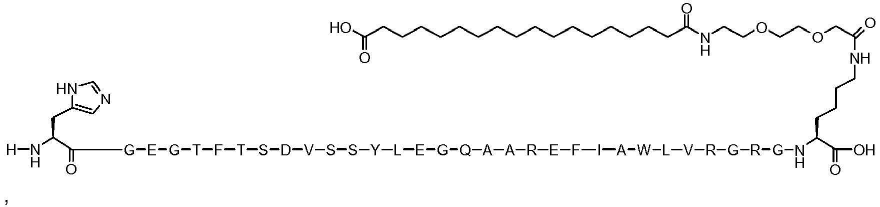
[Gly⁸,Arg^{26,34}]GLP-1(7-37)Lys(2-(2-(2-(4-(19-(carboxy)nonadecanoylamino)-4-carboxybutyrylamino)ethoxy)ethoxy)acetyl)-OH 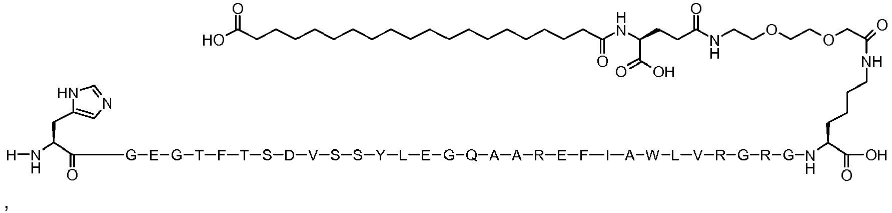
[Gly8,Arg^{26,34}]GLP-1 (7-37)Lys((2-(2-(2-(2-(2-(2-(2-(2-(2-(hexadecanoylamino)ethoxy)ethoxy)acetyl)ethoxy)ethoxy)acetylamino)ethoxy)ethoxy)-acetyl)-OH 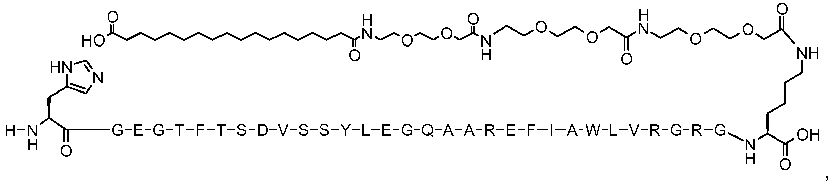
[Gly⁸, Arg^{26,34}]GLP-1(7-37)Lys (2-(2-(2-(2-(2-(2-(octadecanoylamino)ethoxy)ethoxy)-acetylamino)ethoxy)ethoxy)acetyl) NH₂ 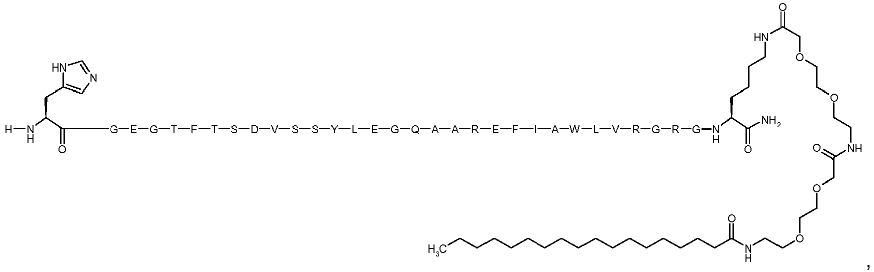
N^{ε20}(2-(2-(2-(2-(2-(2-(2-(2-(2-(2-(2-(2-(4-(17-(carboxy)heptadecanoylamino)-4-carboxybutyrylamino)ethoxy)ethoxy)acetylamino)ethoxy)ethoxy)acetylamino) ethoxy)ethoxy)acetylamino)ethoxy)ethoxy)acetyl) [Lys²⁰]exendin-4 (1-39)-NH₂ 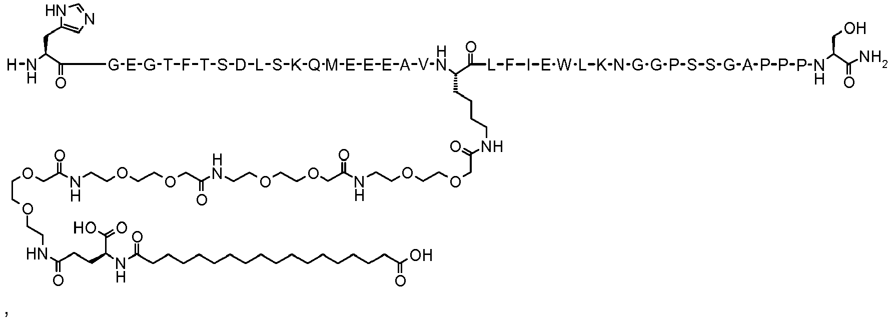
N^{ε36}-(2-(2-(2-(2-(2-(2-(17-Carboxyheptadecanoylamino)ethoxy)ethoxy) acetylamino)ethoxy)ethoxy)acetyl) [Aib⁸,Arg^{26,34}, Lys³⁶] GLP-1 (7-37) 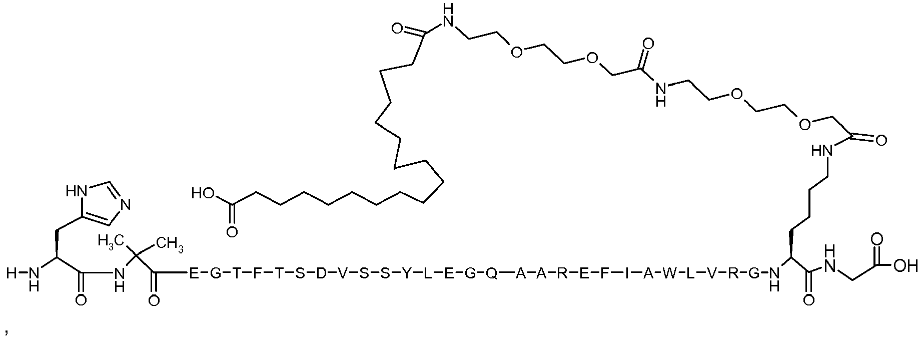
N-^{ε36}-(2-(2-(2-(2-(2-(2-(17-Carboxyheptadecanoylamino)ethoxy)ethoxy) acetylamino)ethoxy)ethoxy)acetyl) [Arg^{26,34}, Lys³⁶] GLP-1 (7-37) 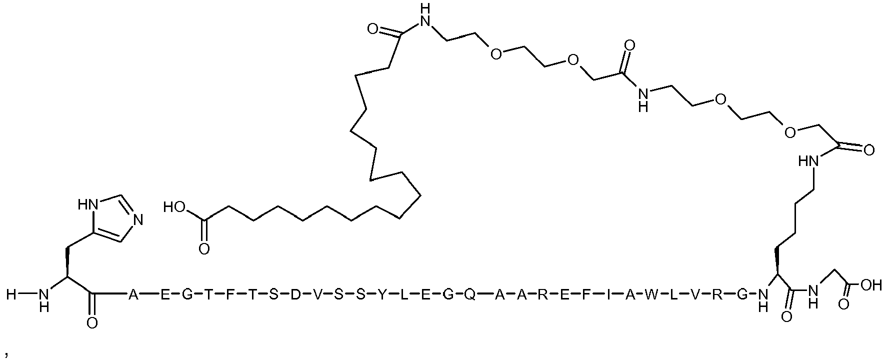
N^{ε36}-(2-(2-(2-(2-(2-(2-(17-Carboxyheptadecanoylamino)ethoxy)ethoxy) acetylamino)ethoxy)ethoxy)acetyl) [Gly⁸,Arg^{26,34},Lys³⁶] GLP-1 (7-37) 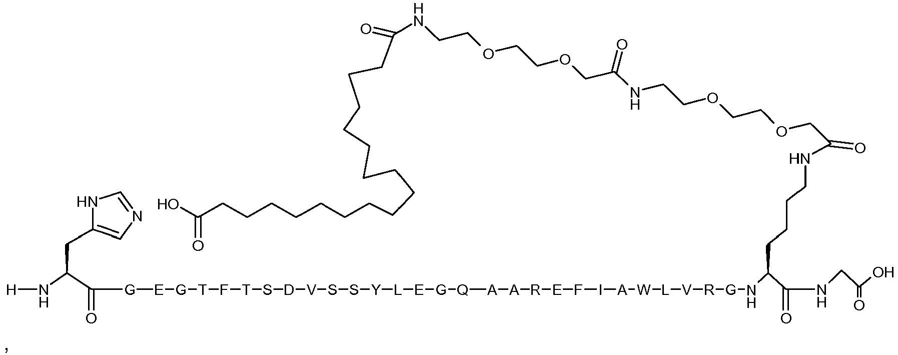
N^{ε20}-(2-(2-(2-(2-(2-(2-(2-(2-(2-(Octadecanoylamino)ethoxy)ethoxy)acetylamino)ethoxy)ethoxy)acetylamino)ethoxy)-ethoxy)acetyl)[Lys²⁰] Exendin-4 (1-39)amide 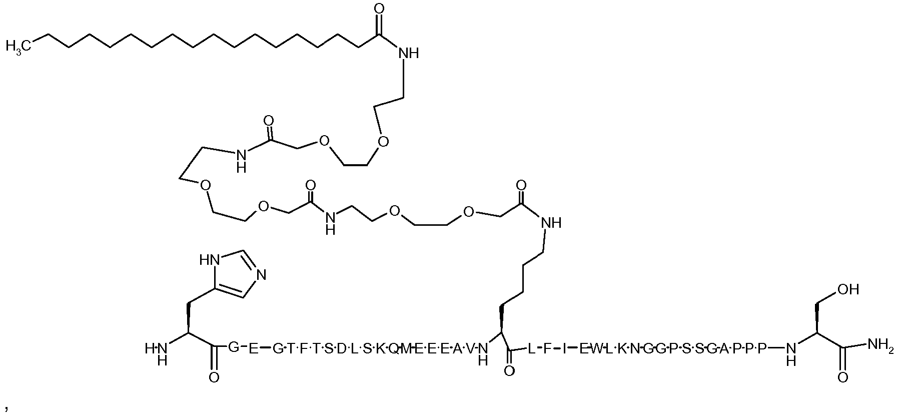
N^{ε36}-(2-(2-(2-(2-(2-(2-(4-(octadecanoylamino)-4(S)-carboxybutyrylamino)ethoxy)ethoxy)acetylamino)ethoxy)ethoxy)acetyl)-[Arg^{26,34},Lys³⁶]GLP-1-(7-37) 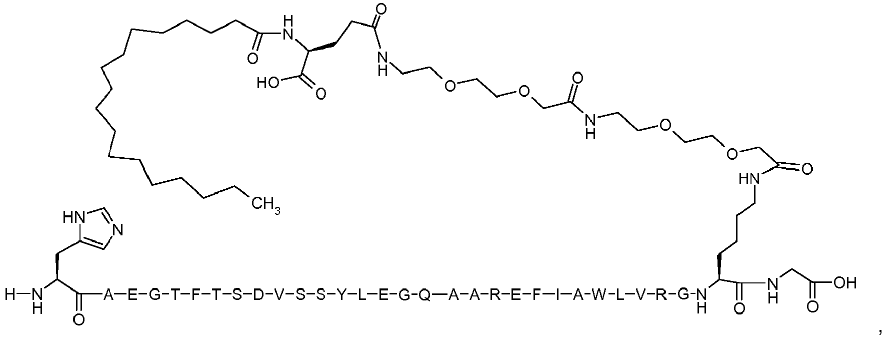
N^{ε26}-(2-[2-(2-[2-(2-[2-(17-Carboxyheptadecanoylamino)ethoxy] ethoxy)acetylamino]ethoxy)ethoxy]acetyl)[Arg³⁴]GLP-1-(7-37)-OH 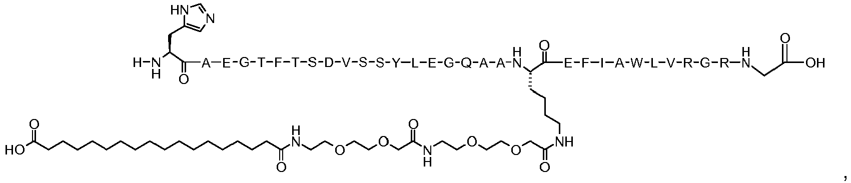
N^{ε26}-[2-(2-[2-(2-[2-(2-[4-(17-Carboxyheptadecanoylamino)-4(S)-carboxybutyrylamino]ethoxy)ethoxy]acetylamino)ethoxy]ethoxy)acetyl][Arg³⁴]GLP-1-(7-37)-OH 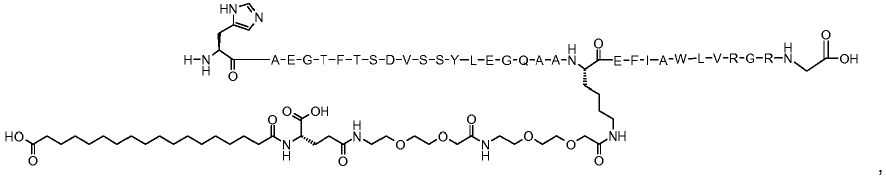
N^{ε20}-(2-(2-(2-(2-(2-(2-(2-(2-(2-(17-Carboxyheptadecanoylamino)ethoxy)ethoxy)acetylamino)ethoxy)ethoxy)acetylamino)ethoxy)ethoxy)acetyl)[Lys²⁰] Exendin-4 (1-39) amide 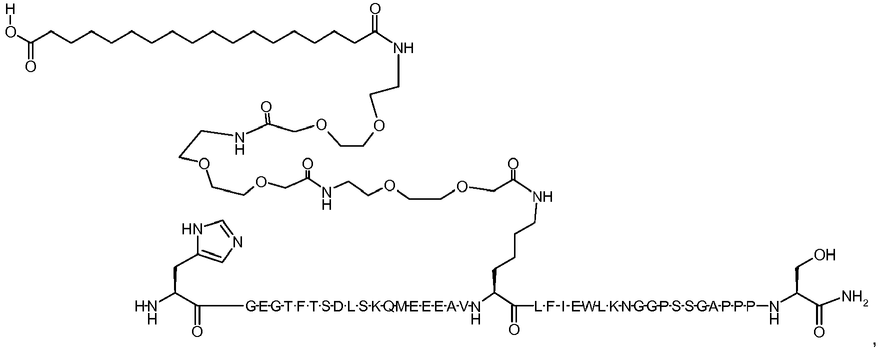
[Gly⁸, Glu^{22,23,30},Arg^{18,26,34}]GLP1 (7-37) Lys(2-(2-(2-(2-(2-(2-(17-carboxyheptadecanoylamino)ethoxy)ethoxy)acetylamino)ethoxy))ethoxy)acetyl)-NH₂ 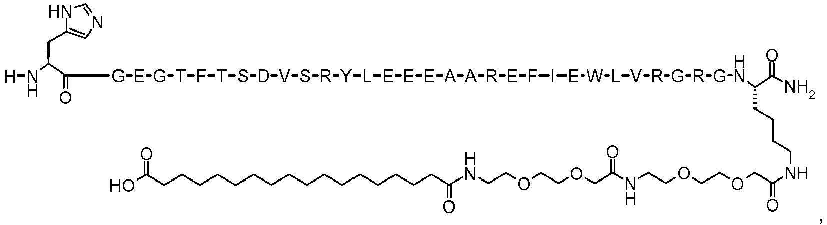
[Imidazolylpropionic acid7, Asp¹⁶, Aib^{22,35}]GLP1(7-37)Lys NH((2-{[4-(17-carboxyheptadecanoylamino)butylcarbamoyl]methoxy}ethoxy)ethoxy)) 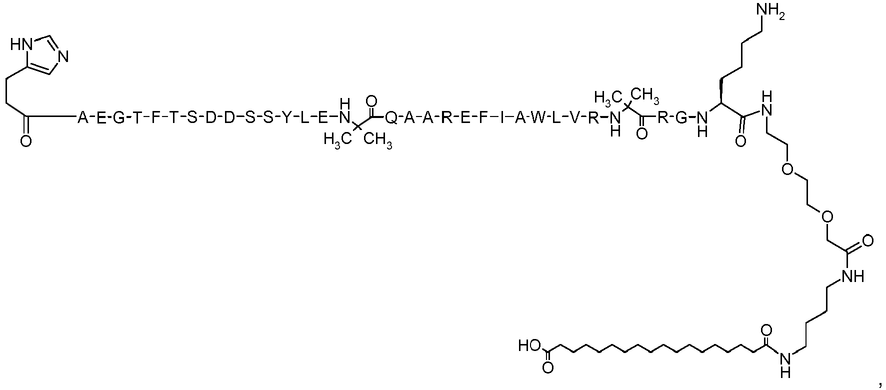
[Imidazolylpropionic acid7, Aib^{22,35}]GLP1(7-37)Lys NH((2-{[4-(17-carboxyheptadecanoylamino)butylcarbamoyl]methoxy}ethoxy)ethoxy)) 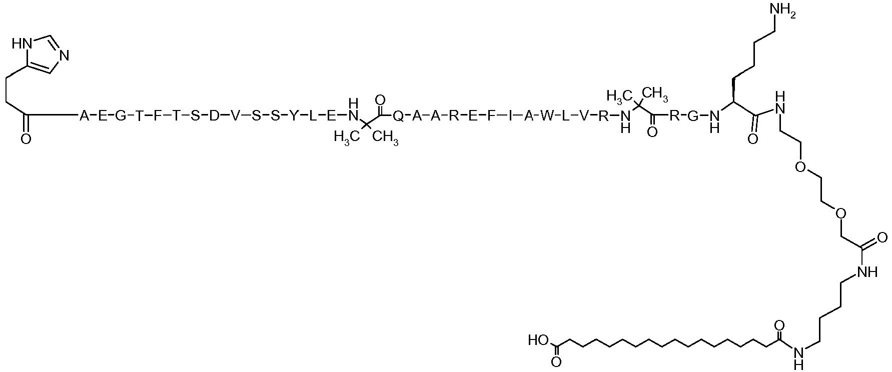 , and
[3-(5-Imidazoyl)propionyl⁷, Aib⁸, Arg^{26,34}] GLP-1 (7-37)Lys{2-(2-(2-(2-[2-(2-(17-carboxyheptanoylamino)ethoxy)ethoxy]acetylamino)ethoxy)ethoxy)acetyl)}-OH

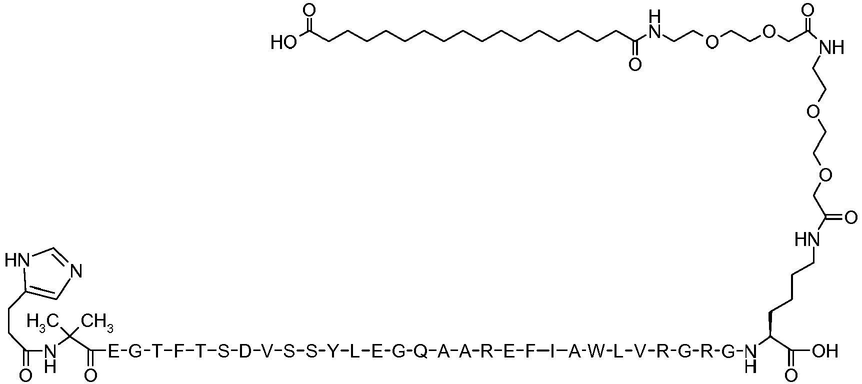

In another embodiment the therapeutic polypeptide is a GLP-2 peptide.

In another embodiment the GLP-2 peptide is a DPPIV-protected GLP-2 peptide.

In another embodiment the GLP-2 peptide is Gly²-GLP-2(1-33).

In yet another embodiment the GLP-2 peptide is Lys¹⁷Arg³⁰-GLP-2(1-33).

In one embodiment, the therapeutic polypeptide has a molar weight of less than 100 kDa, less than 50 kDa, or less than 10 kDa.

In another aspect the present invention relates to a pharmaceutical composition comprising a compound according to the invention, and a pharmaceutically acceptable excipient.

In one embodiment the pharmaceutical composition is suited for parenteral administration.

In another aspect the present invention relates to the use of a compound according to the invention for the preparation of a medicament.

In one embodiment of the invention a compound according to the invention wherein the therapeutic polypeptide is a GLP-1 peptide is used for the preparation of a medicament for the treatment or prevention of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, stroke, inflammatory bowel syndrome, dyspepsia and gastric ulcers.

In another embodiment of the invention a compound according to the invention wherein the therapeutic polypeptide is a GLP-1 peptide is used for the preparation of a medicament for delaying or preventing disease progression in type 2 diabetes.

In another embodiment of the invention a compound according to the invention wherein the therapeutic polypeptide is a GLP-1 peptide is used for the preparation of a medicament for decreasing food intake, decreasing 3-cell apoptosis, increasing 3-cell funtion and 3-cell mass, and/or for restoring glucose sensitivity to β-cells.

The therapeutic polypeptide can be produced by a method which comprises culturing a host cell containing a DNA sequence encoding the polypeptide and capable of expressing the polypeptide in a suitable nutrient medium under conditions permitting the expression of the peptide, after which the resulting peptide is recovered from the culture.

The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The peptide produced by the cells may then be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. ion exchange chromatography, gel filtration chromatography, affinity chromatography, or the like, dependent on the type of peptide in question.

The DNA sequence encoding the therapeutic polypeptide may suitably be of genomic or cDNA origin, for instance obtained by preparing a genomic or cDNA library and screening for DNA sequences coding for all or part of the polypeptide by hybridisation using synthetic oligonucleotide probes in accordance with standard techniques (see, for example, Sambrook, J, Fritsch, EF and Maniatis, T, Molecular Cloning: A Laboratory Manual, Cold Spring Harbor Laboratory Press, New York, 1989). The DNA sequence encoding the polypeptide may also be prepared synthetically by established standard methods, e.g. the phosphoamidite method described by Beaucage and Caruthers, Tetrahedron Letters 22 (1981), 1859 - 1869, or the method described by Matthes et al., EMBO Journal 3 (1984), 801 - 805. The DNA sequence may also be prepared by polymerase chain reaction using specific primers, for instance as described in US 4,683,202 or Saiki et al., Science 239 (1988), 487 - 491.

The DNA sequence may be inserted into any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, *e.g.* a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the peptide is operably linked to additional segments required for transcription of the DNA, such as a promoter. The promoter may be any DNA sequence which shows transcriptional activity in the host cell of choice and may be derived from genes encoding proteins either homologous or heterologous to the host cell. Examples of suitable promoters for directing the transcription of the DNA encoding the peptide of the invention in a variety of host cells are well known in the art, cf. for instance Sambrook *et al., supra.*

The DNA sequence encoding the peptide may also, if necessary, be operably connected to a suitable terminator, polyadenylation signals, transcriptional enhancer sequences, and translational enhancer sequences. The recombinant vector of the invention may further comprise a DNA sequence enabling the vector to replicate in the host cell in question.

The vector may also comprise a selectable marker, e.g. a gene the product of which complements a defect in the host cell or one which confers resistance to a drug, e.g. ampicillin, kanamycin, tetracyclin, chloramphenicol, neomycin, hygromycin or methotrexate.

To direct a parent peptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the peptide in the correct reading frame. Secretory signal sequences are commonly positioned 5' to the DNA sequence encoding the peptide. The secretory signal sequence may be that normally associated with the peptide or may be from a gene encoding another secreted protein.

The procedures used to ligate the DNA sequences coding for the present peptide, the promoter and optionally the terminator and/or secretory signal sequence, respectively, and to insert them into suitable vectors containing the information necessary for replication, are well known to persons skilled in the art (cf., for instance, Sambrook *et al.., supra*).

The host cell into which the DNA sequence or the recombinant vector is introduced may be any cell which is capable of producing the present peptide and includes bacteria, yeast, fungi and higher eukaryotic cells. Examples of suitable host cells well known and used in the art are, without limitation, *E. coli, Saccharomyces cerevisiae,* or mammalian BHK or CHO cell lines.

Examples of compounds which can be useful as GLP-1 moieties according to the present invention are described in International Patent Application No. WO 87/06941 (The General Hospital Corporation) which relates to a peptide fragment which comprises GLP-1(7-37) and functional derivatives thereof and to its use as an insulinotropic agent.

Further GLP-1 analogues are described in International Patent Application No. 90/11296 (The General Hospital Corporation) which relates to peptide fragments which comprise GLP-1(7-36) and functional derivatives thereof and have an insulinotropic activity which exceeds the insulinotropic activity of GLP-1(1-36) or GLP-1 (1-37) and to their use as insulinotropic agents.

International Patent Application No. 91/11457 (Buckley et al..) discloses analogues of the active GLP-1 peptides 7-34, 7-35, 7-36, and 7-37 which can also be useful as GLP-1 moieties according to the present invention.

### Pharmaceutical compositions

Pharmaceutical compositions containing a compound according to the present invention may be prepared by conventional techniques, *e.g.* as described in Remington's Pharmaceutical Sciences, 1985 or in Remington: The Science and Practice of Pharmacy, 19th edition, 1995.

One object of the present invention is to provide a pharmaceutical formulation comprising a compound according to the present invention which is present in a concentration from about 0.1 mg/ml to about 25 mg/ml, and wherein said formulation has a pH from 2.0 to 10.0. The pharmaceutical formulation may comprise a compound according to the present invention which is present in a concentration from about 0.1 mg/ml to about 50 mg/ml, and wherein said formulation has a pH from 2.0 to 10.0. The formulation may further comprise a buffer system, preservative(s), isotonicity agent(s), chelating agent(s), stabilizers and surfactants. In one embodiment of the invention the pharmaceutical formulation is an aqueous formulation, i.e. formulation comprising water. Such formulation is typically a solution or a suspension. In a further embodiment of the invention the pharmaceutical formulation is an aqueous solution. The term "aqueous formulation" is defined as a formulation comprising at least 50 %w/w water. Likewise, the term "aqueous solution" is defined as a solution comprising at least 50 %w/w water, and the term "aqueous suspension" is defined as a suspension comprising at least 50 %w/w water.

In another embodiment the pharmaceutical formulation is a freeze-dried formulation, whereto the physician or the patient adds solvents and/or diluents prior to use.

In another embodiment the pharmaceutical formulation is a dried formulation (e.g. freeze-dried or spray-dried) ready for use without any prior dissolution.

In a further aspect the invention relates to a pharmaceutical formulation comprising an aqueous solution of a compound according to the present invention, and a buffer, wherein said compound is present in a concentration from 0.1 mg/ml or above, and wherein said formulation has a pH from about 2.0 to about 10.0.

In a further aspect the invention relates to a pharmaceutical formulation comprising an aqueous solution of a compound according to the present invention, and a buffer, wherein said compound is present in a concentration from 0.1 mg/ml or above, and wherein said formulation has a pH from about 7.0 to about 8.5.

In a another embodiment of the invention the pH of the formulation is selected from the list consisting of 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10.0. Preferably, the pH of the formulation is at least 1 pH unit from the isoelectric point of the compound according to the present invention, even more preferable the pH of the formulation is at least 2 pH unit from the isoelectric point of the compound according to the present invention.

In a further embodiment of the invention the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethane, hepes, bicine, tricine, malic acid, succinate, maleic acid, fumaric acid, tartaric acid, aspartic acid or mixtures thereof. Each one of these specific buffers constitutes an alternative embodiment of the invention.

In a further embodiment of the invention the formulation further comprises a pharmaceutically acceptable preservative. In a further embodiment of the invention the preservative is selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, ethanol, chlorobutanol, and thiomerosal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further embodiment of the invention the preservative is present in a concentration from 0.1 mg/ml to 30 mg/ml. In a further embodiment of the invention the preservative is present in a concentration from 0.1 mg/ml to 20 mg/ml. In a further embodiment of the invention the preservative is present in a concentration from 0.1 mg/ml to 5 mg/ml. In a further embodiment of the invention the preservative is present in a concentration from 5 mg/ml to 10 mg/ml. In a further embodiment of the invention the preservative is present in a concentration from 10 mg/ml to 20 mg/ml. Each one of these specific preservatives constitutes an alternative embodiment of the invention. The use of a preservative in pharmaceutical compositions is well-known to the skilled person. For convenience reference is made to Remington: The Science and Practice of Pharmacy, 19th edition, 1995.

In a further embodiment of the invention the formulation further comprises an isotonic agent. In a further embodiment of the invention the isotonic agent is selected from the group consisting of a salt (e.g. sodium chloride), a sugar or sugar alcohol, an amino acid (e.g. L-glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C4-C8 hydrocarbon having at least one --OH group and includes, for example, mannitol, sorbitol, inositol, galacititol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. In one embodiment, the sugar or sugar alcohol concentration is between about 1 mg/ml and about 150 mg/ml. In a further embodiment of the invention the isotonic agent is present in a concentration from 1 mg/ml to 50 mg/ml. In a further embodiment of the invention the isotonic agent is present in a concentration from 1 mg/ml to 7 mg/ml. In a further embodiment of the invention the isotonic agent is present in a concentration from 8 mg/ml to 24 mg/ml. In a further embodiment of the invention the isotonic agent is present in a concentration from 25 mg/ml to 50 mg/ml. Each one of these specific isotonic agents constitutes an alternative embodiment of the invention. The use of an isotonic agent in pharmaceutical compositions is well-known to the skilled person. For convenience reference is made to Remington: The Science and Practice of Pharmacy, 19th edition, 1995.

In a further embodiment of the invention the formulation further comprises a chelating agent. In a further embodiment of the invention the chelating agent is selected from salts of ethylenediaminetetraacetic acid (EDTA), citric acid, and aspartic acid, and mixtures thereof. In a further embodiment of the invention the chelating agent is present in a concentration from 0.1mg/ml to 5mg/ml. In a further embodiment of the invention the chelating agent is present in a concentration from 0.1mg/ml to 2mg/ml. In a further embodiment of the invention the chelating agent is present in a concentration from 2mg/ml to 5mg/ml. Each one of these specific chelating agents constitutes an alternative embodiment of the invention. The use of a chelating agent in pharmaceutical compositions is well-known to the skilled person. For convenience reference is made to Remington: The Science and Practice of Pharmacy, 19th edition, 1995.

In a further embodiment of the invention the formulation further comprises a stabiliser. The use of a stabilizer in pharmaceutical compositions is well-known to the skilled person. For convenience reference is made to Remington: The Science and Practice of Pharmacy, 19th edition, 1995.

More particularly, compositions of the invention are stabilized liquid pharmaceutical compositions whose therapeutically active components include a polypeptide that possibly exhibits aggregate formation during storage in liquid pharmaceutical formulations. By "aggregate formation" is intended a physical interaction between the polypeptide molecules that results in formation of oligomers, which may remain soluble, or large visible aggregates that precipitate from the solution. By "during storage" is intended a liquid pharmaceutical composition or formulation once prepared, is not immediately administered to a subject. Rather, following preparation, it is packaged for storage, either in a liquid form, in a frozen state, or in a dried form for later reconstitution into a liquid form or other form suitable for administration to a subject. By "dried form" is intended the liquid pharmaceutical composition or formulation is dried either by freeze drying (i.e., lyophilization; see, for example, Williams and Polli (1984) J. Parenteral Sci. Technol. 38:48-59), spray drying (see Masters (1991) in Spray-Drying Handbook (5th ed; Longman Scientific and Technical, Essez, U.K.), pp. 491-676; Broadhead et al. (1992) Drug Devel. Ind. Pharm. 18:1169-1206; and Mumenthaler et al. (1994) Pharm. Res. 11:12-20), or air drying (Carpenter and Crowe (1988) Cryobiology 25:459-470; and Roser (1991) Biopharm. 4:47-53). Aggregate formation by a polypeptide during storage of a liquid pharmaceutical composition can adversely affect biological activity of that polypeptide, resulting in loss of therapeutic efficacy of the pharmaceutical composition. Furthermore, aggregate formation may cause other problems such as blockage of tubing, membranes, or pumps when the polypeptide-containing pharmaceutical composition is administered using an infusion system.

The pharmaceutical compositions of the invention may further comprise an amount of an amino acid base sufficient to decrease aggregate formation by the polypeptide during storage of the composition. By "amino acid base" is intended an amino acid or a combination of amino acids, where any given amino acid is present either in its free base form or in its salt form. Where a combination of amino acids is used, all of the amino acids may be present in their free base forms, all may be present in their salt forms, or some may be present in their free base forms while others are present in their salt forms. In one embodiment, amino acids used for preparing the compositions of the invention are those carrying a charged side chain, such as arginine, lysine, aspartic acid, and glutamic acid. In one embodiment, the amino acid used for preparing the compositions of the invention is glycine. Any stereoisomer (i.e. L or D) of a particular amino acid (e.g. methionine, histidine, imidazole, arginine, lysine, isoleucine, aspartic acid, tryptophan, threonine and mixtures thereof) or combinations of these stereoisomers, may be present in the pharmaceutical compositions of the invention so long as the particular amino acid is present either in its free base form or its salt form. In one embodiment the L-stereoisomer is used. Compositions of the invention may also be formulated with analogues of these amino acids. By "amino acid analogue" is intended a derivative of the naturally occurring amino acid that brings about the desired effect of decreasing aggregate formation by the polypeptide during storage of the liquid pharmaceutical compositions of the invention. Suitable arginine analogues include, for example, aminoguanidine, ornithine and N-monoethyl L-arginine, suitable methionine analogues include ethionine and buthionine and suitable cystein analogues include S-methyl-L cystein. As with the other amino acids, the amino acid analogues are incorporated into the compositions in either their free base form or their salt form. In a further embodiment of the invention the amino acids or amino acid analogues are used in a concentration, which is sufficient to prevent or delay aggregation of the protein.

In a further embodiment of the invention methionine (or other sulphuric amino acids or amino acid analogous) may be added to inhibit oxidation of methionine residues to methionine sulfoxide when the polypeptide acting as the therapeutic agent is a polypeptide comprising at least one methionine residue susceptible to such oxidation. By "inhibit" is intended minimal accumulation of methionine oxidized species over time. Inhibiting methionine oxidation results in greater retention of the polypeptide in its proper molecular form. Any stereoisomer of methionine (L, D or a mixture thereof) can be used. The amount to be added should be an amount sufficient to inhibit oxidation of the methionine residues such that the amount of methionine sulfoxide is acceptable to regulatory agencies. Typically, this means that the composition contains no more than about 10% to about 30% methionine sulfoxide. Generally, this can be achieved by adding methionine such that the ratio of methionine added to methionine residues ranges from about 1:1 to about 1000:1, such as 10:1 to about 100:1.

In a further embodiment of the invention the formulation further comprises a stabiliser selected from the group of high molecular weight polymers or low molecular compounds. In a further embodiment of the invention the stabilizer is selected from polyethylene glycol (e.g. PEG 3350), polyvinylalcohol (PVA), polyvinylpyrrolidone, carboxy-/hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention.

The pharmaceutical compositions may also comprise additional stabilizing agents, which further enhance stability of a therapeutically active polypeptide therein. Stabilizing agents of particular interest to the present invention include, but are not limited to, methionine and EDTA, which protect the polypeptide against methionine oxidation, and a nonionic surfactant, which protects the polypeptide against aggregation associated with freeze-thawing or mechanical shearing.

In a further embodiment of the invention the formulation further comprises a surfactant. In a further embodiment of the invention the surfactant is selected from a detergent, ethoxylated castor oil, polyglycolyzed glycerides, acetylated monoglycerides, sorbitan fatty acid esters, polyoxypropylene-polyoxyethylene block polymers (eg. poloxamers such as Pluronic® F68, poloxamer 188 and 407, Triton X-100), polyoxyethylene sorbitan fatty acid esters, starshaped PEO, polyoxyethylene and polyethylene derivatives such as alkylated and alkoxylated derivatives (tweens, e.g. Tween-20, Tween-40, Tween-80 and Brij-35), polyoxyethylene hydroxystearate, monoglycerides or ethoxylated derivatives thereof, diglycerides or polyoxyethylene derivatives thereof, alcohols, glycerol, lecitins and phospholipids (eg. phosphatidyl serine, phosphatidyl choline, phosphatidyl ethanolamine, phosphatidyl inositol, diphosphatidyl glycerol and sphingomyelin), derivates of phospholipids (eg. dipalmitoyl phosphatidic acid) and lysophospholipids (eg. palmitoyl lysophosphatidyl-L-serine and 1-acyl-sn-glycero-3-phosphate esters of ethanolamine, choline, serine or threonine) and alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (eg. cephalins), glyceroglycolipids (eg. galactopyransoide), sphingoglycolipids (eg. ceramides, gangliosides), dodecylphosphocholine, hen egg lysolecithin, fusidic acid derivatives- (e.g. sodium tauro-dihydrofusidate etc.), long-chain fatty acids and salts thereof C6-C12 (eg. oleic acid and caprylic acid), acylcarnitines and derivatives, N^{α}-acylated derivatives of lysine, arginine or histidine, or side-chain acylated derivatives of lysine or arginine, N^{α}-acylated derivatives of dipeptides comprising any combination of lysine, arginine or histidine and a neutral or acidic amino acid, N^{α}-acylated derivative of a tripeptide comprising any combination of a neutral amino acid and two charged amino acids, DSS (docusate sodium, CAS registry no [577-11-7]), docusate calcium, CAS registry no [128-49-4]), docusate potassium, CAS registry no [7491-09-0]), SDS (sodium dodecyl sulfate or sodium lauryl sulfate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-1-propanesulfonate, anionic (alkyl-aryl-sulphonates) monovalent surfactants, zwitterionic surfactants (e.g. N-alkyl-N,N-dimethylammonio-1-propanesulfonates, 3-cholamido-1-propyldimethylammonio-1-propanesulfonate, cationic surfactants (quarternary ammonium bases) (e.g. cetyltrimethylammonium bromide, cetylpyridinium chloride), non-ionic surfactants (eg. Dodecyl β-D-glucopyranoside), poloxamines (eg. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the invention.

The use of a surfactant in pharmaceutical compositions is well-known to the skilled person. For convenience reference is made to Remington: The Science and Practice of Pharmacy, 19th edition, 1995.

A composition for parenteral administration of GLP-1 compounds may, for example, be prepared as described in WO 03/002136.

It is possible that other ingredients may be present in the peptide pharmaceutical formulation of the present invention. Such additional ingredients may include wetting agents, emulsifiers, antioxidants, bulking agents, tonicity modifiers, chelating agents, metal ions, oleaginous vehicles, proteins (e.g., human serum albumin, gelatin or proteins) and a zwitterion (e.g., an amino acid such as betaine, taurine, arginine, glycine, lysine and histidine). Such additional ingredients, of course, should not adversely affect the overall stability of the pharmaceutical formulation of the present invention.

Pharmaceutical compositions containing a compound according to the present invention may be administered to a patient in need of such treatment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen.

Administration of pharmaceutical compositions according to the invention may be through several routes of administration, for example, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

Compositions of the current invention may be administered in several dosage forms, for example, as solutions, suspensions, emulsions, microemulsions, multiple emulsion, foams, salves, pastes, plasters, ointments, tablets, coated tablets, rinses, capsules, for example, hard gelatine capsules and soft gelatine capsules, suppositories, rectal capsules, drops, gels, sprays, powder, aerosols, inhalants, eye drops, ophthalmic ointments, ophthalmic rinses, vaginal pessaries, vaginal rings, vaginal ointments, injection solution, in situ transforming solutions, for example in situ gelling, in situ setting, in situ precipitating, in situ crystallization, infusion solution, and implants.

Compositions of the invention may further be compounded in, or attached to, for example through covalent, hydrophobic and electrostatic interactions, a drug carrier, drug delivery system and advanced drug delivery system in order to further enhance stability of the compound, increase bioavailability, increase solubility, decrease adverse effects, achieve chronotherapy well known to those skilled in the art, and increase patient compliance or any combination thereof. Examples of carriers, drug delivery systems and advanced drug delivery systems include, but are not limited to, polymers, for example cellulose and derivatives, polysaccharides, for example dextran and derivatives, starch and derivatives, poly(vinyl alcohol), acrylate and methacrylate polymers, polylactic and polyglycolic acid and block copolymers thereof, polyethylene glycols, carrier proteins, for example albumin, gels, for example, thermogelling systems, for example block co-polymeric systems well known to those skilled in the art, micelles, liposomes, microspheres, nanoparticulates, liquid crystals and dispersions thereof, L2 phase and dispersions there of, well known to those skilled in the art of phase behaviour in lipid-water systems, polymeric micelles, multiple emulsions, self-emulsifying, self-microemulsifying, cyclodextrins and derivatives thereof, and dendrimers.

Compositions of the current invention are useful in the formulation of solids, semi-solids, powder and solutions for pulmonary administration of the compound, using, for example a metered dose inhaler, dry powder inhaler and a nebulizer, all being devices well known to those skilled in the art.

Compositions of the current invention are specifically useful in the formulation of controlled, sustained, protracting, retarded, and slow release drug delivery systems. More specifically, but not limited to, compositions are useful in formulation of parenteral controlled release and sustained release systems (both systems leading to a many-fold reduction in number of administrations), well known to those skilled in the art. Even more preferably, are controlled release and sustained release systems administered subcutaneous. Without limiting the scope of the invention, examples of useful controlled release system and compositions are hydrogels, oleaginous gels, liquid crystals, polymeric micelles, microspheres, nanoparticles,

Methods to produce controlled release systems useful for compositions of the current invention include, but are not limited to, crystallization, condensation, co-cystallization, precipitation, co-precipitation, emulsification, dispersion, high pressure homogenization, encapsulation, spray drying, microencapsulation, coacervation, phase separation, solvent evaporation to produce microspheres, extrusion and supercritical fluid processes. General reference is made to Handbook of Pharmaceutical Controlled Release (Wise, D.L., ed. Marcel Dekker, New York, 2000) and Drug and the Pharmaceutical Sciences vol. 99: Protein Formulation and Delivery (MacNally, E.J., ed. Marcel Dekker, New York, 2000).

Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of the compound according to the present invention in the form of a nasal or pulmonal spray. As a still further option, the pharmaceutical compositions containing the compound of the invention can also be adapted to transdermal administration, *e.g.* by needle-free injection or from a patch, optionally an iontophoretic patch, or transmucosal, *e.g.* buccal, administration.

The term "stabilized formulation" refers to a formulation with increased physical stability, increased chemical stability or increased physical and chemical stability.

The term "physical stability" of the protein formulation as used herein refers to the tendency of the protein to form biologically inactive and/or insoluble aggregates of the protein as a result of exposure of the protein to thermo-mechanical stresses and/or interaction with interfaces and surfaces that are destabilizing, such as hydrophobic surfaces and interfaces. Physical stability of the aqueous protein formulations is evaluated by means of visual inspection and/or turbidity measurements after exposing the formulation filled in suitable containers (e.g. cartridges or vials) to mechanical/physical stress (e.g. agitation) at different temperatures for various time periods. Visual inspection of the formulations is performed in a sharp focused light with a dark background. The turbidity of the formulation is characterized by a visual score ranking the degree of turbidity for instance on a scale from 0 to 3 (a formulation showing no turbidity corresponds to a visual score 0, and a formulation showing visual turbidity in daylight corresponds to visual score 3). A formulation is classified physical unstable with respect to protein aggregation, when it shows visual turbidity in daylight. Alternatively, the turbidity of the formulation can be evaluated by simple turbidity measurements well-known to the skilled person. Physical stability of the aqueous protein formulations can also be evaluated by using a spectroscopic agent or probe of the conformational status of the protein. The probe is preferably a small molecule that preferentially binds to a non-native conformer of the protein. One example of a small molecular spectroscopic probe of protein structure is Thioflavin T. Thioflavin T is a fluorescent dye that has been widely used for the detection of amyloid fibrils. In the presence of fibrils, and perhaps other protein configurations as well, Thioflavin T gives rise to a new excitation maximum at about 450 nm and enhanced emission at about 482 nm when bound to a fibril protein form. Unbound Thioflavin T is essentially non-fluorescent at the wavelengths.

Other small molecules can be used as probes of the changes in protein structure from native to non-native states. For instance the "hydrophobic patch" probes that bind preferentially to exposed hydrophobic patches of a protein. The hydrophobic patches are generally buried within the tertiary structure of a protein in its native state, but become exposed as a protein begins to unfold or denature. Examples of these small molecular, spectroscopic probes are aromatic, hydrophobic dyes, such as antrhacene, acridine, phenanthroline or the like. Other spectroscopic probes are metal-amino acid complexes, such as cobalt metal complexes of hydrophobic amino acids, such as phenylalanine, leucine, isoleucine, methionine, and valine, or the like.

The term "chemical stability" of the protein formulation as used herein refers to chemical covalent changes in the protein structure leading to formation of chemical degradation products with potential less biological potency and/or potential increased immunogenic properties compared to the native protein structure. Various chemical degradation products can be formed depending on the type and nature of the native protein and the environment to which the protein is exposed. Elimination of chemical degradation can most probably not be completely avoided and increasing amounts of chemical degradation products is often seen during storage and use of the protein formulation as well-known by the person skilled in the art. Most proteins are prone to deamidation, a process in which the side chain amide group in glutaminyl or asparaginyl residues is hydrolysed to form a free carboxylic acid. Other degradations pathways involves formation of high molecular weight transformation products where two or more protein molecules are covalently bound to each other through transamidation and/or disulfide interactions leading to formation of covalently bound dimer, oligomer and polymer degradation products (Stability of Protein Pharmaceuticals, Ahern. T.J. & Manning M.C., Plenum Press, New York 1992). Oxidation (of for instance methionine residues) can be mentioned as another variant of chemical degradation. The chemical stability of the protein formulation can be evaluated by measuring the amount of the chemical degradation products at various time-points after exposure to different environmental conditions (the formation of degradation products can often be accelerated by for instance increasing temperature). The amount of each individual degradation product is often determined by separation of the degradation products depending on molecule size and/or charge using various chromatography techniques (e.g. SEC-HPLC and/or RP-HPLC).

Hence, as outlined above, a "stabilized formulation" refers to a formulation with increased physical stability, increased chemical stability or increased physical and chemical stability. In general, a formulation must be stable during use and storage (in compliance with recommended use and storage conditions) until the expiration date is reached.

In one embodiment of the invention the pharmaceutical formulation comprising the compound according to the present invention is stable for more than 6 weeks of usage and for more than 3 years of storage.

In another embodiment of the invention the pharmaceutical formulation comprising the compound according to the present invention is stable for more than 4 weeks of usage and for more than 3 years of storage.

In a further embodiment of the invention the pharmaceutical formulation comprising the compound according to the present invention is stable for more than 4 weeks of usage and for more than two years of storage.

In an even further embodiment of the invention the pharmaceutical formulation comprising the compound is stable for more than 2 weeks of usage and for more than two years of storage.

Pharmaceutical compositions containing a GLP-1 derivative according to the present invention may be administered parenterally to patients in need of such a treatment. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of the GLP-1 derivative in the form of a nasal or pulmonal spray. As a still further option, the GLP-1 derivatives of the invention can also be administered transdermally, *e.g.* from a patch, optionally a iontophoretic patch, or transmucosally, *e.g.* bucally.

Thus, the injectable compositions of the GLP-1 derivative of the invention can be prepared using the conventional techniques of the pharmaceutical industry which involves dissolving and mixing the ingredients as appropriate to give the desired end product.

According to one procedure, the GLP-1 derivative is dissolved in an amount of water which is somewhat less than the final volume of the composition to be prepared. An isotonic agent, a preservative and a buffer is added as required and the pH value of the solution is adjusted - if necessary - using an acid, *e.g.* hydrochloric acid, or a base, *e.g.* aqueous sodium hydroxide as needed. Finally, the volume of the solution is adjusted with water to give the desired concentration of the ingredients.

Further to the above-mentioned components, solutions containing a GLP-1 derivative according to the present invention may also contain a surfactant in order to improve the solubility and/or the stability of the GLP-1 derivative.

A composition for nasal administration of certain peptides may, for example, be prepared as described in European Patent No. 272097 (to Novo Nordisk A/S) or in WO 93/18785.

According to one preferred embodiment of the present invention, the GLP-1 derivative is provided in the form of a composition suitable for administration by injection. Such a composition can either be an injectable solution ready for use or it can be an amount of a solid composition, *e.g.* a lyophilised product, which has to be dissolved in a solvent before it can be injected. The injectable solution preferably contains not less than about 2 mg/ml, preferably not less than about 5 mg/ml, more preferred not less than about 10 mg/ml of the GLP-1 derivative and, preferably, not more than about 100 mg/ml of the GLP-1 derivative.

The GLP-1 derivatives of this invention can be used in the treatment of various diseases. The particular GLP-1 derivative to be used and the optimal dose level for any patient will depend on the disease to be treated and on a variety of factors including the efficacy of the specific peptide derivative employed, the age, body weight, physical activity, and diet of the patient, on a possible combination with other drugs, and on the severity of the case. It is recommended that the dosage of the GLP-1 derivative of this invention be determined for each individual patient by those skilled in the art.

In particular, it is envisaged that the GLP-1 derivative will be useful for the preparation of a medicament with a protracted profile of action for the treatment of non-insulin dependent diabetes mellitus and/or for the treatment of obesity.

In another aspect the present invention relates to the use of a compound according to the invention for the preparation of a medicament.

In one embodiment the present invention relates to the use of a compound according to the invention for the preparation of a medicament for the treatment of hyperglycemia, type 2 diabetes, impaired glucose tolerance, type 1 diabetes, obesity, hypertension, syndrome X, dyslipidemia, β-cell apoptosis, 3-cell deficiency, myocardial infarction, inflammatory bowel syndrome, dyspepsia, cognitive disorders, e.g. cognitive enhancing, neuroprotection, atheroschlerosis, coronary heart disease and other cardiovascular disorders.

In another embodiment the present invention relates to the use of a compound according to the invention for the preparation of a medicament for the treatment of small bowel syndrome, inflammatory bowel syndrome or Crohns disease.

In another embodiment the present invention relates to the use of a compound according to the invention for the preparation of a medicament for the treatment of hyperglycemia, type 1 diabetes, type 2 diabetes or 3-cell deficiency.

The treatment with a compound according to the present invention may also be combined with combined with a second or more pharmacologically active substances, e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. In the present context the expression "antidiabetic agent" includes compounds for the treatment and/or prophylaxis of insulin resistance and diseases wherein insulin resistance is the pathophysiological mechanism.

Examples of these pharmacologically active substances are : Insulin, GLP-1 agonists, sulphonylureas (e.g. tolbutamide, glibenclamide, glipizide and gliclazide), biguanides e.g. metformin, meglitinides, glucosidase inhibitors (e.g. acorbose), glucagon antagonists, DPPIV (dipeptidyl peptidase-IV) inhibitors, inhibitors of hepatic enzymes involved in stimulation of gluconeogenesis and/or glycogenolysis, glucose uptake modulators, thiazolidinediones such as troglitazone and ciglitazone, compounds modifying the lipid metabolism such as antihyperlipidemic agents as HMG CoA inhibitors (statins), compounds lowering food intake, RXR agonists and agents acting on the ATP-dependent potassium channel of the β-cells, e.g. glibenclamide, glipizide, gliclazide and repaglinide; Cholestyramine, colestipol, clofibrate, gem-fibrozil, lovastatin, pravastatin, simvastatin, probucol, dextrothyroxine, neteglinide, repaglinide; β-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TR β agonists; histamine H3 antagonists.

It should be understood that any suitable combination of the compounds according to the invention with one or more of the above-mentioned compounds and optionally one or more further pharmacologically active substances are considered to be within the scope of the present invention.

The present invention is further illustrated by the following examples. The features disclosed in the foregoing description and in the following examples may, both separately and in any combination thereof, be material for realising the invention in diverse forms thereof.

### EXAMPLES

The following acronyms for commercially available chemicals are used:
DMF N,N-Dimethylformamide.
DCC N,N-Dicyclohexylcarbodiimide
NMP N-Methyl-2-pyrrolidone.
TFA Trifluoroacetic acid.
THF Tetrahydrofuran
DIEA diisopropylethylamine
H₂O water
CH₃CN : acetonitrile
HBTU 2-(1H-Benzotriazol-1-yl)-1,1,3,3 tetramethyluronium hexafluoro-phosphate
Fmoc 9 H-fluoren-9-ylmethoxycarbonyl
Boc tert butyloxycarbonyl
OtBu tert butyl ester
tBu tert butyl
Trt triphenylmethyl
Pmc 2,2,5,7,8-Pentamethyl-chroman-6-sulfonyl
Dde 1-(4,4-Dimethyl-2,6-dioxocyclohexylidene)ethyl
DCM dichloromethane
TIS triisopropylsilane)
Et₂O: diethylether
H-Glu(OH)-OBu^{t}: : L-Glutamic acid α-tert-butyl ester
HOOC-(CH₂)₁₂-COONSu: ω-Carboxytridecanoic acid 2,5-dioxopyrrolidin-1-yl ester.
HOOC-(CH₂)₁₄-COONSu: ω-Carboxypentadecanoic acid 2,5-dioxopyrrolidin-1-yl ester.
HOOC-(CH₂)₁₆-COONSu: ω-Carboxyheptadecanoic acid 2,5-dioxopyrrolidin-1-yl ester.
HOOC-(CH₂)₁₈-COONSu: ω-Carboxynonadecanoic acid 2,5-dioxopyrrolidin-1-yl ester.

### Abbreviations:

- r.t: Room temperature
- PDMS:: Plasma Desorption Mass Spectrometry
- MALDI-MS:: Matrix Assisted Laser Desorption/lonisation Mass Spectrometry
- HPLC:: High Performance Liquid Chromatography
- amu:: atomic mass units

### Analytical:

Resistance of a peptide to degradation by dipeptidyl aminopeptidase IV is determined by the following degradation assay :
Aliquots of the peptides are incubated at 37 °C with an aliquot of purified dipeptidyl aminopeptidase IV for 4-22 hours in an appropriate buffer at pH 7-8 (buffer not being albumin). Enzymatic reactions are terminated by the addition of trifluoroacetic acid, and the peptide degradation products are separated and quantified using HPLC or LC-MS analysis. One method for performing this analysis is: The mixtures are applied onto a Zorbax 300SB-C18 (30 nm pores, 5 µm particles) 150 x 2.1 mm column and eluted at a flow rate of 0.5 ml/min with a linear gradient of acetonitrile in 0.1% trifluoroacetic acid (0% -100% acetonitrile over 30 min). Peptides and their degradation products may be monitored by their absorbance at 214 nm (peptide bonds) or 280 nm (aromatic amino acids), and are quantified by integration of their peak areas. The degradation pattern can be determined by using LC-MS where MS spectra of the separated peak can be determined. Percentage intact/degraded compound at a given time is used for estimation of the peptides DPPIV stability.
A peptide is defined as DPPIV stabilised when it is 10 times more stable than the natural peptide based on percentage intact compound at a given time. Thus, a DPPIV stabilised GLP-1 compound is at least 10 times more stable than GLP-1(7-37).

### General Synthetic methods

The peptides may be synthesized on Fmoc protected Rink amide resin (Novabiochem) or chlorotrityl resin or a similar resin suitable for solid phase peptide synthesis. Boc chemistry may be used but more conveinient is using Fmoc strategy eventually on an Applied Biosystems 433A peptide synthesizer in 0.25 mmol scale using the FastMoc UV protocols which employ HBTU (2-(1H-Benzotriazol-1-yl)-1,1,3,3 tetramethyluronium hexafluorophosphate) mediated couplings in N-methyl pyrrolidone (N-methyl pyrrolidone) (HATU is better suited for hindered couplings) and UV monitoring of the deprotection of the Fmoc protection group. Other coupling reagents besides from HBTU and HATU as described in e.g. Current Opinion in Chemical Biology, 2004, 8:211-221 may also be used. The protected amino acid derivatives used may be standard Fmoc-amino acids supplied in preweighed cartridges (Applied Biosystems) suitable for the ABI433A synthesizer with the exception of unnatural aminoacids such as Fmoc-Aib-OH (Fmoc-aminoisobutyric acid) which are purchased from a supplier such as Bachem and transferred to empty cartridges. The last amino acid coupled may be Boc protected.

The attachment of sidechains and linkers to specific lysine residues on the crude resin bound protected peptide may eventually beintroduced in a specific position by incorporation of Fmoc-Lys(Dde)-OH during automated synthesis followed by selective deprotection with hydrazine. Other orthogonal protecting groups may be used on Lysine.

Procedure for removal of Dde-protection. The resin (0.25 mmol) may be placed placed in a manual shaker/filtration apparatus and treated with 2% hydrazine in N-methyl pyrrolidone (20 ml, 2x12 min) to remove the DDE group and subsequently washed with N-methyl pyrrolidone (4x20 ml).

### Procedure for attachment of sidechains to Lysine residues.

The amino acid (4 molar equivalents relative to resin) may be dissolved in N-methyl pyrrolidone/methylene chloride (1:1, 10 ml). Hydroxybenzotriazole (HOBt) (4 molar equivalents relative to resin) and diisopropylcarbodiimide (4 molar equivalents relative to resin) is added and the solution was stirred for 15 min. The solution is added to the resin and diisopropy-ethylamine (4 molar equivalents relative to resin) is added. The resin is shaken 24 hours at room temperature. The resin is washed with N-methyl pyrrolidone (2x20 ml), N-methyl pyrrolidone/Methylene chloride (1:1) (2x20ml) and methylene chloride (2x20 ml).

Procedure for removal of Fmoc-protection: The resin (0.25 mmol) is placed in a filter flask in a manual shaking apparatus and treated with N-methyl pyrrolidone/methylene chloride (1:1) (2x20 ml) and with N-methyl pyrrolidone (1x20 ml), a solution of 20% piperidine in N-methyl pyrrolidone (3x20 ml, 10 min each). The resin is washed with N-methyl pyrrolidone (2x20 ml), N-methyl pyrrolidone/methylene chloride (1:1) (2x20ml) and methylene chloride (2x20 ml).

### Procedure for cleaving the peptide off the resin:

The peptide is cleaved from the resin by stirring for 180 min at room temperature with a mixture of trifluoroacetic acid, water and triisopropylsilane (95:2.5:2.5). The cleavage mixture is filtered and the filtrate is concentrated to an oil by a stream of nitrogen. The crude peptide is precipitated from this oil with 45 ml diethyl ether and washed 3 times with 45 ml diethyl ether.

Purification: The crude peptide may be purified by semipreparative HPLC on a 20 mm x 250 mm column packed with 7µ C-18 silica. Depending on the peptide one or two purification systems may used:
Ammonium sulphate: The column is equilibrated with 40% CH₃CN in 0.05M (NH₄)₂SO₄, which is adjusted to pH 2.5 with concentrated H₂SO₄. After drying the crude peptide is dissolved in 5 ml 50% acetic acid H₂O and diluted to 20 ml with H₂O and injected on the column which then is eluted with a gradient of 40% - 60% CH₃CN in 0.05M (NH₄)₂SO₄, pH 2.5 at 10 ml/min during 50 min at 40 °C. The peptide containing fractions is collected and diluted with 3 volumes of H₂O and passed through a Sep-Pak® C18 cartridge (Waters part. #:51910) which has been equilibrated with 0.1% TFA. It is then eluted with 70% CH₃CN containing 0.1% TFA and the purified peptide is isolated by lyophilisation after dilution of the eluate with water.

TFA: After drying the crude peptide is dissolved in 5 ml 50% acetic acid H₂O and diluted to 20 ml with H₂O and injected on the column which then is eluted with a gradient of 40-60 % CH₃CN in 0.1% TFA 10 ml/min during 50 min at 40 °C. The peptide containing fractions is collected. The purified peptide is lyophilized after dilution of the eluate with water.
The final product obtained may be characterised by analytical RP-HPLC (retention time) and by LCMS .

The RP-HPLC analysis performed in these in the experimental section was performed using UV detection at 214 nm and a Vydac 218TP54 4.6mm x 250mm 5µ C-18 silica column (The Separations Group, Hesperia, USA) which was eluted at 1 ml/min at 42 °C. The different elution conditions were:

| | |
|---|---|
| A1: | Equilibration of the column with in a buffer consisting of 0.1 M (NH₄)₂SO₄, which was adjusted to pH 2.5 with concentrated H₂SO₄ and elution by a gradient of 0% to 60% CH₃CN in the same buffer during 50 min. |
| B1: | Equilibration of the column with 0.1% TFA / H₂O and elution by a gradient of 0% CH₃CN / 0.1% TFA / H₂O to 60% CH₃CN /0.1% TFA / H₂O during 50 min. |
| B6: | Equilibration of the column with 0.1% TFA / H₂O and elution by a gradient of 0% CH₃CN / 0.1% TFA / H₂O to 90% CH₃CN / 0.1% TFA / H₂O during 50 min. |

An alternative system was:

| | |
|---|---|
| B4 : | The RP-analyses was performed using a Alliance Waters 2695 system fitted with a Waters 2487 dualband detector. UV detections at 214nm and 254nm were collected using a Symmetry300 C18 , 5 um, 3.9 mm x 150 mm column, 42 °C. Eluted with a linear gradient of 5-95% acetonitrile, 90-0% water, and 5% trifluoroacetic acid (1.0%) in water over 15 minutes at a flow-rate of 1.0 min/min. |

LCMS was performed on a setup consisting of Hewlett Packard series 1100 G1312A Bin Pump, Hewlett Packard series 1100 Column compartment, Hewlett Packard series 1100 G1315A DAD diode array detector, Hewlett Packard series 1100 MSD and Sedere 75 Evaporative Light Scattering detectorcontrolled by HP Chemstation software. The HPLC pump is connected to two eluent reservoirs containing:
A: 0.05% TFA/water
B: 0.05%TFA/acetonitrile
Or alternatively the two systems may be:
A: 10mM NH₄OH in water
B: 10mM NH₄OH in 90% acetonitrile

The analysis was performed at 23° C by injecting an appropriate volume of the sample (preferably 20 µl) onto the column which is eluted with a gradient of A and B.
The HPLC conditions, detector settings and mass spectrometer settings used are giving in the following table.
Column Waters Xterra MS C-18 (50 X 3 mm id 5 µm)
Gradient 5% - 100% acetonitrile linear during 6.5 min at 1.5ml/min
Detection 210 nm (analogue output from DAD)
ELS (analogue output from ELS)
MS ionisation mode API-ES. Scan 550-1500 amu step 0.1 amu

Alternatively, LC-MS analysis could be performed on a PE-Sciex API 100 mass spectrometer equipped with two Perkin Elmer Series 200 Micropumps, a Perkin Elmer Series 200 autosampler, a Applied Biosystems 785A UV detector and a Sedex 75 Evaporative Light scattering detector. A Waters Xterra 3.0 mm x 50 mm 5µ C-18 silica column was eluted at 1.5 ml/min at room temperature. It was equilibrated with 5 % CH₃CN / 0.05% TFA / H₂O and eluted for 1.0 min with 5% CH₃CN / 0.05% TFA / H₂O and then with a linear gradient to 90% CH₃CN / 0.05% TFA / H₂O over 7 min. Detection was by UV detection at 214nm and Evaporative light Scattering. A fraction of the column eluate was introduced into the ionspray interface of a PE-Sciex API 100 mass spectrometer. The mass range 300 - 2000 amu was scanned every 2 seconds during the run.

MALDI-TOF MS analysis was carried out using a Voyager RP instrument (PerSeptive Biosystems Inc., Framingham, MA) equipped with delayed extraction and operated in linear mode. Alpha-cyano-4-hydroxy-cinnamic acid was used as matrix, and mass assignments were based on external calibration.

### Reference example 1

### N^{ε37}-(2-(2-(2-(dodecylamino)ethoxy)ethoxy)acetyl)-[Aib^{8,22,35},LyS³⁷]GLP-1(7-37)amide

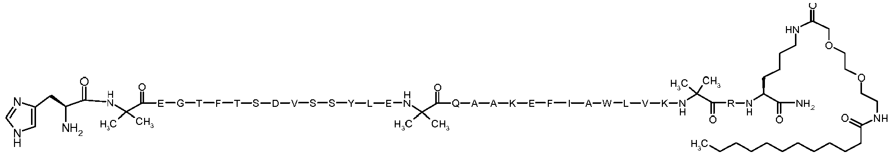

A resin (Rink amide, 0.68 mmol/g Novabiochem 0.25 mmole) was used to produce the primary sequence on an ABI433A machine according to manufacturers guidelines. All protecting groups were acid labile with the exception of the residue used in position 37 (FmocLys(ivDde)-OH, Novabiochem) allowing specific deprotection of this lysine rather than any other lysine.

### Procedure

The above prepared resin (0.25 mmole) containing the GLP-1 analogue amino acid sequence was placed in a manual shaker/filtration apparatus and treated with 2% hydrazine in N-methyl pyrrolidone in (2x12 min. 2x20 ml) to remove the Dde group. The resin was washed with N-methyl pyrrolidone (4x20 ml). Fmoc-8-amino-3,6-dioxaoctanoic acid (Neosystem FA03202) (4 molar equivalents relative to resin) was dissolved in N-methyl pyrrolidone/methylene chloride (1:1, 20 ml). Hydroxybenzotriazole (HOBt) (4 molar equivalents relative to resin) and diisopropylcarbodiimide (4 molar equivalents relative to resin) was added and the solution was stirred for 15 min. The solution was added to the resin and diisopropylethylamine (4 molar equivalents relative to resin) was added. The resin was shaken 24 hours at room temperature. The resin was washed with N-methyl pyrrolidone (4x20 ml). A solution of 20% piperidine in N-methyl pyrrolidone (3x20 ml, 10 min each) was added to the resin while shaking. The resin was washed with N-methyl pyrrolidone (4x20 ml). Dodecanoic acid (4 molar equivalents relative to resin) was dissolved in N-methyl pyrrolidone/methylene chloride (1:1, 20 ml). Hydroxybenzotriazole hydrate (HOBt; H₂O) (4 molar equivalents relative to resin) and diisopropylcarbodiimide (4 molar equivalents relative to resin) were added and the solution was stirred for 15 min. The solution was added to the resin and diisopropylethylamine (4 molar equivalents relative to resin) was added. The resin was shaken 24 hours at room temperature. The resin was washed with N-methyl pyrrolidone (2x20 ml), N-methyl pyrrolidone/methylene chloride (1:1) (2x20ml) and methylene chloride (2x20 ml). The peptide was cleaved from the resin by stirring for 180 min at room temperature with a mixture of trifluoroacetic acid, water and triisopropylsilane (95:2.5:2.5 15 ml). The cleavage mixture was filtered and the filtrate was concentrated to an oil in vaccuum. The crude peptide was precipitated from this oil with 45 ml diethyl ether and washed 3 times with 45 ml diethyl ether. The crude peptide was purified by preparative HPLC on a 20 mm x 250 mm column packed with 7µ C-18 silica. The crude peptide was dissolved in 5 ml 50% acetic acid in water and diluted to 20 ml with H₂O and injected on the column which then was eluted with a gradient of 40-60 % (CH₃CN in water with 0.1% TFA) 10 ml/min during 50 min at 40 °C. The peptide containing fractions were collected. The purified peptide was lyophilized after dilution of the eluate with water.
HPLC: (method B6): RT=32.8 min
HPLC: (method A1): RT=43.6 min
LCMS: m/z = 765.0 (M+5H)⁵⁺, 957.0 (M+4H)⁴⁺, 1275.0 (M+3H)³⁺. Calculated (M+H)⁺ = 3825.0

### Reference example 2

### N^{ε37}-(2-(2-(2-(17-sulphohexadecanoylamino)ethoxy)ethoxy)acetyl)-[Aib^{8,22,35},Lys³⁷] GLP-1 (7-37)amide

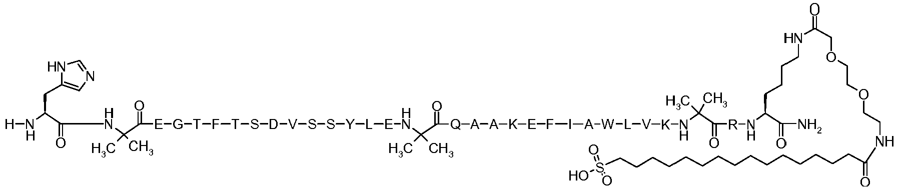

The compound was prepared according to the methods in Example 1 and in "General Synthetic methods".
HPLC: (method A1): RT=45.5 min
LCMS: m/z = 792.9 (M+5H)⁵⁺, 990.9 (M+4H)⁴⁺, 1320.9 (M+3H)³⁺ Calculated (M+H)⁺ = 3959.9

### Example 3

### N^{ε37}-{2-[2-(2-(15-carboxypentadecanoylamino)ethoxy)ethoxy]acetyl}-[Aib^{8,22,35}LyS³⁷] GLP-1(7-37)amide

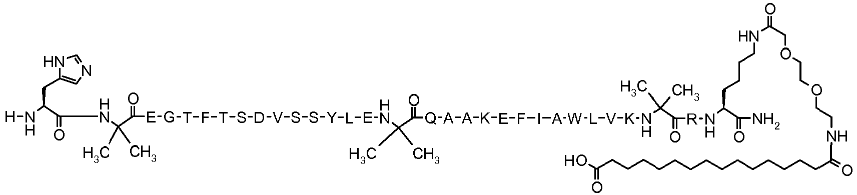

The compound was prepared according to the methods in Example 1 and in "General Synthetic methods".
HPLC: (method B1): RT=43.8 min
HPLC: (method A1): RT=42.0 min
LCMS: m/z = 978.3 (M+4H)⁴⁺, 1303.8 (M+3H)³⁺ Calculated (M+H)⁺ = 3909.6

### Example 4

### N^{ε37}-(2-(2-(2-(17-carboxyheptadecanoylamino)ethoxy)ethoxy)acetyl)[Aib^{8,22,35},Lys³⁷]GLP-1(7-37)amide

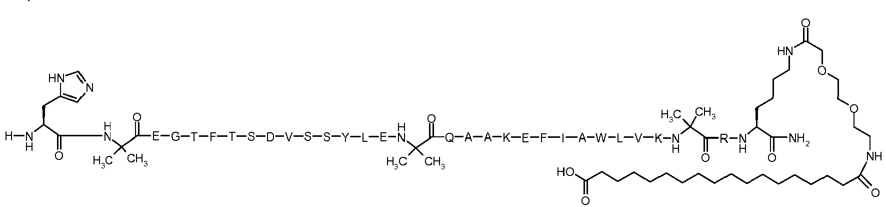

Prepared according to the methods in Example 1 and in "General Synthetic methods".
HPLC: (method B1): RT=46.4 min
HPLC: (method A1): RT=44.4 min
LCMS: m/z = 985.5 (M+4H)⁴⁺, 1313.4 (M+3H)³⁺ Calculated (M+H)⁺ = 3937.6

### Example 5

### N^{ε37}-(2-(2-(2-(19-carboxynonadecanoylamino)ethoxy)ethoxy)acetyl)[Aib^{8,22,35},Lys³⁷]GLP-1(7-37)amide

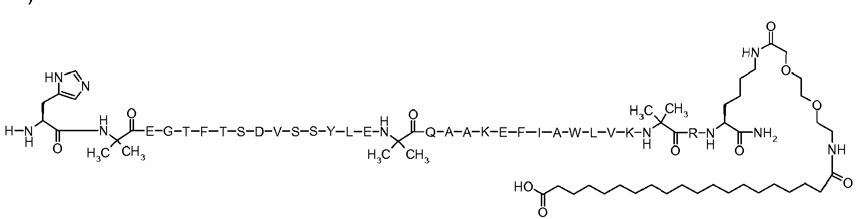

The compound was prepared according to the methods in Example 1 and in "General Synthetic methods".
HPLC: (method B1): RT=49.5 min
HPLC: (method A1): RT=47.1 min
LCMS: m/z = 992.5 (M+4H)⁴⁺, 1322.6 (M+3H)³⁺ Calculated (M+H)⁺ = 3965.7

### Reference example 6

### [Aib^{8,22,35}Arg^{26,34}]GLP-1-(7-37)Lys(4-(Hexadecanoylamino)-4(S)-carboxybutyryl)-OH

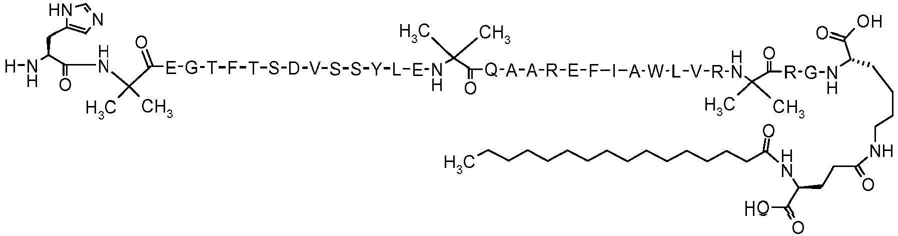

The compound was prepared according to the methods in Example 1 and in "General Synthetic methods".
HPLC: (method B6): RT= 36.28min
LCMS: m/z = 995 (M+4H)⁴⁺, 1326 (M+3H)³⁺ Calculated (M+H)⁺ = 3977.6

### Reference example 7

### [Aib^{8,22,35}Arg^{26,34}]GLP-1-(7-37)Lys(2-(2-(2-(hexadecanoylamino)ethoxy)ethoxy)acetyl)-OH

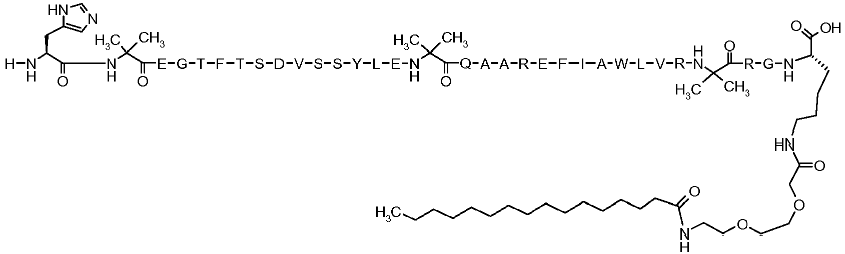

The compound was prepared according to the methods in Example 1 and in "General Synthetic methods".
HPLC: (method B6): RT=37.1min
LCMS: m/z = 999 (M+4H)⁴⁺, 1332 (M+3H)³⁺ Calculated (M+H)⁺ = 3993.7

Reference example **8**

### N^{ε37}-(2-[2-(2,6-(S)-Bis-{2-[2-(2-(dodecanoylamino)ethoxy)ethoxy]acetylamino}hexanoylamino)ethoxy]ethoxy}) acetyl-[Aib^{8,22,35}]GLP-1(7-37)amide

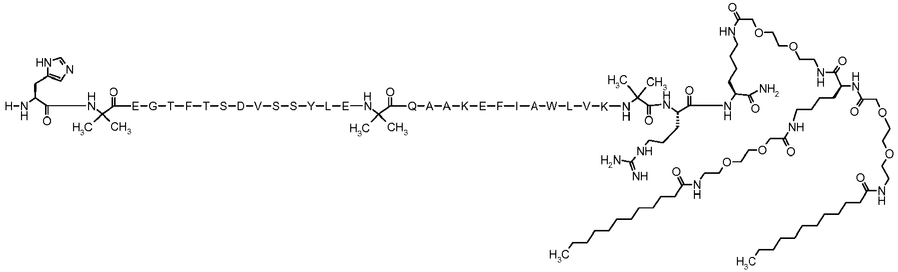

The compound was prepared according to the methods in Example 1 and in "General Synthetic methods".
HPLC: (method B6): RT=38.2 min
LCMS: m/z = 1106.7 (M+4H)⁴⁺, 1475.3 (M+3H)³⁺ Calculated (M+H)⁺ = 4433.0

### Reference example 9

### N^{ε37}-(2-[2-(2,6-(S)-Bis-{2-[2-(2-(tetradecanoylamino)ethoxy)ethoxy]acetylamino}hexanoylamino)ethoxy]ethoxy}) acetyl-[Aib^{8,22,35}]GLP-1(7-37)amide

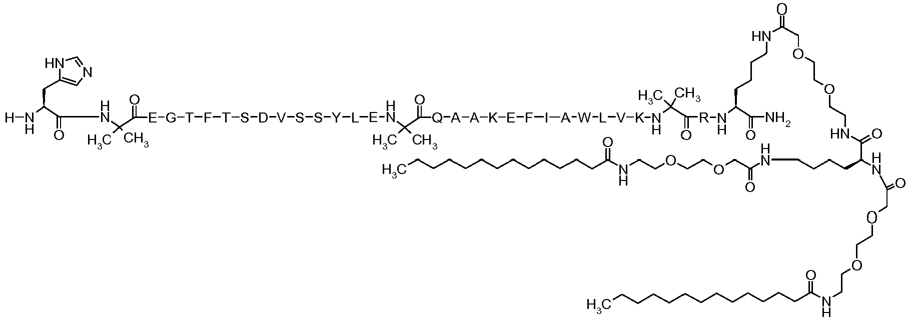

The compound was prepared according to the methods in Example 1 and in "General Synthetic methods".
HPLC: (method B6): RT=42.9 min
LCMS: m/z = 1120.9 (M+4H)⁴⁺, 1494.2 (M+3H)³⁺ Calculated (M+H)⁺ = 4480.4

### Reference example 10

### [Aib^{8,22,35}Arg^{26,34}]GLP-1-(7-37)Lys(2-(2-(2-(4-(Hexadecanoylamino)-4(S)-carboxybutyrylamino)ethoxy)ethoxy)acetyl)-OH

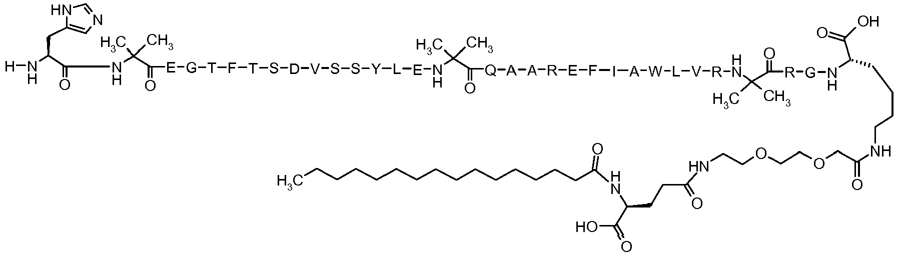

The compound was prepared according to the methods in Example 1 and in "General Synthetic methods".
HPLC: (method B6): RT=36.0 min
LCMS: m/z = 1032.0 (M+4H)⁴⁺, 1374.0 (M+3H)³⁺ Calculated (M+H)⁺ = 4122.8

### Reference example 11

### [Aib^{8,22,35}]GLP-1(7-37)Lys((2-{2-[4-[4-(4-Amino-9,10-dioxo-3-sulfo-9,10-dihydro-anthracen-1-ylamino)-2-sulfo-phenylamino]-6-(2-sulfo-phenylamino)-[1,3,5]triazin-2-ylamino]-ethoxy}-ethoxy)-acetyl))amide

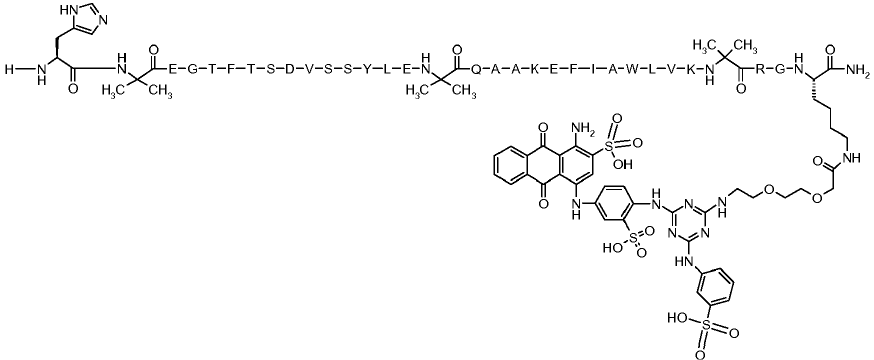

Prepared by loading DdeLys(Fmoc)-OH onto Rink resin. The resin was then treated with piperidine as in "Synthetic methods" to remove Fmoc selectively. 2-(2-(2-(Fmoc-amino)ethoxy)ethoxy)acetic acid was coupled onto the epsilon amingroup of lysine and Fmoc was removed. DMSO and Cibacron Blue 3GA (17 equivalents) (Sigma C-9534) was added and the mixture was heated at 60 °C for 15 hours, washed with water (3 times), methanol (2 times), THF (2 times) and diethyl ether (2 times). The Dde protecting group was removed and the remaining amino acids were added as in "Synthetic methods"
HPLC: (method A1): RT=38.1 min
LCMS: m/z = 1110.4 (M+4H)⁴⁺, 1436.4 (M+3H)³⁺ Calculated (M+H)⁺ = 4435.9

### Example 12

### [Aib^{8,22,35}]GLP-1(7-37)Lys(({2-[2-(2-{2-[2-(2-{2-[2-(15-carboxypentadecanoylamino)-ethoxy]ethoxy}acetylamino)ethoxy]ethoxy}acetyl amino)ethoxy]ethoxy}acetyl))amide

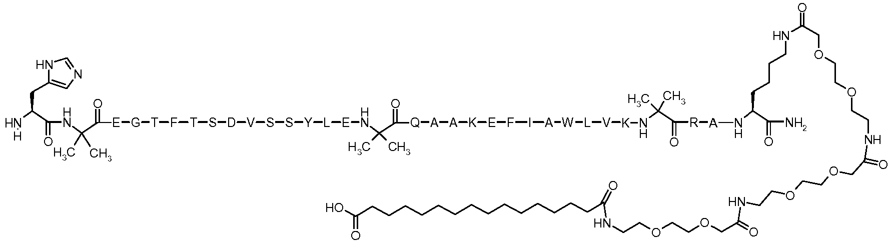

The compound was prepared according to the methods in Example 1 and in "General Synthetic methods".
HPLC: (method A1): RT=41.2 min
HPLC: (method B6): RT=30.7 min
LCMS: m/z = 1069.1 (M+4H)⁴⁺, 1424.6 (M+3H)³⁺ Calculated (M+H)⁺ = 4271

### Example 13

### N^{ε37}-([2-(2-{3-[2,5-dioxo-3-(15-carboxypentadecylsulfanyl)-pyrrolidin-1-yl]-propionylamino}ethoxy)ethoxy)acetyl]-[D-Ala⁸,Lys³⁷]-GLP-1-[7-37]amide

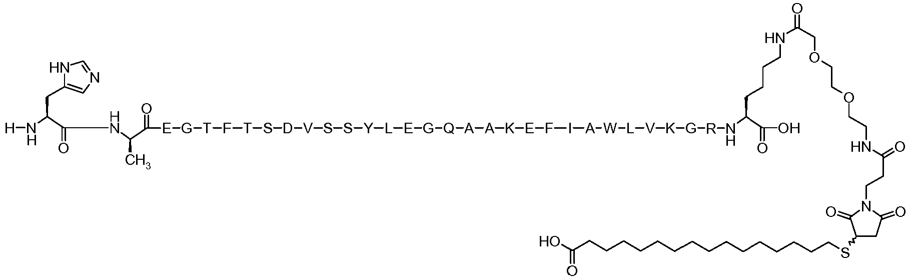

The compound was prepared according to the methods in Example 1 and in "General Synthetic methods".
HPLC: (method A1): RT=45.2 min
LCMS: m/z = 1004.0 (M+4H)⁴⁺, 1338.2 (M+3H)³⁺ Calculated (M+H)⁺ = 4010.7

### Example 14

### [Aib^{8,22,35}Ala³⁷]GLP-1(7-37)Lys((2-(2-(2-(11-(oxalylamino)undecanoylamino)ethoxy)ethoxy)acetyl-)))amide

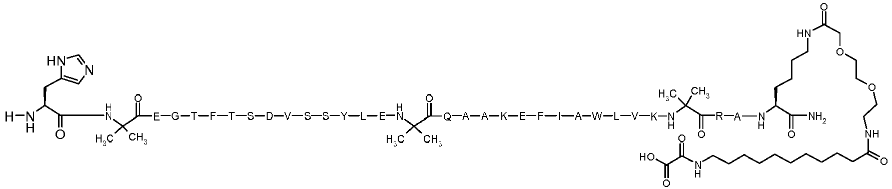

The compound was prepared according to the methods in Example 1 and in "General Synthetic methods".
HPLC (method A1): RT=37.9 min
HPLC (method B1): RT=39.5 min
LCMS: m/z = 993.3 (M+4H)⁴⁺, 1323.9 (M+3H)³⁺ Calculated (M+H)⁺ = 3967.6

### Example 15

### [Aib^{8,22,35},Ala³⁷]-GLP-1(7-37)Lys({2-[2-(2-{2-[2-(2-(15-carboxy-pentadecanoylamino)-ethoxy]ethoxy}acetylamino)ethoxy]ethoxy}acetyl)amide

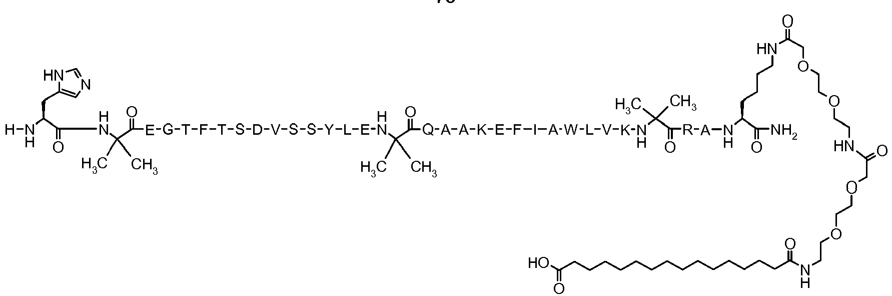

The compound was prepared according to the methods in Example 1 and in "General Synthetic methods".
HPLC (method B6): RT=31.1 min
HPLC (method A1): RT=41.9 min
LCMS: m/z = 1376.3 (M+3H)³⁺ Calculated (M+H)⁺ = 4125.8

### Reference example 16

### [Aib^{8,22,35},Ala³⁷]-GLP-1(7-37)Lys((2-{2-[11-(5-Dimethylaminonaphthalene-1-sulfonylamino)undecanoylamino]ethoxy}ethoxy)acetyl)amide

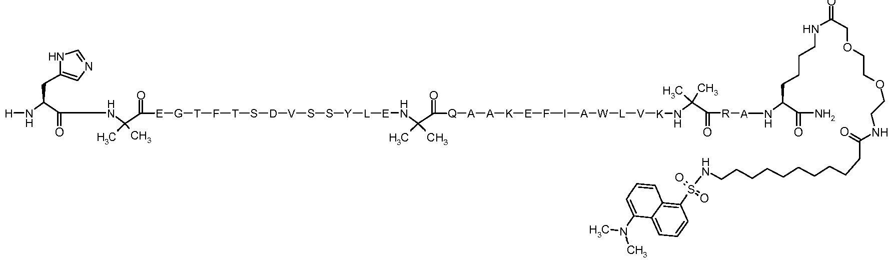

The compound was prepared according to the methods in Example 1 and in "General Synthetic methods".
HPLC (method A1): RT=42.6 min
HPLC (method B6): RT=30.4 min
LCMS: m/z = 1377.3 (M+3H)³⁺ Calculated (M+H)⁺ = 4128.8

### Reference example 17

### [Aib^{8,22,35},Ala³⁷]-GLP-1(7-37)Lys(([2-(2-{2-[1-(4-Chlorobenzoyl)-5-methoxy-2-methyl-1H-indol-3-yl]acetylamino}ethoxy)ethoxy]acetyl))amide

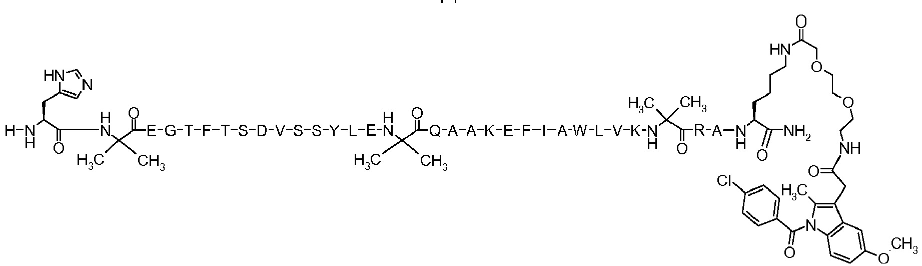

The compound was prepared according to the methods in Example 1 and in "General Synthetic methods".
HPLC (method A1): RT=41.1 min
HPLC (method B6): RT=31.1 min
LCMS: m/z = 1351.8 (M+3H)³⁺ Calculated (M+H)⁺ = 4052.0

### Reference example 18

### [Aib⁸,Arg^{26,34},Glu^{22,23,30}]GLP-1H(7-37)Lys(2-(2-(2-(octadecanoylamino)ethoxy)ethoxy)acetyl)amide

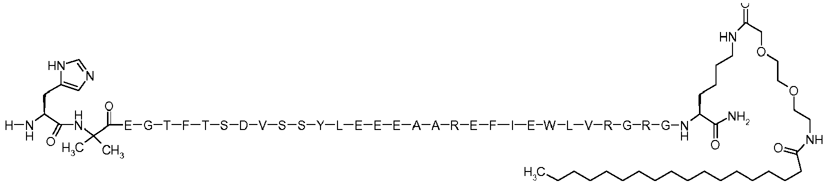

The compound was prepared according to the methods in Example 1 and in "General Synthetic methods".
HPLC (method B6): RT=39.3 min
LCMS: m/z = 1366.6 (M+3H)³⁺ Calculated (M+H)⁺ = 4095.6

### Reference example 19

### [Aib⁸,Arg^{26,34},Glu^{22,23,30}]GLP-1(7-37)Lys(2-(2-(2-(eicosanoylamino)ethoxy)ethoxy)acetyl)amide

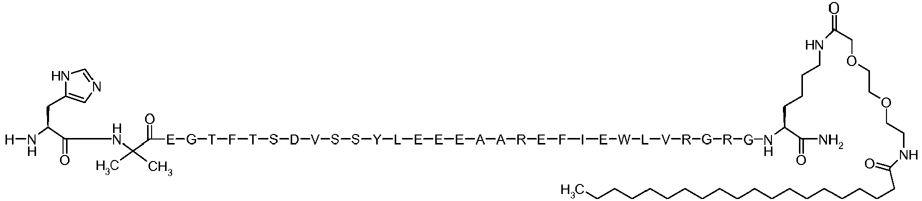

The compound was prepared according to the methods in Example 1 and in "General Synthetic methods".
HPLC (method B6): RT=42.6 min
LCMS: m/z = 1375.7 (M+3H)³⁺ Calculated (M+H)⁺ = 4123.7

### Reference example 20

### [Gly⁸,Arg^{26,34}] GLP-1 H-(7-37)Lys(2-(2-(2-(2-(2-(2-(4-(octadecanoylamino)-4(S)-carboxybutyrylamino)ethoxy)ethoxy)acetyl)ethoxy)ethoxy)acetyl)-OH

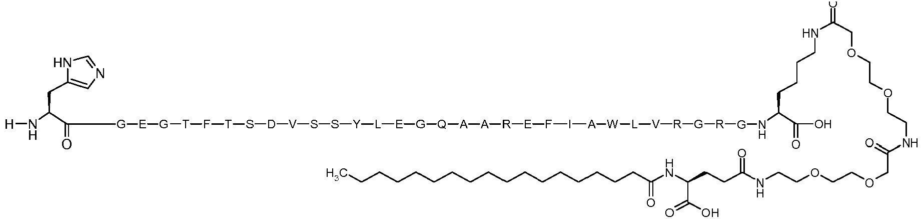

The compound was prepared according to the methods in Example 1 and in "General Synthetic methods".
HPLC (method B6): RT=38.0 min (99.9%)
HPLC (method A1): RT=49.0 min
LCMS: m/z = 1054.6 (M+4H)⁴⁺ 1405.3(M+3H)³⁺ Calculated (M+H)⁺ = 4211.8

### Reference example 21

### [Aib⁸,Arg^{26,34}]GLP-1 (7-37)Lys{2-(2-(2-(2-[2-(2-(octadecanoylamino)ethoxy)ethoxy]acetyl)ethoxy)ethoxy)acetyl)}-OH

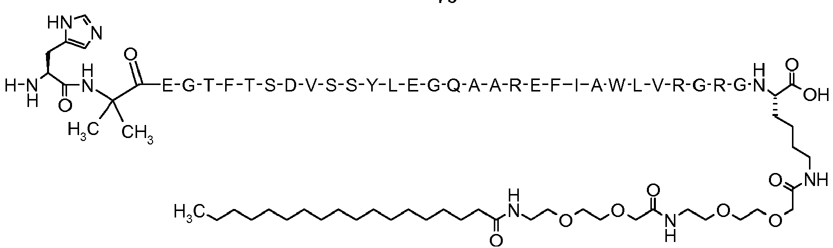

The compound was prepared according to the methods in Example 1 and in "General Synthetic methods".
HPLC (method B6): RT=38.7 min
LCMS: m/z = 1029.2 (M+4H)⁴⁺ 1371.4 (M+3H)³⁺ Calculated (M+H)⁺ = 4110.8

### Reference example 22

### [Aib⁸]-GLP-1-(7-37)Lys (2-(2-(2-(4-(Hexadecanoylamino)-4(S)-carboxybutyrylamino)ethoxy)ethoxy)acetyl)-OH

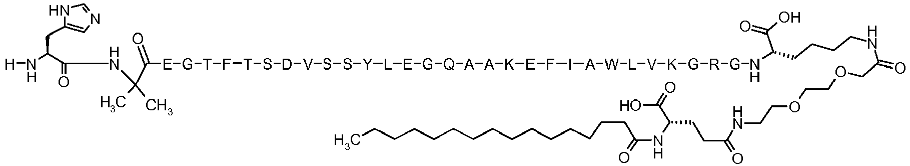

The compound was prepared according to the methods in Example 1 and in "General Synthetic methods".
HPLC (method B6): RT= 34.7 min
LCMS: m/z = 1000.3 (M+4H)⁴⁺ 1337.4 (M+3H)³⁺ Calculated (M+H)⁺ = 4110.8

### Reference example 23

### [Aib⁸,Arg^{26,34}] GLP-1(7-37) Lys{2-(2-(2-(2-[2-(2-(4-(octadecanoylamino)-4-carboxybutyrylamino)ethoxy)ethoxy]acetyl)ethoxy)ethoxy)acetyl)}-OH

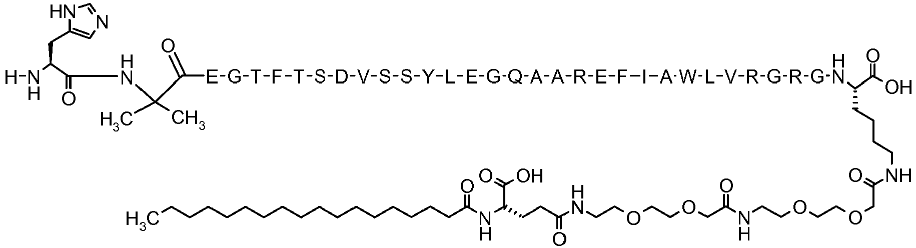

The compound was prepared according to the methods in Example 1 and in "General Synthetic methods".
HPLC (method B6): RT= 37.5 min
LCMS: m/z = 1414.9 (M+3H)³⁺ Calculated (M+H)⁺ = 4239.8

### Example 24

### [Aib⁸,Arg^{26,34}] GLP-1(7-37)Lys{2-(2-(2-(2-[2-(2-(17-carboxyheptanoylamino)ethoxy)ethoxy]acetylamino)ethoxy)ethoxy)acetyl)}-OH

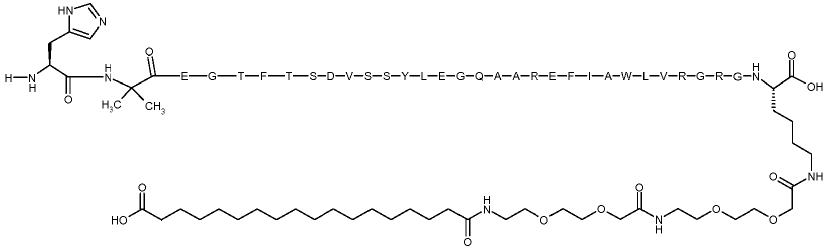

The compound was prepared according to the methods in Example 1 and in "General Synthetic methods".
HPLC (method B6): RT= 32.4 min
HPLC (method A1): RT= 43.8 min
LCMS: m/z = 1381.3 (M+3H)³⁺ Calculated (M+H)⁺ = 4140.0

### Example 25

### [Gly⁸, Arg^{26,34}] GLP1-(7-37) Lys{2-(2-(2-(2-[2-(2-(17-carboxyheptadecanoylamino)ethoxy)ethoxy]acetyl)ethoxy)ethoxy)acetyl)}-OH

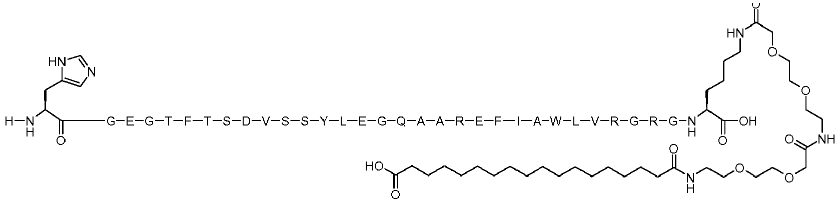

The compound was prepared according to the methods in Example 1 and in "General Synthetic methods".
HPLC (method A1): RT= 42.3 min
LCMS: m/z = 1372.3 (M+3H)³⁺ Calculated (M+H)⁺ = 4112.7

### Reference example 26

### [Aib⁸]GLP-1-(7-37)Lys(2-(2-(2-(2-(2-(2-(4-(Hexadecanoylamino)-4(S)-carboxybutyrylamino)ethoxy)ethoxy)acetylamino) ethoxy)ethoxy)acetyl)-OH

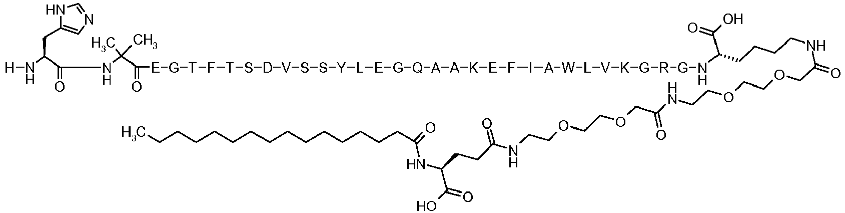

The compound was prepared according to the methods in Example 1 and in "General Synthetic methods".
HPLC (method B6): RT= 33.5 min
LCMS: m/z = 1040.3 (M+4H)⁴⁺ 1386.6 (M+3H)³⁺ Calculated (M+H)⁺ = 4155.8

### Reference example 27

### N^{ε37}-(2-(2-(2-(dodecanoylamino)ethoxy)ethoxy)acetyl)-[Aib^{8,22,35}Lys³⁷] GLP-1 H(7-37)-amide

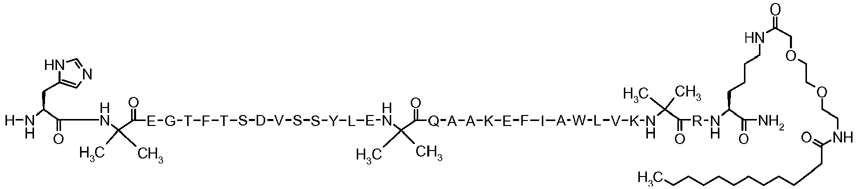

The compound was prepared according to the methods in Example 1 and in "General Synthetic methods".
HPLC: (method B6): RT=32.8 min
LC-MS: m/z = 765.7 (M+H)⁵⁺, 957.0 (M+H)⁴⁺, 1275.7 (M+H)³⁺ = Calculated (M+H)⁺ = 3822.9

### Reference example 28

### N^{ε37}-(2-(2-(2-(tetradecanoylamino)ethoxy)ethoxy)acetyl)-[Aib^{8,22,35}Lys³⁷] GLP-1 H(7-37)-amide

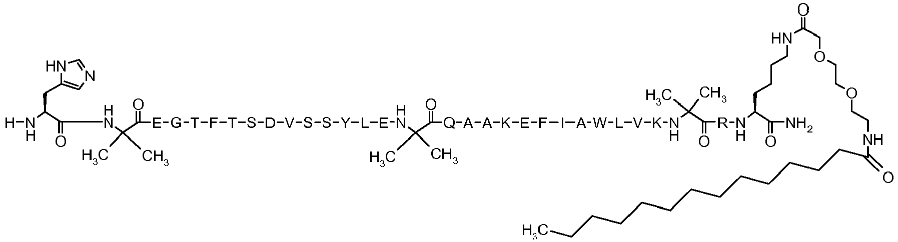

The compound was prepared according to the methods in Example 1 and in "General Synthetic methods".
HPLC: (method B6): RT= 34,6 min
LC-MS: m/z = 771,4 (M+5H)⁵⁺, 964,1(M+4H)⁴⁺, 1284,9(M+H)³⁺ Calculated (M+H)⁺ = 3851,5

### Reference example 29

### N^{ε37}-(2-(2-(2-(hexadecanoylamino)ethoxy)ethoxy)acetyl)-[Aib^{8,22,35}Lys³⁷] GLP-1 (7-37)-amide

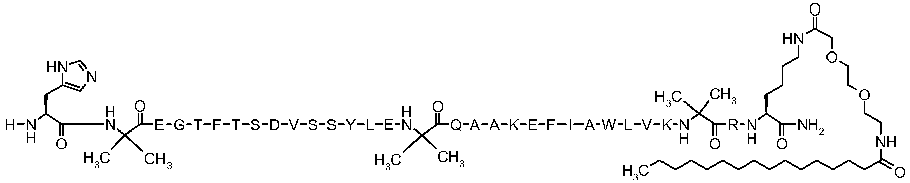

Prepared according to the methods in Example 1 and in "General Synthetic meth-ods".
HPLC: (method B6): RT= 36,8 min
LC-MS: m/z = 970.7(M+4H)⁴⁺, 1294.3 (M+3H)³⁺ Calculated (M+H)⁺= 3879,6

### Reference example 30

### N^{ε37}-(2-(2-(2-(octadecanoylamino)ethoxy)ethoxy)acetyl)-[Aib^{8,22,35}Lys³⁷] GLP-1 (7-37)-amide

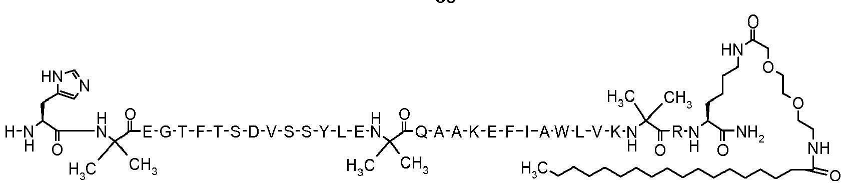

The compound was prepared according to the methods in Example 1 and in "General Synthetic meth-ods".
HPLC: (method B6): RT= 39,4 min
LC-MS: m/z = 977,9 (M+4H)⁴⁺, 1303,7(M+H)³⁺ Calculated (M+H)⁺ = 3907,6

### Reference example 31

### N^{ε37}-(2-(2-(2-(eicosanoylamino)ethoxy)ethoxy)acetyl)-[Aib^{8,22,35}Lys³⁷]GLP-1(7-37)-amide

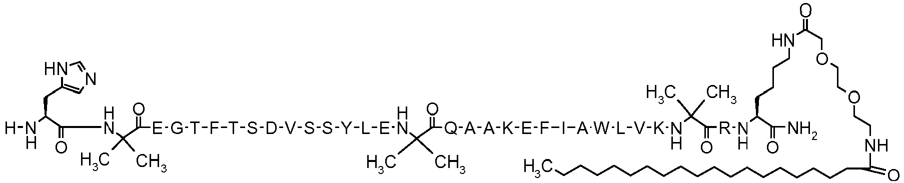

The compound was prepared according to the methods in Example 1 and in "General Synthetic methods".
HPLC: (method B6): RT= 42.7min
LC-MS: m/z = 984.8 (M+4H)⁴⁺, 1312.8 (M+3H)³⁺ Calculated (M+H)⁺ = 3935.7

### Reference example 32

### N^{ε36}-(2-(2-(2-(2-(2-(2-(octadecanoylamino)ethoxy)ethoxy)acetylamino)ethoxy)ethoxy)acetyl))-[Aib⁸,Arg^{26,34},Lys³⁶]GLP-1-(7-37)-OH

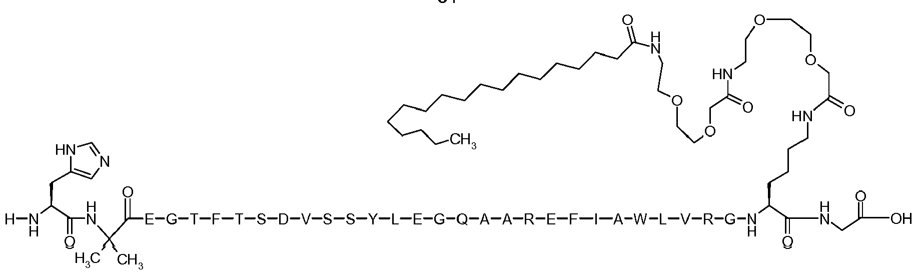

The compound was prepared according to the methods in Example 1 and in "General Synthetic methods".
HPLC: (method B6) RT= 40.7min
LC-MS: m/z = 792.3(M+5H)⁵⁺, 989.8(M+4H)⁴⁺, 1319.2(M+3H)³⁺ Calculated (M+H)⁺= 3955.5

### Reference example 33

### N^{ε36}-(2-(2-(2-(2-(2-(2-(octadecanoylamino)ethoxy)ethoxy)acetylamino)ethoxy)ethoxy)acetyl))[Arg^{26,34},Lys³⁶]GLP-1(7-37)-OH

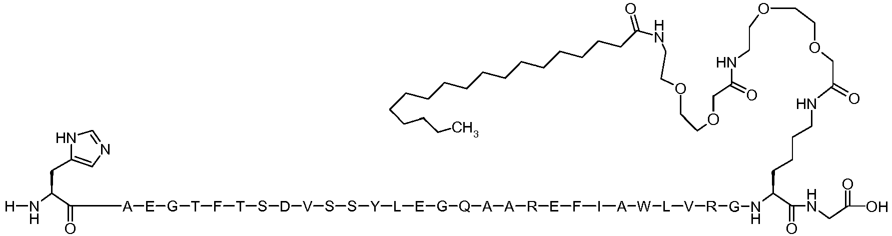

The compound was prepared according to the methods in Example 1 and in "General Synthetic methods".
HPLC: (method B6) RT= 40.5min
LC-MS: m/z = 789.5 (M+5H)⁵⁺, 986.3 (M+4H)⁴⁺, 1314.8 (M+3H)³⁺ Calculated (M+H)⁺= 3941.5

### Reference example 34

### N^{ε36}-{2-(2-(2-(2-[2-(2-(octadecanoylamino)ethoxy)ethoxy]acetylamino)ethoxy)ethoxy)acetyl)}-[Gly⁸,Arg^{26,34},Lys³⁶]GLP-1-(7-37)-OH

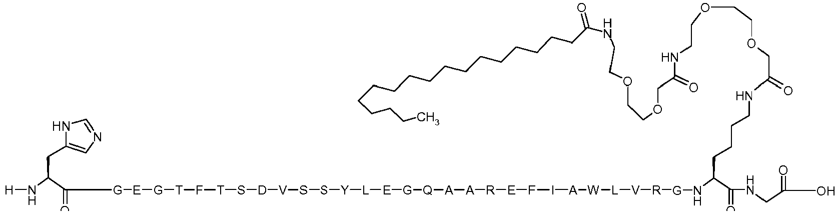

The compound was prepared according to the methods in Example 1 and in "General Synthetic methods".
HPLC: (method B6) RT= 38,3min
LC-MS: m/z = 786.8 (M+5H)⁵⁺, 982.8 (M+4H)⁴⁺, 1310.1 (M+3H)³⁺ Calculated (M+H)⁺ = 3927,5

### Reference example 35

### N^{ε37}-(2-(2-(2-(4-4(4,4,5,5,6,6,7,7,8,8,9,9,9-tridecafluorononanoylsulfamoyl-butyrylamino)ethoxy)ethoxy)acetyl))[Aib^{8,22,35},LyS³⁷]GLP-1-(7-37)-OH

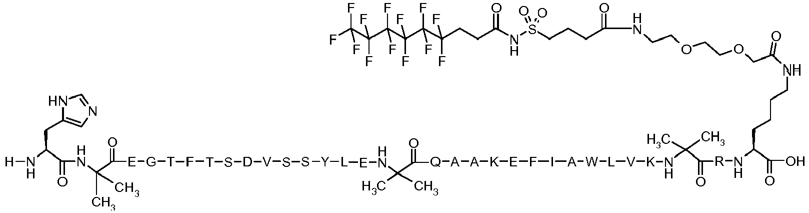

The compound was prepared according to the methods in Example 1 and in "General Synthetic methods".
HPLC: (method B6) RT= 32.4min
LC-MS: m/z = 1042.7(M+4H)⁴⁺, 1389.9 (M+3H)³⁺ Calculated (M+H)⁺= 4166.4

### Reference example 36

### N^{ε37}-(2-(2-(2-(3,3,4,4,5,5,6,6,7,7,8,8,9,9,10,10,11,11,12,12,12-Heneicosafluoro-dodecyloxyacetylamino)ethoxy) ethoxy)acetyl)[Aib^{8,22,35},Lys³⁷]GLP-1-(7-37)-OH

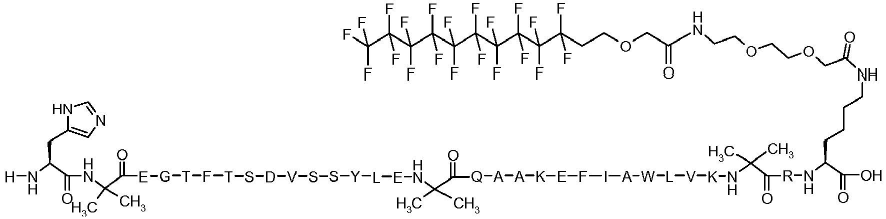

The compound was prepared according to the methods in Example 1 and in "General Synthetic methods".
HPLC: (method B6) RT= 36.7 min
LC-MS: m/z = 1062.8 (M+4H)⁴⁺, 1416.9 (M+3H)³⁺ Calculated (M+H)⁺= 4247.3

### Reference example 37

### N^{ε37}-(2-(2-(2-(4-(hexadecanoylsulfamoyl)butyrylamino)ethoxy)ethoxy)acetyl)[Aib^{8,22,35},Lys³⁷] GLP-1-(7-37)-OH

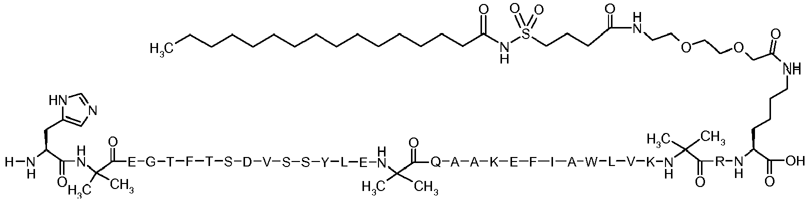

The compound was prepared according to the methods in Example 1 and in "General Synthetic methods".
HPLC: (method B6): RT= 37.4min
LC-MS: m/z = 1008.8 (M+4H)⁴⁺ 1344.3 (M+3H)³⁺ Calculated (M+H)⁺ = 4030.7

### Reference example 38

### [Arg^{26,34}]GLP-1(7-37)Lys({2-(2-(2-(2-[2-(2-(octadecanoylamino)ethoxy)ethoxy]acetylamino)ethoxy)ethoxy)acetyl)})-OH

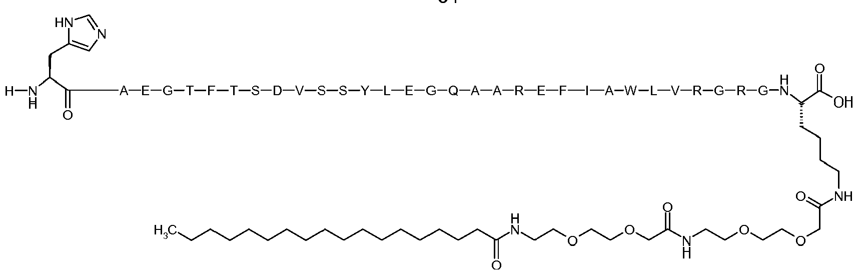

The compound was prepared according to the methods in Example 1 and in "General Synthetic methods".
HPLC (method B6): RT= 38.5 min
LCMS: m/z = (M+4H)⁴⁺ 1025.1 (M+3H)³⁺ 1366.7 Calculated (M+H)⁺ = 4096.0

### Reference example 39

### [Arg^{26,34}] GLP-1(7-37)Lys{2-(2-(2-(2-[2-(2-(4-(octadecanoylamino)-4-carboxybutyrylamino)ethoxy)ethoxy]acetylamino)ethoxy)ethoxy)acetyl)}-OH

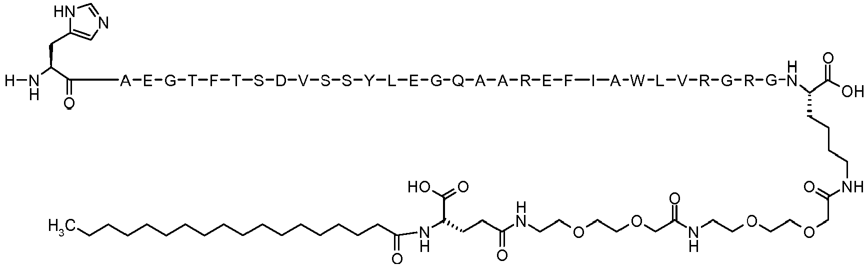

The compound was prepared according to the methods in Example 1 and in "General Synthetic methods".
HPLC (method B6): RT= 37.7 min
LCMS: m/z = (M+4H)⁴⁺ 1057.8 (M+3H)³⁺ 1410.2 Calculated (M+H)⁺ = 4235.9

### Reference example 40

### N^{ε20}-{2-(2-(2-(2-[2-(2-(4-(hexadecanoylamino)-4-carboxybutyrylamino)ethoxy)ethoxy]acetylamino)ethoxy)ethoxy)acetyl)}-exendin(1-39)

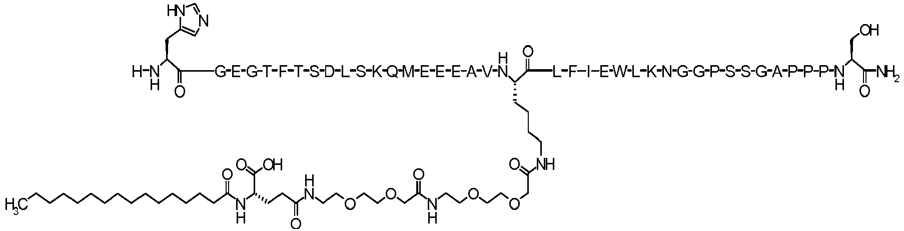

The compound was prepared according to the methods in Example 1 and in "General Synthetic methods".
HPLC (method B6): RT= 33.6 min
LCMS: m/z = (M+4H)⁴⁺ 1205.3 (M+3H)³⁺ 1606.9 Calculated (M+H)⁺ = 4816.5

### Example 41

### [Ala⁸, Arg^{26,34}]GLP-1(7-37)Lys((2-[2-((2-oxalylamino-3-carboxy-2-4,5,6,7-tetrahydrobenzo[b]thiophen-6-yl-acetylamino))ethoxy]ethoxyacetyl) amide

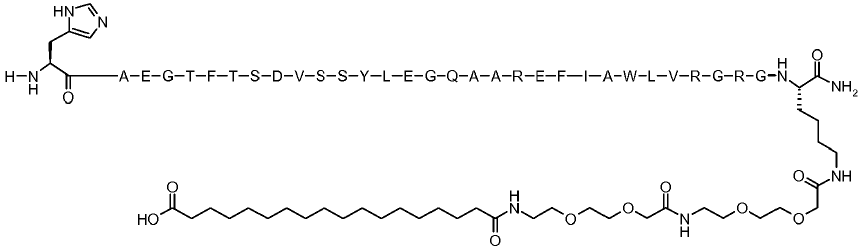

The compound was prepared according to the methods in Example 1 and in "General Synthetic methods".
HPLC (method B6): RT= 32.1 min
HPLC (method A1): RT= 42.2 min
LCMS: m/z = 1033.3 (M+4H)⁴⁺ 1376.6(M+3H)³⁺ Calculated (M+H)⁺ = 4126.7

### Reference example 42

### [Aib^{8,22,35}]GLP-1(7-37)Lys((2-[2-((2-oxalylamino-3-carboxy-2-4,5,6,7-tetrahydro-benzo[b]thiophen-6-yl-acetylamino))ethoxy]ethoxyacetyl) amide

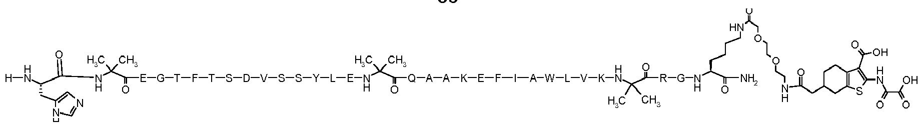

The compound was prepared according to the methods in Example 1 and in "General Synthetic methods".
HPLC (method B1): RT= 37.4 min
HPLC (method A1): RT= 35.5 min
LCMS: m/z = 1002.5 (M+4H)⁴⁺ 1336.7 (M+3H)³⁺ Calculated (M+H)⁺ = 4007.5

### Reference example 43

### N^{ε36}-(2-(2-(2-(2-(2-(2-(4-(octadecanoylamino)-4(S)-carboxybutyrylamino)ethoxy)ethoxy)acetylamino)ethoxy)ethoxy)acetyl)-[Aib⁸,Arg^{26,34},Lys³⁶]GLP-1-(7-37)-OH

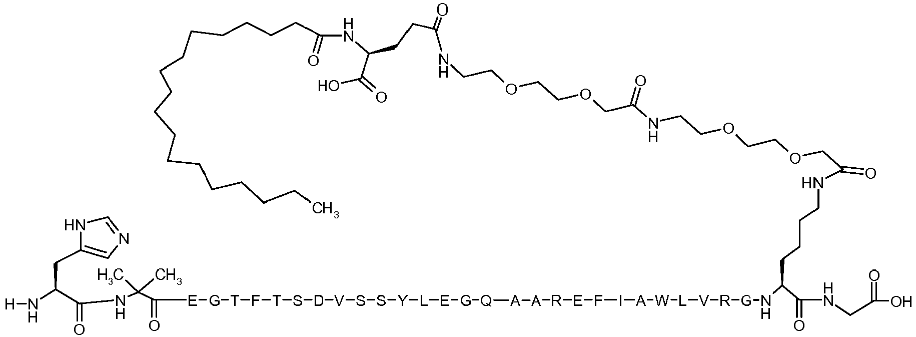

The compound was prepared according to the methods in Example 1 and in "General Synthetic methods".
HPLC: (method B6): RT= 39.0 min
LC-MS: m/z = 1022.3 (M+4H)⁴⁺, 1362.3 (M+3H)³⁺, Calculated (M+H)⁺= 4084.6

### Reference example 44

### N^{ε36}-(2-(2-(2-(2-(2-(2-(4-(octadecanoylamino)-4(S)-carboxybutyrylamino)ethoxy)ethoxy)acetylamino)ethoxy)ethoxy)acetyl)-[Gly⁸,Arg^{26,34},Lys³⁶]GLP-1-(7-37)-OH

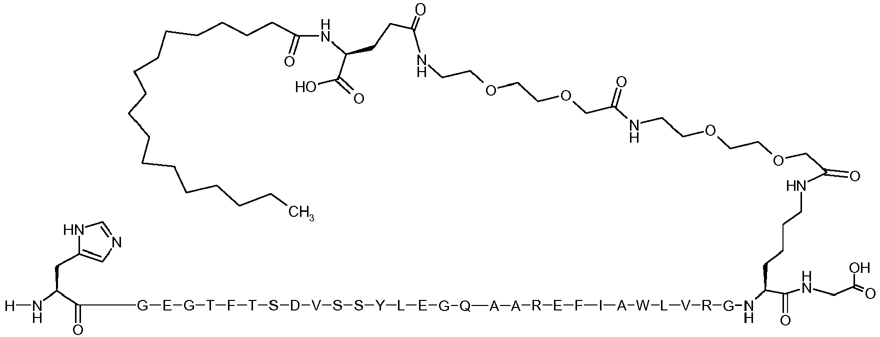

The compound was prepared according to the methods in Example 1 and in "General Synthetic methods".
HPLC: (method B6): RT= 38,6 min
LC-MS: m/z = 1015.2 (M+4H)⁴⁺, 1353.4 (M+3H)³⁺, Calculated (M+H)⁺= 4056.6

### Reference example 45

### N^{ε37}-2-(2-(2-(4-(4-(Heptadecanoylamino)-4-(S)-carboxybutyrylamino)-4-(S)-carboxybutyrylamino)ethoxy)ethoxy) acetyl-[Aib^{8,22,35},Lys³⁷]GLP-1-(7-37)-NH₂

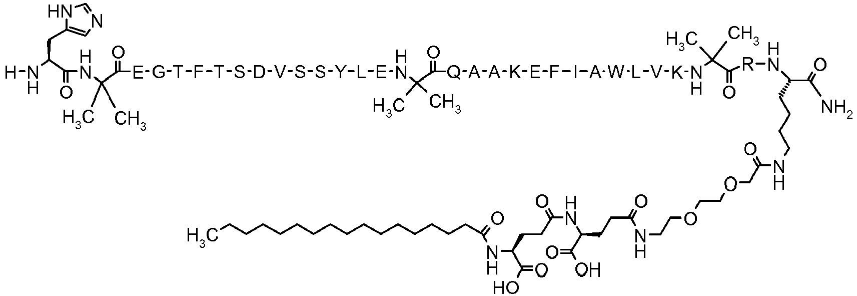

The compound was prepared according to the methods in Example 1 and in "General Synthetic methods".
HPLC: (method B4): RT=10.72min
LCMS: m/z = 1039.0 (M+4H)⁴⁺, 1385.0 (M+3H)³⁺ Calculated (M+H)⁺ = 4152.0

### Reference example 46

### N^{ε37}-2-(2-[2-(2-[2-(4-[4-(Heptadecanoylamino)-4-(S) carboxybutyrylamino]-4-(S)-carboxybutyrylamino)ethoxy] ethoxy)acetylamino)ethoxy]ethoxy)acetyl-[Aib^{8,22,35},Lys³⁷]GLP-1-(7-37)-NH₂

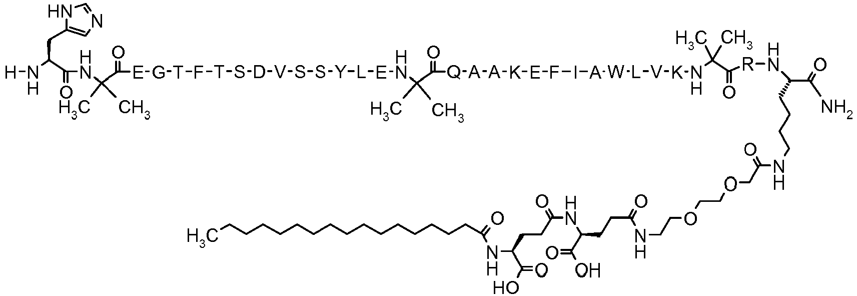

The compound was prepared according to the methods in Example 1 and in "General Synthetic methods".
HPLC: (method B4): RT=10.74 min
LCMS: m/z = 1074 (M+4H)⁴⁺, 1433 (M+3H)³⁺ Calculated (M+H)⁺ = 4297

### Reference example 47

### N^{ε26}-(2-(2-(2-(4-(Hexadecanoylamino)-4(S)-carboxybutyrylamino) ethoxy)ethoxy)acetyl)-[Aib⁸,Arg³⁴]GLP-1-(7-37)--OH

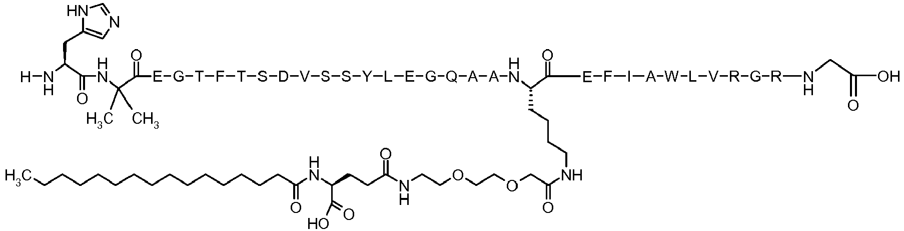

The compound was prepared according to the methods in Example 1 and in "General Synthetic methods".
HPLC: (method B4): RT=10.71 min
LCMS: m/z = 979.0 (M+4H)⁴⁺, 1304.0 (M+3H)³⁺ Calculated (M+H)⁺ = 3910.0

### Reference example 48

### N^{ε26}-2-(2-2-(2-(2-(2-(4-(Octadecanoylamino)-4(S)-carboxybutyrylamino)ethoxy)ethoxy)acetylamino)ethoxy)ethoxy)acetyl-[Aib⁸,Arg³⁴]GLP-1-(7-37)-OH

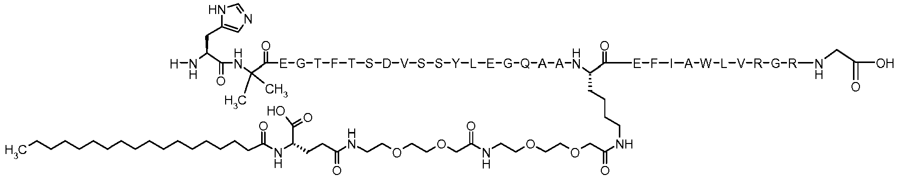

The compound was prepared according to the methods in Example 1 and in "General Synthetic methods".
HPLC: (method B4): RT=11.32 min
LCMS: m/z = 1021 (M+4H)⁴⁺, 1362 (M+3H)³⁺ Calculated (M+H)⁺ = 4084

The peptide was synthesized on a chlorotrityl resin (Novabiochem) using the Fmoc strategy on an Advanced Chemtech 348 peptide synthesizer (0.5 mmol/g, 100 mg resin/hole and 10 holes were used). The couplings were mediated in Diisopropylcarbodiimide (DIC) (Fluka) and 1-hydroxybenzotriazol (HOBt)/1-hydroxy-7-aza-benzotriazole (HOAt) (2:1) (Senn Chemicals) in 1-methyl-pyrrolidin-2-one (NMP) and 10 molar equivalents of amino acids and coupling reagents were applied. The used protected amino acid derivatives were standard Fmoc-amino acids (Advanced Chemtech) with the exception of the amino acids Fmoc-Lys(ivDde) (Novabiochem) and Fmoc-Glu-OtBu (Bachem). The resin was afterwards divided into 5 portions (0.1 mmol) and the N-terminal was then treated with (Boc)₂O and DIEA (5 molar equivalent) in NMP.

The attachment of sidechains and linkers to specific lysine residues on the crude resin bound protected peptide was carried out in a specific position by incorporation of Fmoc-Lys(ivDde)-OH during automated synthesis followed by selective deprotection with hydrazine.

Procedure for removal of Dde-protection. The resin (0.1 mmol) was placed in a syringe and treated with 3% hydrazine and 3% piperidine in NMP (50 min at r.t.) to remove the Dde group and wash with NMP (4x5 ml).

### Procedure for attachment of sidechains to Lysine residues.

The OEG or amino acid (7 molar equivalents relative to resin) was dissolved in NMP. HOAt (7 molar equivalents relative to resin) and diisopropylcarbodiimide (7 molar equivalents relative to resin) was added and the solution was stirred for 15 min. Then, the solution was added to the resin. The resin was shaken overnight at room temperature. The resin was washed with NMP (3x5 ml).

Procedure for removal of Fmoc-protection: The resin (0.1 mmol) was placed in a syringe treated with a solution of 30% piperidine in NMP (5ml in 20 min). The resin was washed with NMP (2x5 ml) and methylene chloride (2x5 ml).

### Procedure for cleaving the peptide off the resin:

The peptide was cleaved from the resin by stirring for 120 min at room temperature with a mixture of trifluoroacetic acid, water and triisopropylsilane (94:3:3). The cleavage mixture was filtered and the filtrate was concentrated to an oil by a stream of nitrogen. The crude peptide was precipitated from this oil with 10 ml diethyl ether and washed 2 times with 10 ml diethyl ether.

### Example 49

### [Gly⁸,Arg^{26,34}]GLP-1(7-37)Lys(2-(2-(19-(carboxy)nonadecanoylamino)ethoxy)ethoxy)acetyl)-OH

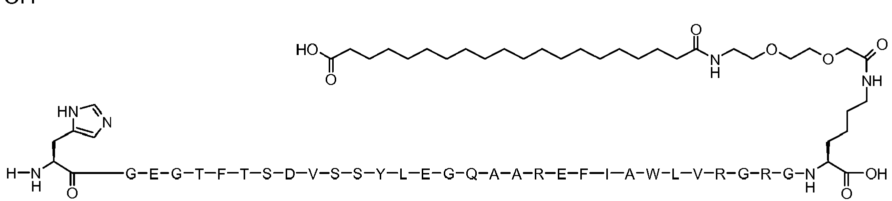

A chlorotrityl resin (0.5 mmol/g Novabiochem, 0.1 mmole) was used to produce the primary sequence on an Advanced Chemtech 348 machine. All protecting groups were acid labile with the exception of the residue used in position 37 (FmocLys(ivDde)-OH, Novabiochem) allowing specific deprotection of this lysine rather than any other lysine.

### Procedure

The above prepared resin (0.1 mmole) containing the GLP-1 analogue amino acid sequence was placed in a syringe and treated with 3% hydrazine and 3% piperidine in N-methyl pyrrolidone (50 min) to remove the Dde group. The resin was washed with NMP (4x5 ml). Fmoc-8-amino-3,6-dioxaoctanoic acid (Neosystem FA03202) (7 molar equivalents relative to resin) was dissolved in NMP. HOAt (7 molar equivalents relative to resin) and diisopropylcarbodiimide (7 molar equivalents relative to resin) was added and the solution was stirred for 15 min. The solution was then added to the resin. The resin was shaken overnight at room temperature. The resin was washed with NMP (4x5 ml). A solution of 30% piperidine in NMP (5 ml, 20min) was added to the resin. The resin was washed with NMP (4x5 ml). The N-hydroxysuccinimide ester of C20 (6 molar equivalents relative to resin, KJ. Ross-Petersen A/S) and DIEA was dissolved in NMP and added to the resin. The resin was shaken overnight at room temperature. The resin was washed with NMP (3x5 ml) and methylene chloride (2x5 ml). The peptide was cleaved from the resin by stirring for 120 min at room temperature with a mixture of trifluoroacetic acid, water and triisopropylsilane (94:3:3, 3 ml). The cleavage mixture was filtered and the filtrate was concentrated to an oil in vacuum. The crude peptide was precipitated from this oil with 10 ml diethyl ether and washed 2 times with 10 ml diethyl ether.

### Purification

The crude peptide dissolved in DMSO at a concentration of 5-10 mg/200 µl and applied to a 7.8 x 300 mm X-Terra Prep MS C18 10 µm column running at 40°C. After 5 minutes at 30% CH₃CN, 0.08% TFA, 4 ml/min, the column was eluted with a linear gradient of 30 to 65% CH₃CN over 35 minutes. The main UV peaks were collected manually and the desired peak identified by MALDI-MS.

The concentration of the peptide in the eluate was determined by measurement of the UV absorption at 280 nm assuming molar extinction coefficients of 1280 and 3690 for tyrosine and tryptophan respectively.

After the concentration determination the eluate was aliquotted into vials containing the desired amount and dried by vacuum centrifugation.
HPLC: elutes at 27.9 min = 52.9% CH₃CN
MALDI-MS: 3996 (MH⁺)

### Example 50

### [Gly⁸,Arg^{26,34}]GLP-1(7-37)Lys((2-(2-(17-(carboxy)heptadecanoylamino)ethoxy)ethoxy)acetyl))-OH

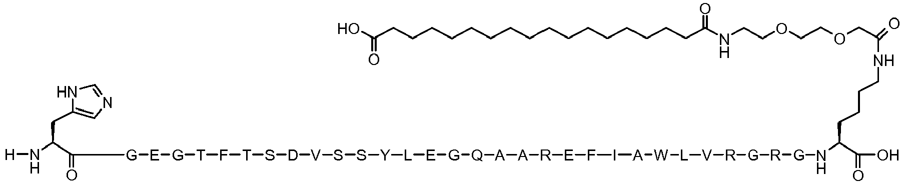

The compound was prepared as in previous example and according to "Synthetic methods" except that octadecanedioic acid C18 was attached as a monoprotected tert-butyl ester (3 molar equivalents relative to resin) and the coupling was mediated with HOAt and DIC (also 3 molar equivalents relative to resin) in NMP. The crude peptide was dissolved in 22.5% CH₃CN, 0.1 N NaOH for purification.
HPLC: elutes at 25.4 min = 50.4% CH₃CN
MALDI-MS: 3969 (MH⁺)

### Example 51

### [Gly⁸,Arg^{26,34}]GLP-1(7-37)Lys(2-(2-(2-(4-(19-(carboxy)nonadecanoylamino)-4-carboxybutyrylamino)ethoxy)ethoxy)acetyl)-OH

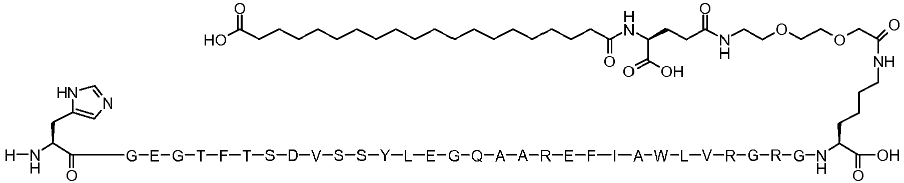

The compound was prepared as in the two previous examples and according to "Synthetic methods". The amino acid Fmoc-Glu(OtBu) (6 molar equivalents relative to resin) was coupled to the resin with HOAt and DIC (6 molar equivalents relative to resin). The crude peptide was dissolved in 22.5% CH₃CN, 0.1 N NaOH for purification.
HPLC: elutes at 27.2 min = 52.2% CH₃CN
MALDI-MS: 4124 (MH⁺)

### Example 52

### [Gly⁸,Arg^{26,34}]GLP-1(7-37)Lys((2-(2-(2-(2-(2-(2-(2-(2-(2-(hexadecanoylamino)ethoxy)ethoxy)acetyl)ethoxy)ethoxy)acetylamino)ethoxy)ethoxy)-acetyl)-OH

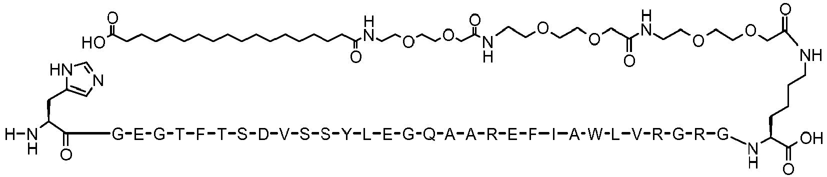

The compound was prepared as in the three previous examples and according to "Synthetic methods" except that additional two OEG was coupled to the side chain of Lys.
HPLC: elutes at 25.0 min = 50.0% CH₃CN
MALDI-MS: 4259 (MH⁺)

### Reference example 53

### [Gly⁸, Arg^{26,34}]GLP-1 (7-37)Lys (2-(2-(2-(2-(2-(2-(octadecanoylamino)ethoxy)ethoxy)-acetylamino)ethoxy)ethoxy)acetyl) NH₂

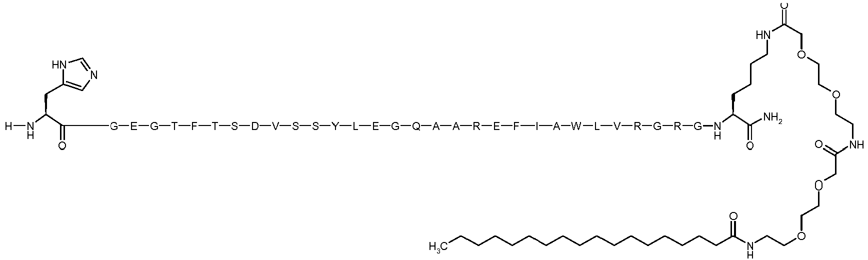

The compound was prepared as in Example 1 and in accord with "Synthetic methods"
HPLC (method B6): RT=38.8 min
LCMS: m/z = 1022.3 (M+4H)³⁺ Calculated (M+H)⁺ = 4081.7

### Example 54

### N^{ε20}(2-(2-(2-(2-(2-(2-(2-(2-(2-(2-(2-(2-(4-(17-(carboxy)heptadecanoylamino)-4-carboxybutyrylamino)ethoxy)ethoxy)acetylamino)ethoxy)ethoxy)acetylamino) ethoxy)ethoxy)acetylamino)ethoxy)ethoxy)acetyl) [Lys²⁰]exendin-4 (1-39)-NH₂

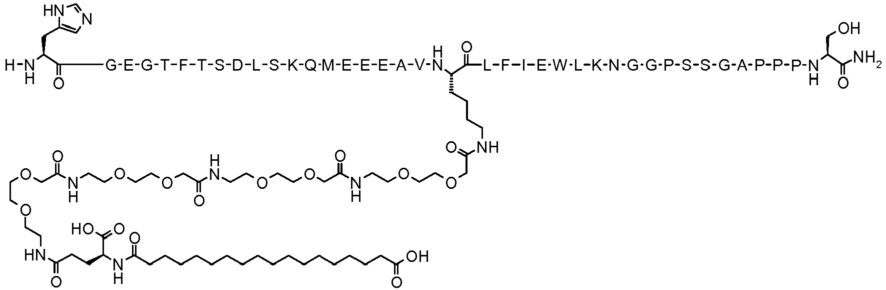

The compound was prepared according to the methods in Example 1 and in "General Synthetic meth-ods".
HPLC (method A1): RT= 41.9 min
HPLC (method B6): RT= 31.3 min
LCMS: m/z = 1722.7 (M+3H)³⁺ Calculated (M+H)⁺ = 5164.9

### Example 55

### N^{ε36}-(2-(2-(2-(2-(2-(2-(17-Carboxyheptadecanoylamino)ethoxy)ethoxy)acetylamino)ethoxy)ethoxy)acetyl)[Aib⁸,Arg^{26,34},Lys³⁶]GLP-1(7-37)

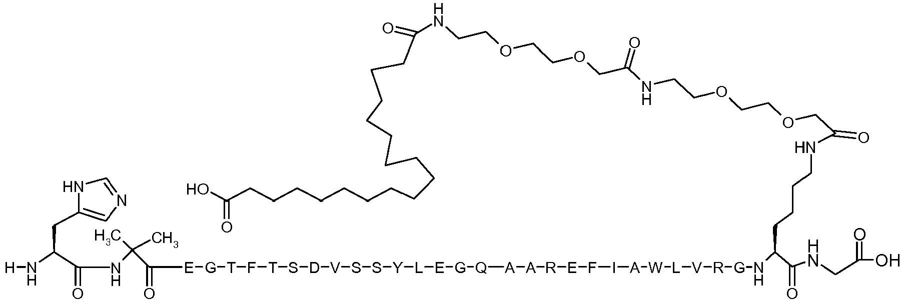

The compound was prepared according to the methods in Example 1 and in "General Synthetic methods".
HPLC: (method B6): RT= 34,2 min
LC-MS: m/z = 997,2 (M+4H)⁴⁺, 1329,4 (M+3H)³⁺, 1993,2 (M+2H)²⁺, Calculated (M+H)⁺= 3985,5

### Example 56

### N-^{ε36}-(2-(2-(2-(2-(2-(2-(17-Carboxyheptadecanoylamino)ethoxy)ethoxy)acetylamino)ethoxy)ethoxy)acetyl)[Arg^{26,34},Lys³⁶]GLP-1(7-37)

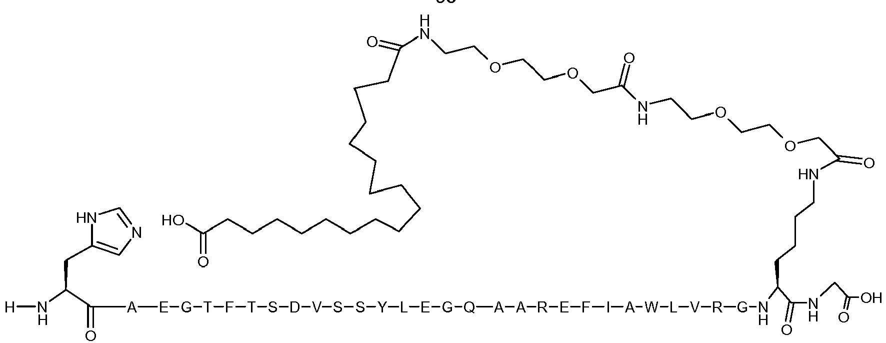

The compound was prepared according to the methods in Example 1 and in "General Synthetic methods".
HPLC: (method B6): RT= 34,2 min
LC-MS: m/z = 993.8 (M+4H)⁴⁺, 1324.6 (M+3H)³⁺, 1987.2 (M+2H)²⁺, Calculated (M+H)⁺= 3971.5

### Example 57

### N^{ε36}-(2-(2-(2-(2-(2-(2-(17-Carboxyheptadecanoylamino)ethoxy)ethoxy)acetylamino)ethoxy)ethoxy)acetyl) [Gly⁸,Arg^{26,34},Lys³⁶]GLP-1 (7-37)

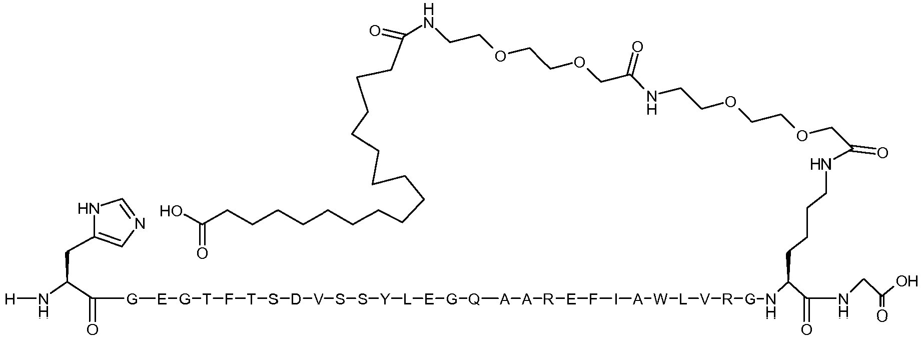

The compound was prepared according to the methods in Example 1 and in "General Synthetic methods".
HPLC: (method B6): RT= 34,2 min
LC-MS: m/z = 990.3 (M+4H)⁴⁺, 1320.3(M+3H)³⁺, Calculated (M+H)⁺= 3957.4

### Reference example 58

### N^{ε20}-(2-(2-(2-(2-(2-(2-(2-(2-(2-(Octadecanoylamino)ethoxy)ethoxy)acetylamino)ethoxy)ethoxy)acetylamino)ethoxy)-ethoxy)acetyl)[Lys²⁰]Exendin-4 (1-39)amide

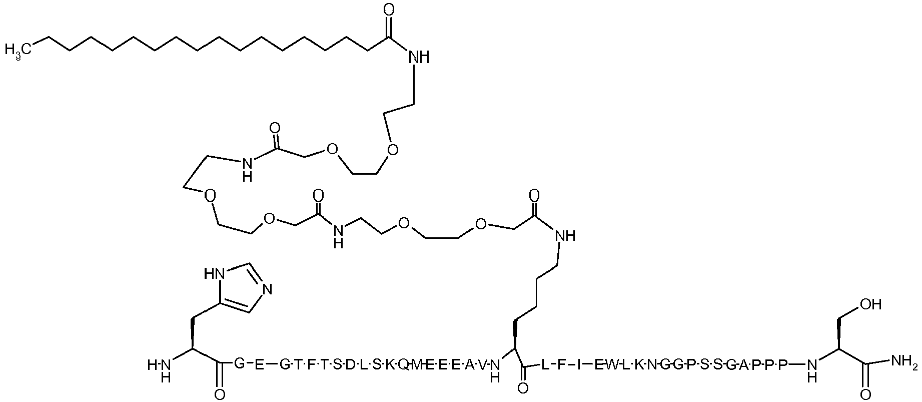

The compound was prepared according to the methods in Example 1 and in "General Synthetic methods".
HPLC: (method B6): RT= 37.7 min
LC-MS: m/z = 1216.6 (M+4H)⁴⁺, 1621.4 (M+3H)³⁺, Calculated (M+H)⁺= 4861.5

### Reference example 59

### N^{ε36}-(2-(2-(2-(2-(2-(2-(4-(octadecanoylamino)-4(S)-carboxybutyrylamino)ethoxy)ethoxy)acetylamino)ethoxy)ethoxy)acetyl)-[Arg^{26,34},Lys³⁶]GLP-1-(7-37)

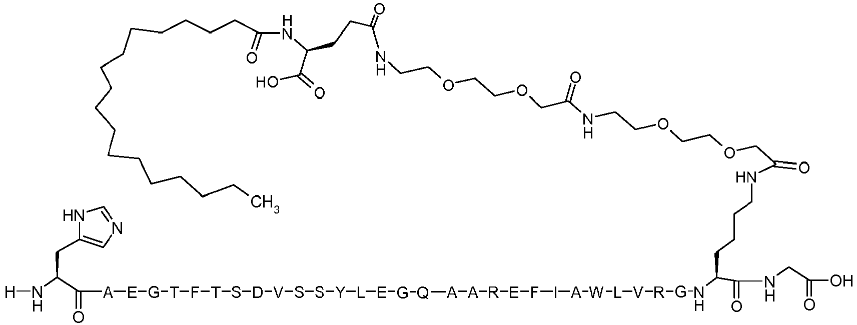

The compound was prepared according to the methods in Example 1 and in "General Synthetic methods".
HPLC: (method B6): RT= 39.1 min
LC-MS: m/z = 1018.8 (M+4H)⁴⁺, 1357.6 (M+3H)³⁺, Calculated (M+H)⁺= 4070.6

### Example 60

### N^{ε26}-(2-[2-(2-[2-(2-[2-(17-Carboxyheptadecanoylamino)ethoxy] ethoxy)acetylamino]ethoxy)ethoxy]acetyl)[Arg³⁴]GLP-1-(7-37)-OH

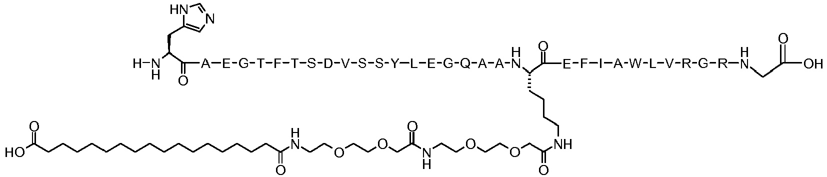

The compound was prepared according to the methods in Example 1 and in "General Synthetic methods".
HPLC: (method B4): RT= 12.1 min
LCMS: m/z = 993.0 (M+4H)⁴⁺, 1325.0 (M+3H)³⁺ Calculated (M+H)⁺ = 3970.0

### Example 61

### N^{ε26}-[2-(2-[2-(2-[2-(2-[4-(17-Carboxyheptadecanoylamino)-4(S)-carboxybutyrylamino]ethoxy)ethoxy]acetylamino)ethoxy]ethoxy)acetyl][Arg³⁴]GLP-1-(7-37)-OH

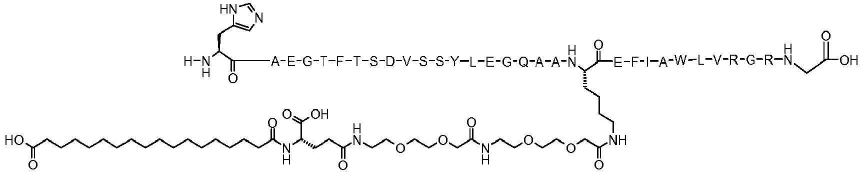

The compound was prepared according to the methods in Example 1 and in "General Synthetic methods".
HPLC: (method B4): RT= 11.8 min
LCMS: m/z = 1026 (M+4H)⁴⁺, 1368 (M+3H)³⁺ Calculated (M+H)⁺ = 4100

### Example 62

### N^{ε20}-(2-(2-(2-(2-(2-(2-(2-(2-(2-(17-Carboxyheptadecanoylamino)ethoxy)ethoxy)acetylamino)ethoxy)ethoxy)acetylamino)ethoxy)ethoxy)acetyl)[Lys²⁰] Exendin-4 (1-39) amide

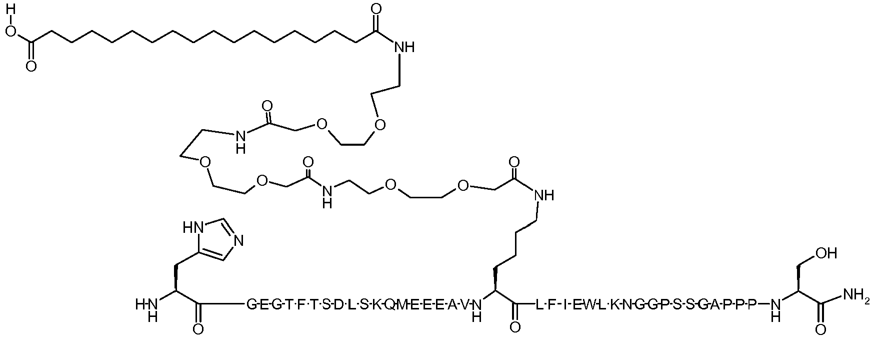

The compound was prepared according to the methods in Example 1 and in "General Synthetic methods".
HPLC: (method B6): RT= 32,3 min
LC-MS: m/z = 1223.9 (M+4H)⁴⁺, 1630.8 (M+3H)³⁺, Calculated (M+H)⁺= 4891.5

### Example 63

### [Gly⁸, Glu^{22,23,30},Arg^{18,26,34}]GLP1 (7-37) Lys(2-(2-(2-(2-(2-(2-(17-carboxyheptadecanoylamino)ethoxy)ethoxy)acetylamino)ethoxy))ethoxy)acetyl)-NH₂

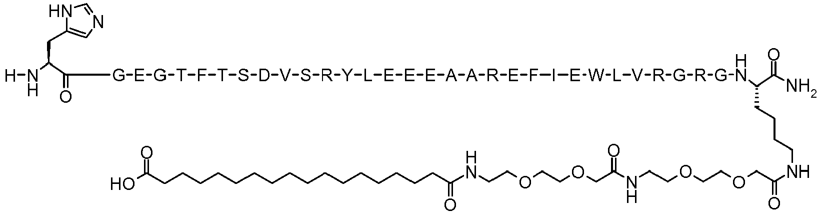

The compound was prepared according to the methods in Example 1 and in "General Synthetic methods".
HPLC: (method B6): RT= 32.0 min
HPLC: (method A1): RT=43.4 min
LCMS: m/z = 1438.7 (M+3H)³⁺ Calculated (M+H)⁺ = 4311.8

### Example 64

### [Imidazolylpropionic acid⁷, Asp¹⁶, Aib^{22,35}]GLP1(7-37)Lys NH((2-{[4-(17-carboxyheptadecanoylamino)butylcarbamoyl]methoxy}ethoxy)ethoxy))

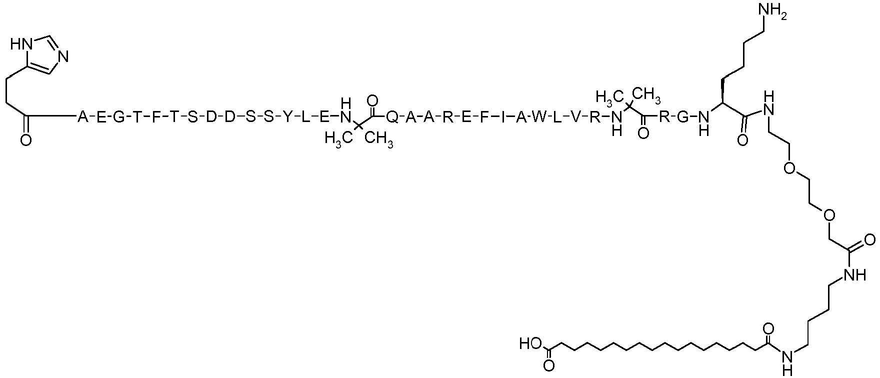

The compound was prepared according to the methods in Example 1 and in "General Synthetic methods".
HPLC: (method B1): RT=32.5 min
HPLC: (method A1): RT=43.5 min
LCMS: m/z = 1028.8 (M+4H)⁴⁺ Calculated (M+H)⁺ = 4108.7

### Example 65

### [Imidazolylpropionic acid⁷, Aib^{22,35} ]GLP1(7-37)Lys NH((2-{[4-(17-carboxyheptadecanoylamino)butylcarbamoyl]methoxy}ethoxy)ethoxy))

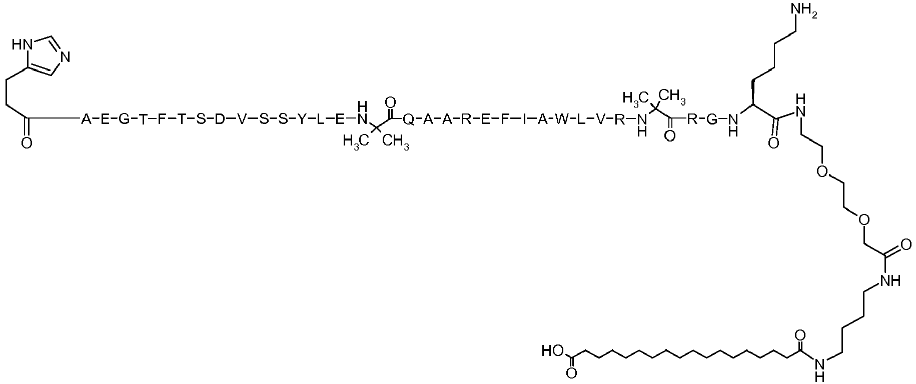

The compound was prepared according to the methods in Example 1 and in "General Synthetic methods".
HPLC: (method B6): RT=33.7 min
HPLC: (method A1): RT=44.8 min
LCMS: m/z = 1024.8 (M+4H)⁴⁺, 1365.4 (M+3H)³⁺ Calculated (M+H)⁺ = 4092.8

### Example 66

### [3-(5-Imidazoyl)propionyl⁷, Aib⁸, Arg^{26,34}] GLP-1 (7-37)Lys{2-(2-(2-(2-[2-(2-(17-carboxyheptanoylamino)ethoxy)ethoxy]acetylamino)ethoxy)ethoxy)acetyl)}-OH

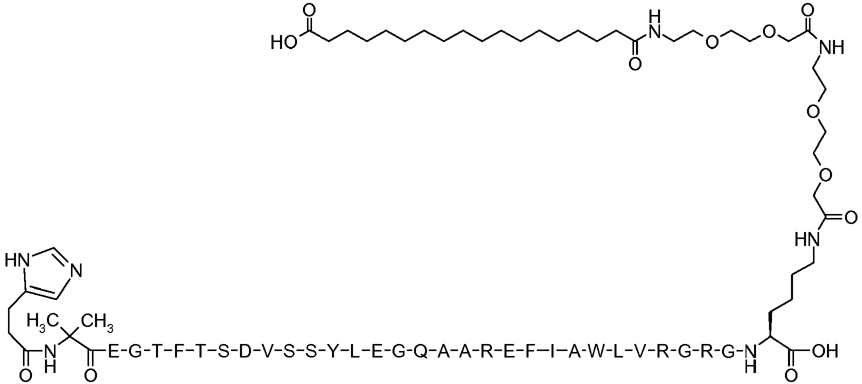

The compound was prepared according to the methods in Example 1 and in "General Synthetic methods".
MALDI-MS: 4127 (MH+)
HPLC: elutes: 25.5 min = 50.6% CH3CN

### BIOLOGICAL FINDINGS

### Protraction of GLP-1 derivatives after i.v. or s.c. administration

The protraction of a number GLP-1 derivatives of the invention was determined by monitoring the concentration thereof in plasma after sc administration to healthy pigs, using the methods described below. For comparison also the concentration in plasma of GLP-1(7-37) after sc. administration was followed. The protraction of other GLP-1 derivatives of the invention can be determined in the same way.

### Pharmacokinetic testing of GLP-1 analogues in minipigs

The test substances were dissolved in a vehicle suitable for subcutaneous or intravenous administration. The concentration was adjusted so the dosing volume was approximately 1 ml.

The study was performed in 12 male Göttingen minipigs from Ellegaard Göttingen Minipigs ApS. An acclimatisation period of approximately 10 days was allowed before the animals entered the study. At start of the acclimatisation period the minipigs were about 5 months old and in the weight range of 8-10 kg.
The study was conducted in a suitable animal room with a room temperature set at 21-23°C and the relative humidity to ≥ 50%. The room was illuminated to give a cycle of 12 hours light and 12 hours darkness. Light was from 06.00 to 18.00 h.
The animals were housed in pens with straw as bedding, six together in each pen.
The animals had free access to domestic quality drinking water during the study, but were fasted from approximately 4 pm the day before dosing until approximately 12 hours after dosing.
The animals were weighed on arrival and on the days of dosing.

The animals received a single intravenous or subcutaneous injection. The subcutaneous injection was given on the right side of the neck, approximately 5-7 cm from the ear and 7-9 cm from the middle of the neck. The injections were given with a stopper on the needle, allowing 0.5 cm of the needle to be introduced.

Each test substance was given to three animals. Each animal received a dose of 2 nmol/kg body weight.
Six animals were dosed per week while the remaining six were rested.

A full plasma concentration-time profile was obtained from each animal. Blood samples were collected according to the following schedule:

### After intravenous administration:

Predose (0), 0.17 (10 minutes), 0.5, 1, 2, 4, 6, 8, 12, 24, 48, 72, 96, and 120 hours after injection.

### After subcutaneous administration:

Predose (0), 0.5, 1, 2, 4, 6, 8, 12, 24, 48, 72, 96, and 120 hours after injection.
At each sampling time, 2 ml of blood was drawn from each animal. The blood samples were taken from a jugular vein.
The blood samples were collected into test tubes containing a buffer for stabilisation in order to prevent enzymatic degradation of the GLP-1 analogues.
Plasma was immediately transferred to Micronic-tubes. Approximately 200 µl plasma was transferred to each Micronic-tube. The plasma was stored at -20°C until assayed. The plasma samples were assayed for the content of GLP-1 analogues using a immunoassay.
The plasma concentration-time profiles were analysed by a non-compartmental pharmacokinetic analysis. The following pharmacokinetic parameters were calculated at each occasion: AUC, AUC/Dose, AUC_{%Extrap}, Cₘₐₓ, tₘₐₓ, λ_{z}, t_{½}, CL, CL/f, V_{z}, V_{z}/f and MRT.

Selected compounds of the invention were tested in Danish Landrace pigs.

### Pharmacokinetic testing of GLP-1 analogues in pigs

Pigs (50% Duroc, 25% Yorkshire, 25% Danish Landrace, app 40 kg) were fasted from the beginning of the experiment. To each pig 0.5 nmol of test compound per kg body weight was administered in a 50 µM isotonic solution (5 mM phosphate, pH 7.4, 0.02% Tween®-20 (Merck), 45 mg/ml mannitol (pyrogen free, Novo Nordisk). Blood samples were drawn from a catheter in vena jugularis. 5 ml of the blood samples were poured into chilled glasses containing 175 µl of the following solution: 0.18 M EDTA, 15000 KIE/ml aprotinin (Novo Nordisk) and 0.30 mM Valine-Pyrrolidide (Novo Nordisk), pH 7.4. Within 30 min, the samples were centrifuged for 10 min at 5-6000*g. Temperature was kept at 4°C. The supernatant was pi-petted into different glasses and kept at minus 20°C until use.

The plasma concentrations of the peptides were determined in a sandwich ELISA or by RIA using different mono- or polyclonal antibodies. Choice of antibodies depends of the GLP-1 derivatives. The time at which the peak concentration in plasma is achieved varies within wide limits, depending on the particular GLP-1 derivative selected.

### General assay protocol for sandwich ELISA in 96-wells microtiterplate

| | |
|---|---|
| Coating buffer (PBS) | : Phosphate buffered saline, pH7.2 |
| Wash-buffer (PBS-wash) : | Phosphate buffered saline, 0.05 % v/v Tween 20, pH 7.2 |
| Assay-buffer (BSA-buffer): | Phosphate buffered saline, 10 g/l Bovin Serum Albumin (Fluka 05477), 0.05 % v/v Tween 20, pH 7.2 |
| Streptavidin-buffer: | Phosphate buffered saline, 0.5 M NaCl, 0.05 % v/v Tween 20, |
| | pH 7.2 |
| Standard: | Individual compounds in a plasma-matrix |
| A-TNP: | Nonsens antibody |
| AMDEX : | Streptavin-horseradish-peroxodase (Amersham RPN4401V) |
| TMB-substrate : | 3,3',5,5'tetramethylbenzidine (<0.02 %), hydrogen peroxide |

The assay was carried out as follows (volumen/well):
1.) coat with 100 µl catching antibody 5 µg/ml in PBS-buffer
   → incubate o/n , 4 °C
   → 5x PBS-wash → blocked with last wash in minimum 30 minute
   →then empty the plate
2.) 20 µl sample + 100 µl biotinylated detecting antibody 1 µg/ml in BSA-buffer with 10 µg/ml A-TNP
   → incubate 2 h, room temperature, on a shaker → 5x PBS-wash, then empty the plate
3.) 100 µl AMDEX 1:8000 in Streptavidin-buffer
   → incubate 45-60 minute, room temperature, on a shaker
   → 5x PBS-wash, then empty the plate
4.) 100 µl TMB-substrate
   → incubate x minute at room temperature on a shaker
   → stop the reaction with 100 µl 4 M H₃PO₄

Read the absorbance at 450 nm with 620 nm as reference

The concentration in the samples was calculated from standard curves.

### General assay protocol for RIA

| | |
|---|---|
| DB-buffer | : 80 mM phosphate buffer, 0.1 % Human serum albumin, 10 mM EDTA, 0.6 mM thiomersal, pH 7.5 |
| FAM-buffer | : 40 mM phosphate buffer, 0.1 % Human Serum Albumin, 0.6 mM thiomersal, pH 7.5 |
| Charcoal | : 40 mM phosphate buffer, 0.6 mM thiomersal, 16.7 % bovine plasma, 15 g/l activated carbon , pH 7.5 (mix the suspension minimum 1 h before use at 4 °C) |
| Standard | : Individual compounds in a plasma-matrix |

The assay was carried out in minisorp tubes 12x75 mm (volumen/tube) as follows:

| | **Db-buffer** | **SAMPLE** | **Antibody** | **FAM-buf.** | **Tracer** | **Charcoal** | **H₂O** |
|---|---|---|---|---|---|---|---|
| **Day 1** | | | | | | | |
| **Total** | | | | | 100 µL | | |
| **NSB** | 330 µL | | | 100 µL | | | |
| **Sample** | 300 µL | 30 µL | 100 µL | | 100 µL | | |

| **Mix, incubate o/n at 4 °C Day 2** | | | | | | | |
|---|---|---|---|---|---|---|---|
| **Total** | | | | | | | 1,5 mL |
| **NSB** | | | | | | 1,5 mL | |
| **Sample** | | | | | | 1,5 mL | |

Mix - incubate 30 min at 4 °C - centrifuge at 3000 rpm, 30 min - immediately after transfer supernatants to new tubes, close with stopper and count on gamma-counter for 1 minute. The concentration in the samples was calculated from individual standard curves.

### GLP-1 radio receptor assay (RRA):

The method is a radiometric-ligand binding assay using LEAD*seeker* imaging particles. The assay is composed of membrane fragments containing the GLP-1 receptor, unlabeled GLP-1 analogues, human GLP-1 labelled with ¹²⁵I and PS LEAD*seeker* particles coated with wheat germ agglutinin (WGA). Cold and ¹²⁵I-labelled GLP-1 will compete for the binding to the receptor. When the LEAD*seeker* particles are added they will bind to carbohydrates residues on the membrane fragments via the WGA-residues. The proximity between the ¹²⁵I-molecules and the LEAD*seeker* particles causes light emission from the particles. The LEADseeker will image the emitted light and it will be reversibly correlated to the amount of GLP-1 analogue present in the sample.

### Reagents & Materials:

*Pre treatment of animal plasma:* Animal plasma was heat treated for 4 hrs at 56°C and centrifuged at 10.000 rpm for 10 minutes. Afterwards, Val-Pyr (10 µM) and aprotenin (500 KIE/mL) was added and stored at <-18°C until use.

*GLP-1 analogues calibrators:* GLP-1 analogues were spiked into heat-treated plasma to produce dilution lines ranging from approximately 1 µM to 1 pM.

*GLP-1 RRA assay buffer:* 25 mM Na-HEPES (pH=7.5), 2.5 mM CaCl₂, 1 mM MgCl₂, 50 mM NaCl, 0.1% ovalbumin, 0.003% tween 20, 0.005% bacitracin, 0.05% NaN₃.

*GLP-1 receptor suspension:* GLP-1 receptor membrane fragments were purified from baby hamster kidney (BHK) cells expressing the human pancreatic GLP-1 receptor. Stored <-80°C until use.

*WGA-coupled polystyrene LEADseeker imaging beads (RPNQ0260, Amersham):* The beads were reconstituted with GLP-1 RRA assay buffer to a concentration of 13.3 mg/mL. The GLP-1 receptor membrane suspension was then added and incubated cold (2-8°C) at end-over-end for at least 1 hr prior to use.

*[^{,25}I]-GLP-1(7-36)amide (Novo Nordisk A*/*S).* Stored <-18°C until use.

*Ethanol* 99.9% *vol (De Dansk Spritfabrikker A*/*S):* Stored <-18°C until use.

*Multiscreen® Solvinert 0.45µm hydrophobic PTFE plates (MSRPN0450, Millipore Corp.) Poly propylene plates (cat. no. 650201, Greiner Bio-One)*
*White polystyrene 384-well plates (cat. no. 781075, Greiner Bio-One)*

### Apparatus:

Horizontal plate mixer
Centrifuge with a standard swinging-bucket microtitre plate rotor assembly
UltraVap - Drydown Sample Concentrator (Porvair)
LEADseeker™ Multimodality Imaging System (Amersham)

### Assay Procedure:

### Sample preparation:

Mount the MultiScreen® Solvinert filter plate on a chemical-comparable receiver plate (i.e. poly propylene plates) to collect the filtrate.

Add 150 µL ice-cold ethanol 99.9% into the empty wells of the MultiScreen® Solvinert filter plate followed by 50 µL calibrator or plasma sample. Place the storage lid on the filter plate. Incubate 15 minutes at 18-22°C on a horizontal plate mixer.

Place the assembled filter and receiver plate, with the lid, into a standard swinging-bucket microtitre plate rotor assembly. The filtrate is then collected in the empty wells of the receiver plate at 1500 rpm for 2 minutes.

Dry down the filtrate by using the UltraVap with heated (40°C) N₂ for duration of 15 miuntes. Reconstitute the dry material by adding 100 µL GLP-1 RRA assay buffer into each well. Incubate for 5 minutes on a horizontal mixer.

### GLP-1 radio receptor assay:

Use the following pipetting scheme and white polystyrene 384-well plates:
- 35 µL GLP-1 RRA assay buffer
- 5 µL reconstituted filtrate.
- 10 µL [¹²⁵I]-GLP-1(7-36)amide. The stock solution was diluted in GLP-1 RRA assay buffer to 20.000 cpm/well prior to use.
- 15 µL GLP-1 receptor membrane fragments (≈0.5 µg/well) pre-coated to WGA-polystyrene LEADseeker imaging beads (0.2 mg/well)

Seal the plates and incubate over night at 18-22°C

The light emission from each wells are detected by using the LEADseeker™ Multimodality Imaging System for duration of 10 minutes.

### Stimulation of cAMP formation in a cell line expressing the cloned human GLP-1 receptor.

Purified plasma membranes from a stable transfected cell line, BHK467-12A (tk-ts13), expressing the human GLP-1 receptor was stimulated with GLP-1 and peptide analogues, and the potency of cAMP production was measured using the AlphaScreen™ cAMP Assay Kit from Perkin Elmer Life Sciences.

A stable transfected cell line has been prepared at NN and a high expressing clone was selected for screening. The cells were grown at 5% CO₂ in DMEM, 5% FCS, 1% Pen/Strep and 0.5 mg/ml G418.

Cells at approximate 80% confluence were washed 2X with PBS and harvested with Versene, centrifuged 5 min at 1000 rpm and the supernatant removed. The additional steps were all made on ice. The cell pellet was homogenized by the Ultrathurax for 20-30 sec. in 10 ml of Buffer 1 (20 mM Na-HEPES, 10 mM EDTA, pH=7.4), centrifuged 15 min at 20.000 rpm and the pellet resuspended in 10 ml of Buffer 2 (20 mM Na-HEPES, 0.1 mM EDTA, pH=7.4). The suspension was homogenized for 20-30 sec and centrifuged 15 min at 20.000 rpm. Suspension in Buffer 2, homogenization and centrifugation was repeated once and the membranes were resuspended in Buffer 2 and ready for further analysis or stored at -80°C. The functional receptor assay was carried out by measurering the peptide induced cAMP production by The AlphaScreen Technology. The basic principle of The AlphaScreen Technology is a competition between endogenous cAMP and exogenously added biotin-cAMP. The capture of cAMP is achieved by using a specific antibody conjugated to acceptor beads. Formed cAMP was counted and measured at a AlphaFusion Microplate Analyzer. The EC₅₀ values was calculated using the Graph-Pad Prisme software.

## Claims

1. A compound which has the formula (I):
A-W-B-Y-therapeutic polypeptide (I)
wherein
said polypeptide is a GLP-1 peptide comprising the amino acid sequence of formula (V):
Xaa₇-Xaa₈-Glu-Gly-Thr-Phe-Thr-Ser-Asp-Val-Ser-Xaa₁₈-Tyr-Leu-Glu-Xaa₂₂-Xaa₂₃-Ala-Ala-Xaa₂₆-Glu-Phe-Ile-Xaa₃₀-Trp-Leu-Val-Xaa₃₄-Xaa₃₅-Xaa₃₆-Xaa₃₇-Xaa₃₈
Formula (V) (SEQ ID No: 3)
wherein
Xaa₇ is L-histidine, D-histidine, desamino-histidine, 2-amino-histidine, β-hydroxy-histidine, homohistidine, N^{α}-acetyl-histidine, α-fluoromethyl-histidine, α-methyl-histidine, 3-pyridylalanine, 2-pyridylalanine or 4-pyridylalanine;
Xaa₈ is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid;
Xaa₁₈ is Ser, Lys or Arg;
Xaa₂₂ is Gly, Glu or Aib;
Xaa₂₃ is Gln, Glu, Lys or Arg;
Xaa₂₆ is Lys, Glu or Arg;
Xaa₃₀ is Ala, Glu or Arg;
Xaa₃₄ is Lys, Glu or Arg;
Xaa₃₅ is Gly or Aib;
Xaa₃₆ is Arg or Lys;
Xaa₃₇ is Gly, Ala, Glu or Lys;
Xaa₃₈ is Lys, amide or is absent;
A is an albumin binding residue selected from the group consisting of 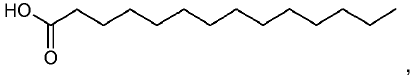 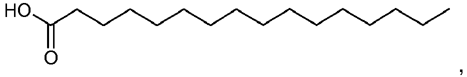 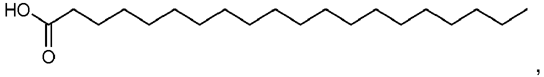 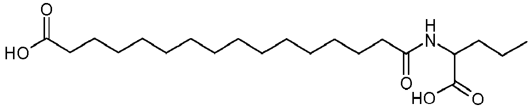 where the chiral carbon atom is either R or S, 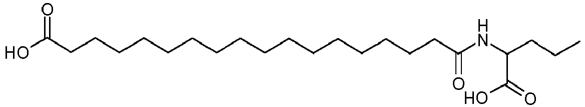 where the chiral carbon atom is either R or S, 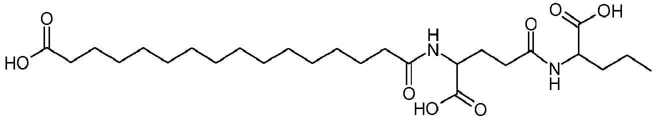 where the two chiral carbon atoms independently are either R or S, 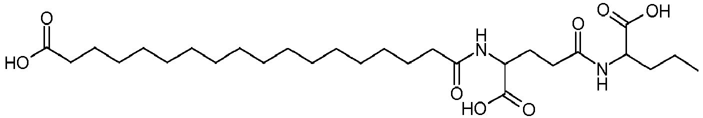 where the two chiral carbon atoms independently are either R or S, 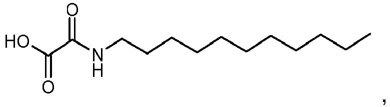 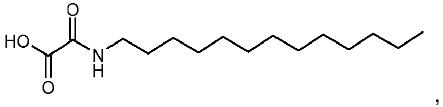 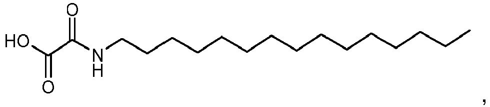 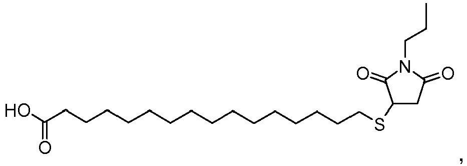 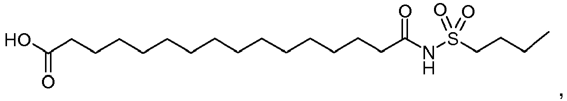 and 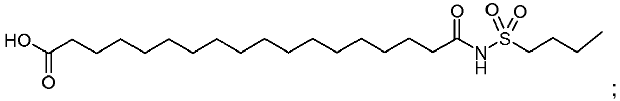
B is -(CH₂)ₗ-O-[(CH₂)ₙ-O]ₘ-(CH₂)ₚ-[C(O)NH-(CH₂)ₗ-O-[(CH₂)ₙ-O]ₘ-(CH₂)ₚ]_{q}-, where l, m, n, and p independently are 1-5, and q is 0-5;
Y is a chemical group linking B and the therapeutic agent, selected from the group consisting of -C(O)NH-, -NHC(O)-, -C(O)NHCH₂-, -CH₂NHC(O)-, -OC(O)NH -, -NHC(O)O-, -C(O)NHCH₂-, CH₂NHC(O)-, -C(O)CH₂-, -CH₂C(O)-, -C(O)CH=CH-, -CH=CHC(O)-, -(CH₂)ₛ- , -C(O)-, -C(O)O-, -OC(O)-, -NHC(O)- and -C(O)NH-, wherein s is 0 or 1;
and
W is a chemical group linking A and B, selected from the group consisting of -C(O)NH-, - NHC(O)-, -C(O)NHCH₂-, -CH₂NHC(O)-, -OC(O)NH -, -NHC(O)O-, -C(O)CH₂-, -CH₂C(O)-, -C(O)CH=CH-, -CH=CHC(O)-, -(CH₂)ₛ-, -C(O)-, -C(O)O-, -OC(O)-, -NHC(O)- and -C(O)NH- , wherein s is 0 or 1;
or a pharmaceutically acceptable salt thereof.

2. A compound according to claim 1, wherein i) q is 0 or 1; ii) q is 1; or iii) q is 0.

3. A compound according to any one of the previous claims, wherein I is 2; and/or wherein n is 2.

4. A compound according to any one of the preceding claims, wherein -W-B-Y- is selected from the group consisting of 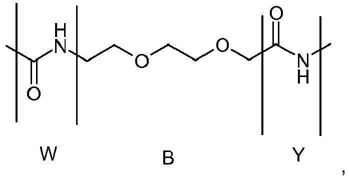 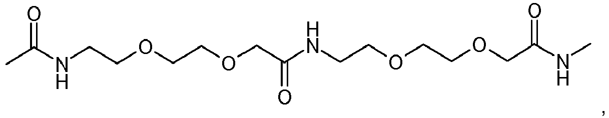 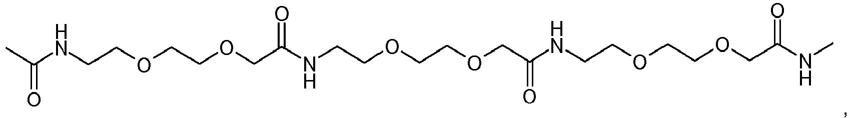 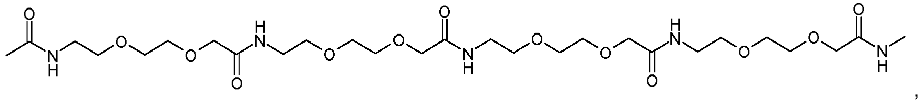 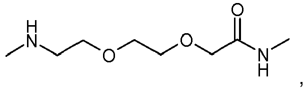 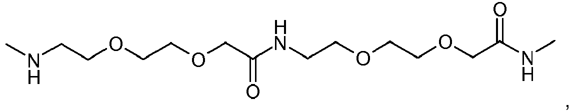 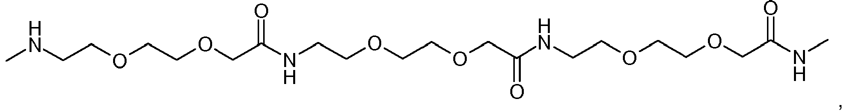 and

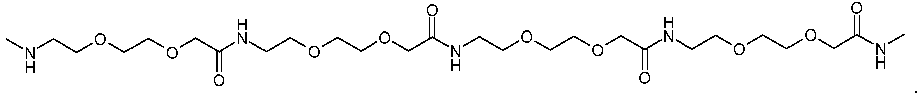

5. A compound according to any one of the previous claims, wherein the albumin binding residue i) via spacer and linkers is attached to said therapeutic polypeptide via the ε-amino group of a lysine residue; and/or ii) via a linker to an amino acid residue selected from cysteine, glutamate and aspartate.

6. A compound according to any one of the previous claims, wherein said GLP-1 peptide comprises i) no more than ten amino acid residues which have been exchanged, added or deleted as compared to GLP-1 (7-37) (SEQ ID No. 1); ii) preferably no more than six amino acid residues which have been exchanged, added or deleted as compared to GLP-1 (7-37) (SEQ ID No. 1); and/or iii) no more than 4 amino acid residues which are not encoded by the genetic code.

7. A compound according to any one of the previous claims, wherein i) said GLP-1 peptide comprises an Aib residue in position 8; and/or ii) wherein the amino acid residue in position 7 of said GLP-1 peptide is selected from the group consisting of D-histidine, desamino-histidine, 2-amino-histidine, β-hydroxy-histidine, homohistidine, N^{α}-acetyl-histidine , α-fluoromethyl-histidine, α-methyl-histidine, 3-pyridylalanine, 2-pyridylalanine and 4-pyridylalanine.

8. A compound according to any one of the previous claims, wherein said GLP-1 peptide is selected from the group consisting of Arg³⁴GLP-1(7-37),
Lys³⁸Arg^{26,34}GLP-1(7-38), Lys³⁸Arg^{26,34}GLP-1(7-38)-OH, Lys³⁶Arg^{26,34}GLP-1(7-36),
Aib^{8,22,35} GLP-1 (7-37), Aib^{8,35} GLP-1 (7-37), Aib^{8,22} GLP-1 (7-37),
Aib^{8,22,35} Arg^{26,34}Lys³⁸GLP-1(7-38), Aib^{8,35}Arg^{26,34}Lys³⁸GLP-1(7-38),
Aib^{8,22}Arg^{26,34}Lys³⁸GLP-1(7-38), Aib^{8,22,35}Arg^{26,34}Lys^{3s}GLP-1(7-38),
Aib^{8,35}Arg^{26,34}Lys³⁸GLP-1(7-38), Aib^{8,22,35}Arg²⁶Lys³⁸GLP-1(7-38),
Aib^{8,35}Arg²⁶Lys³⁸GLP-1(7-38), Aib^{8,22}Arg²⁶ LyS³⁸GLP-1(7-38),
Aib^{8,22,35}Arg³⁴Lys³⁸GLP-1(7-38), Aib^{8,35}Arg³⁴Lys³⁸GLP-1(7-38), Aib^{8,22}Arg³⁴Lys³⁸GLP-1(7-38), Aib^{8,22,35}Ala³⁷Lys³⁸GLP-1(7-38), Aib^{8,35}Ala³⁷Lys³⁸GLP-1(7-38), Aib^{8,22}Ala³⁷Lys³⁸GLP-1(7-38), Aib^{8,22,35}Lys³⁷GLP-1(7-37), Aib^{8,35}Lys³⁷GLP-1(7-37) and Aib^{8,22}Lys³⁷GLP-1(7-38).

9. A compound according to any one of the previous claims, wherein said GLP-1 peptide is attached to said hydrophilic spacer via the amino acid residue in position 23, 26, 34, 36 or 38 relative to the amino acid sequence SEQ ID No:1.

10. A compound according to any one of the previous claims, wherein one albumin binding residue via said hydrophilic spacer is attached to the C-terminal amino acid residue of said GLP-1 peptide.

11. A compound according to claim 10, wherein a second albumin binding residue is attached to an amino acid residue which is not the C-terminal amino acid residue.

12. A compound according to any one of the previous claims, wherein said compound is selected from the group consisting of
N^{ε7}--{2-[2-(2-(15-carboxypentadecanoylamino)ethoxy)ethoxy]acetyl}-[Aib^{8,22,35},Lys³⁷] GLP-1(7-37)amide 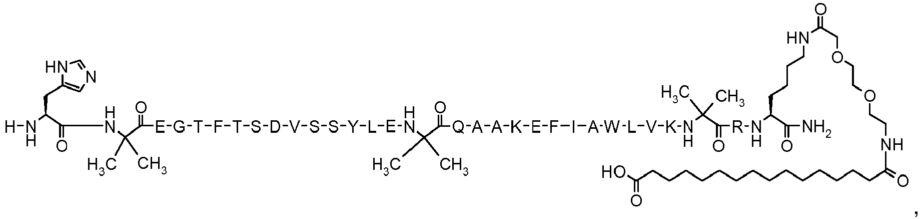
N^{ε37}-(2-(2-(2-(17-carboxyheptadecanoylamino)ethoxy)ethoxy)acetyl)[Aib^{8,22,35},Lys³⁷]GLP-1(7-37)amide, 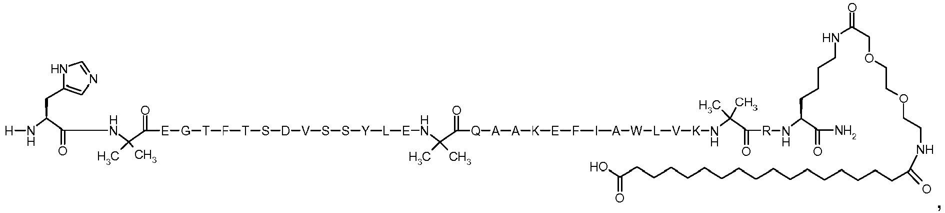
N^{ε37}-(2-(2-(2-(19-carboxynonadecanoylamino)ethoxy)ethoxy)acetyl)[Aib^{8,22,35},Lys³⁷]GLP-1(7-37)amide, 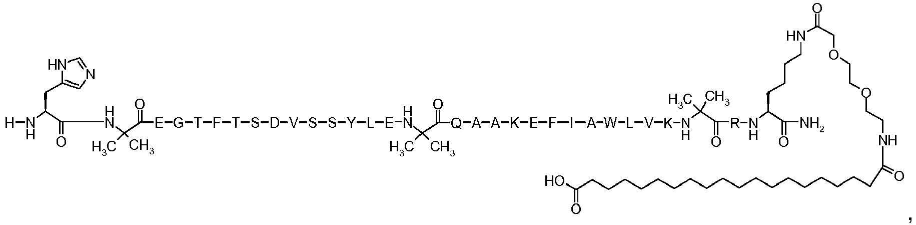
[Aib^{8,22,35}]GLP-1(7-37)Lys(({2-[2-(2-{2-[2-(2-{2-[2-(15-carboxypentadecanoylamino)-ethoxy]ethoxy}acetylamino)ethoxy]ethoxy}acetyl amino)ethoxy]ethoxy}acetyl))amide, 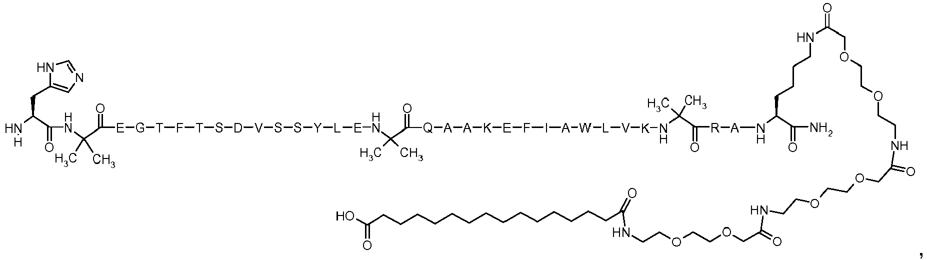
N^{ε37-}([2-(2-{3-[2,5-dioxo-3-(15-carboxypentadecylsulfanyl)-pyrrolidin-1-yl]-propionylamino}ethoxy)ethoxy)acetyl]-[D-Ala⁸,Lys³⁷]-GLP-1-[7-37]amide, 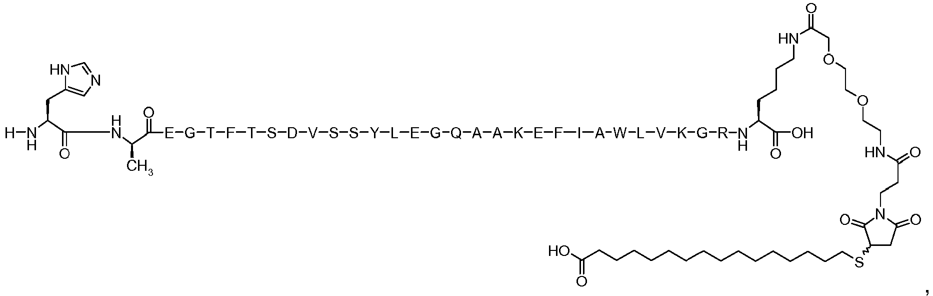
[Aib^{8,22,35}Ala³⁷]GLP-1(7-37)Lys((2-(2-(2-(11-(oxalylamino)undecanoylamino)ethoxy)ethoxy)acetyl-)))amide, 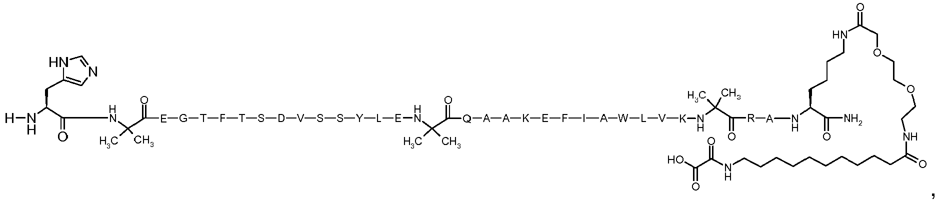
[Aib^{8,22,35}Ala³⁷]-GLP-1(7-37)Lys({2-[2-(2-{2-[2-(2-(15-carboxy-pentadecanoylamino)-ethoxy]ethoxy}acetylamino)ethoxy]ethoxy}acetyl)amide, 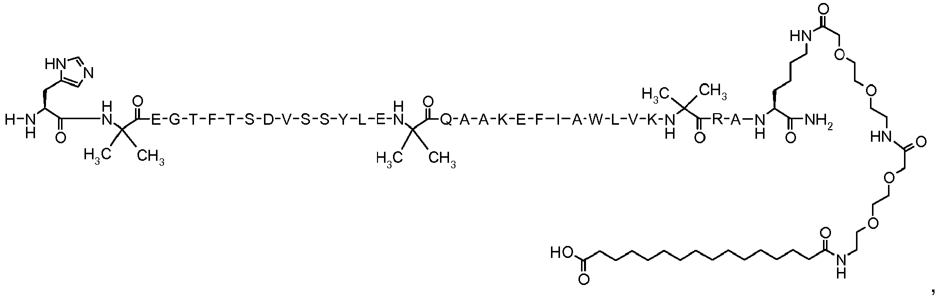
[Aib⁸,Arg^{26,34}] GLP-1 (7-37)Lys{2-(2-(2-(2-[2-(2-(17-carboxyheptanoylamino)ethoxy)ethoxy]acetylamino)ethoxy)ethoxy)acetyl)}-OH, 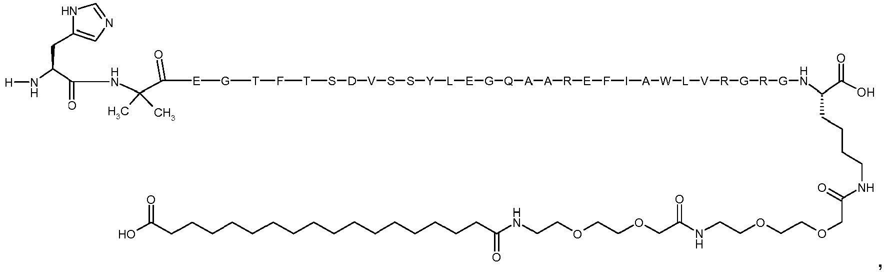
[Gly⁸, Arg^{26,34}] GLP1-(7-37) Lys{2-(2-(2-(2-[2-(2-(17-carboxyheptadecanoylamino)ethoxy)ethoxy]acetyl)ethoxy)ethoxy)acetyl)}-OH, 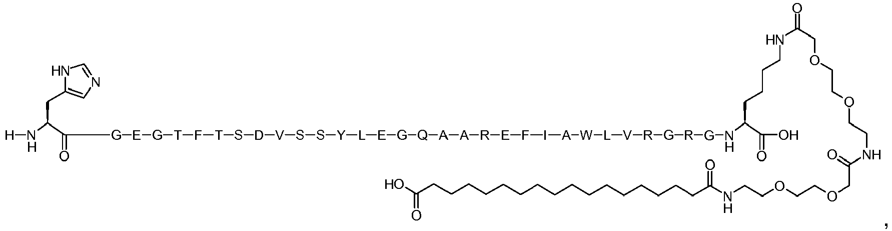 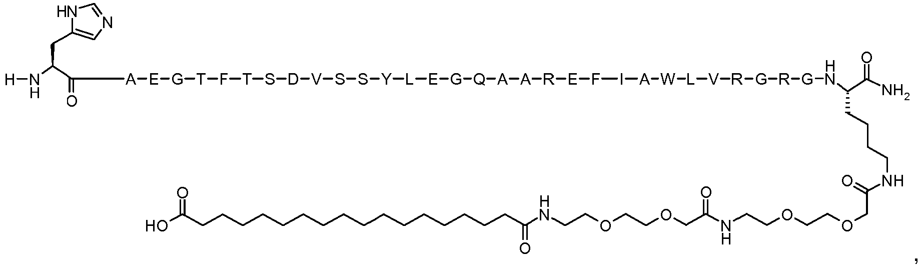
[Gly⁸,Arg^{26,34}]GLP-1(7-37)Lys(2-(2-(19-(carboxy)nonadecanoylamino)ethoxy)ethoxy)acetyl)-OH, 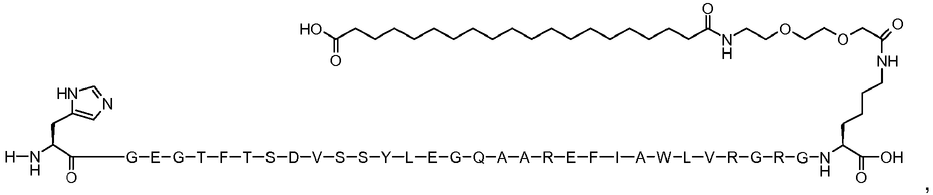
[Gly⁸,Arg^{26,34}]GLP-1(7-37)Lys((2-(2-(17-(carboxy)heptadecanoylamino)ethoxy)ethoxy)acetyl))-OH, 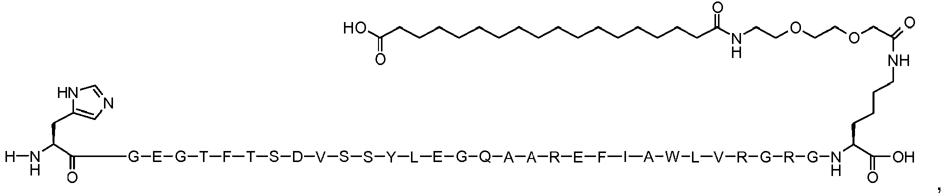
[Gly⁸,Arg^{26,34}]GLP-1(7-37)Lys(2-(2-(2-(4-(19-(carboxy)nonadecanoylamino)-4-carboxybutyrylamino)ethoxy)ethoxy)acetyl)-OH, 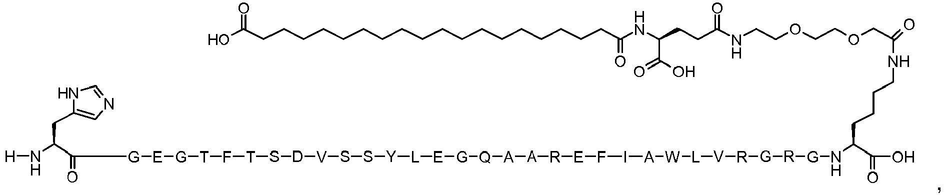 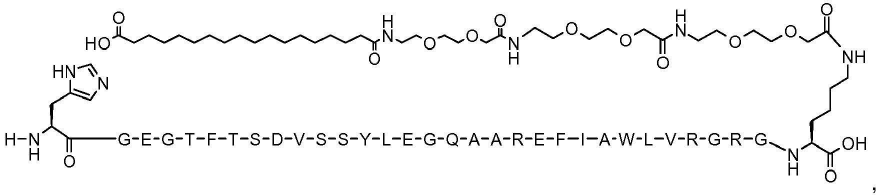
N^{ε20}(2-(2-(2-(2-(2-(2-(2-(2-(2-(2-(2-(2-(4-(17-(carboxy)heptadecanoylamino)-4-carboxybutyηlamino)ethoxy)ethoxy)acetylamino)ethoxy)ethoxy)acetylamino) ethoxy)ethoxy)acetylamino)ethoxy)ethoxy)acetyl) [Lys²⁰]exendin-4 (1-39)-NH₂, 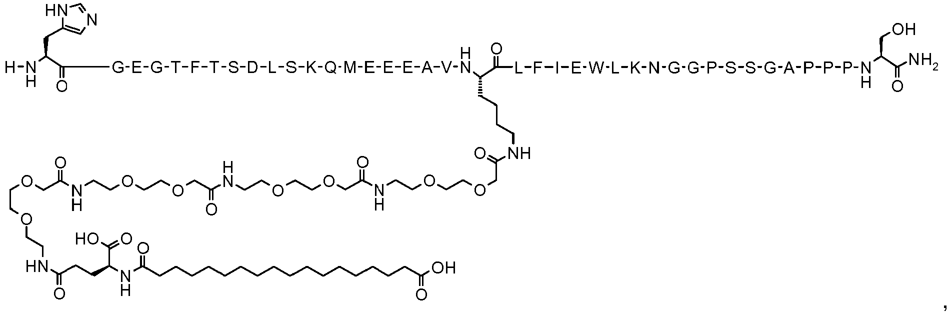
N^{ε36}-(2-(2-(2-(2-(2-(2-(17-Carboxyheptadecanoylamino)ethoxy)ethoxy) acetylamino)ethoxy)ethoxy)acetyl) [Aib⁸,Arg^{26,34}, Lys³⁶] GLP-1 (7-37), 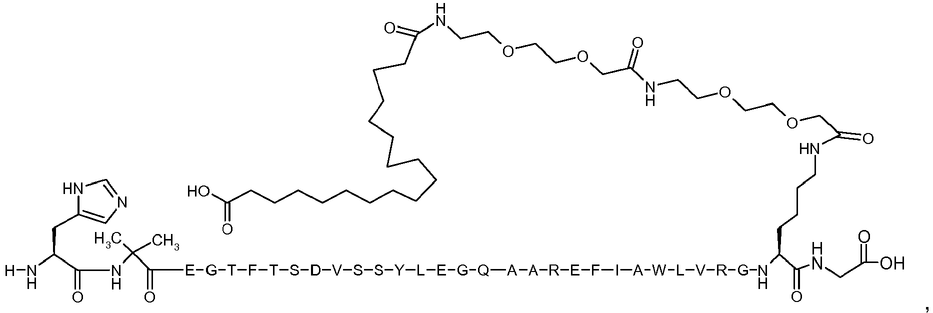
N-^{ε36}-(2-(2-(2-(2-(2-(2-(17-Carboxyheptadecanoylamino)ethoxy)ethoxy) acetylamino)ethoxy)ethoxy)acetyl) [Arg^{26,34}, Lys³⁶] GLP-1 (7-37), 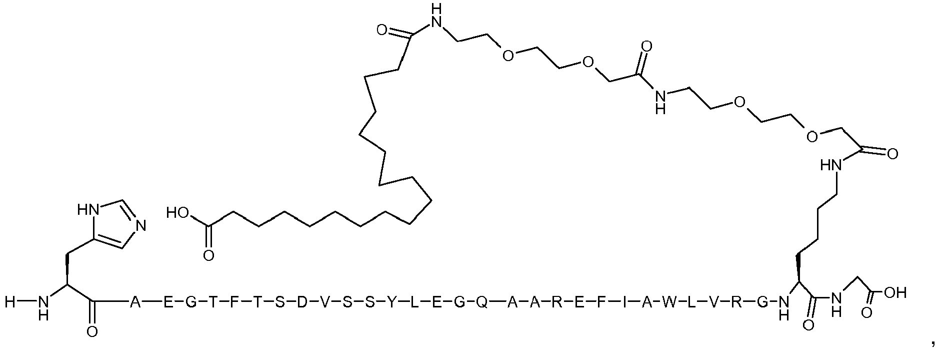
N^{ε36}-(2-(2-(2-(2-(2-(2-(17-Carboxyheptadecanoylamino)ethoxy)ethoxy) acetylamino)ethoxy)ethoxy)acetyl) [Gly⁸,Arg^{26,34},Lys³⁶] GLP-1 (7-37), 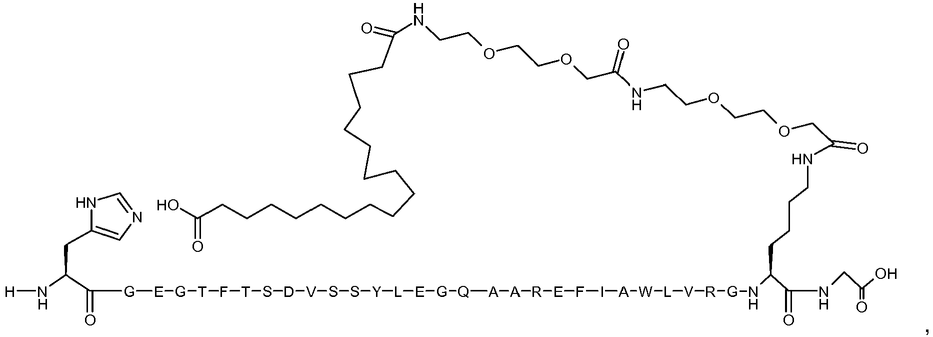
N^{ε26}-(2-[2-(2-[2-(2-[2-(17-Carboxyheptadecanoylamino)ethoxy] ethoxy)acetylamino]ethoxy)ethoxy]acetyl)[Arg³⁴]GLP-1-(7-37)-OH, 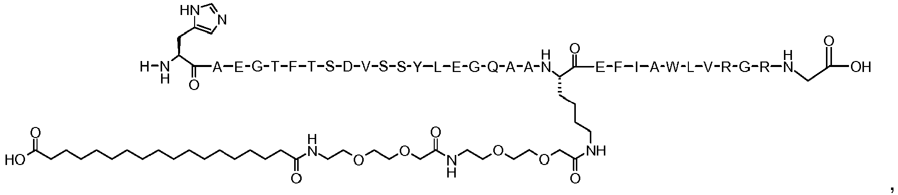
N^{ε26}-[2-(2-[2-(2-[2-(2-[4-(17-Carboxyheptadecanoylamino)-4(S)-carboxybutyrylamino]ethoxy)ethoxy]acetylamino)ethoxy]ethoxy)acetyl][Arg³⁴]GLP-1-(7-37)-OH, 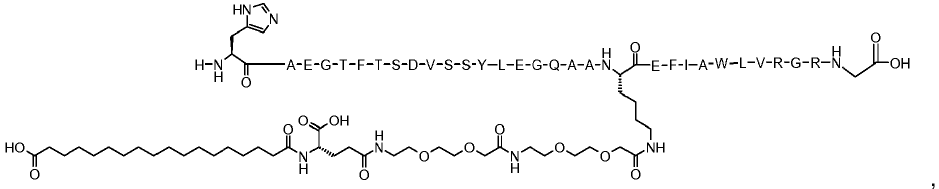
N^{ε20}-(2-(2-(2-(2-(2-(2-(2-(2-(2-(17-Carboxyheptadecanoylamino)ethoxy)ethoxy)acetylamino)ethoxy)ethoxy)acetylamino)ethoxy)ethoxy)acetyl)[Lys²⁰] Exendin-4 (1-39) amide, 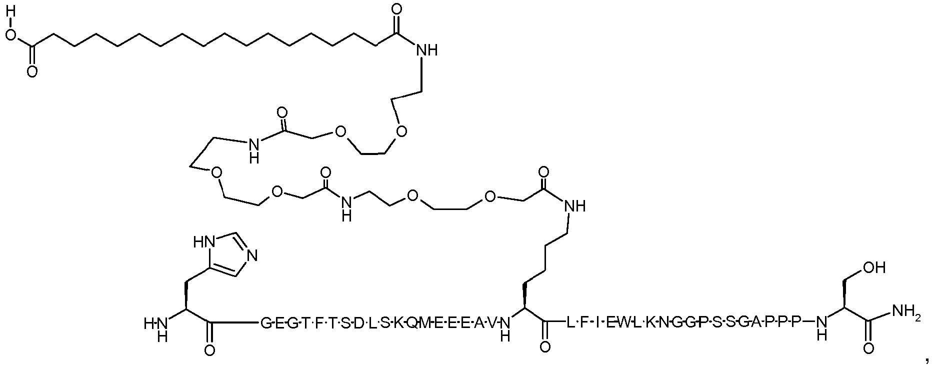
[Gly⁸, Glu^{22,23,30}, Arg^{18,26,34}]GLP1 (7-37) Lys(2-(2-(2-(2-(2-(2-(17-carboxyheptadecanoylamino)ethoxy)ethoxy)acetylamino)ethoxy))ethoxy)acetyl)-NH₂, 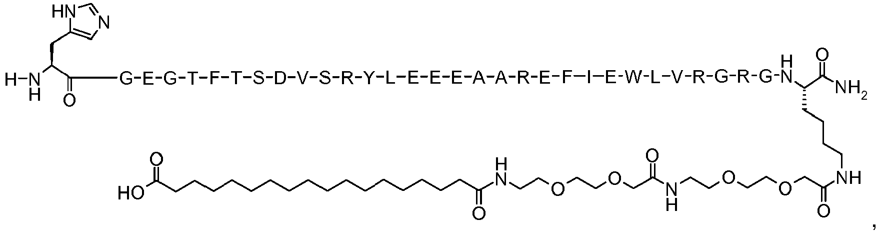
[Imidazolylpropionic acid⁷, Asp¹⁶, Aib^{22,35} ]GLP1(7-37)Lys NH((2-{[4-(17-carboxyheptadecanoylamino)butylcarbamoyl]methoxy}ethoxy)ethoxy)), 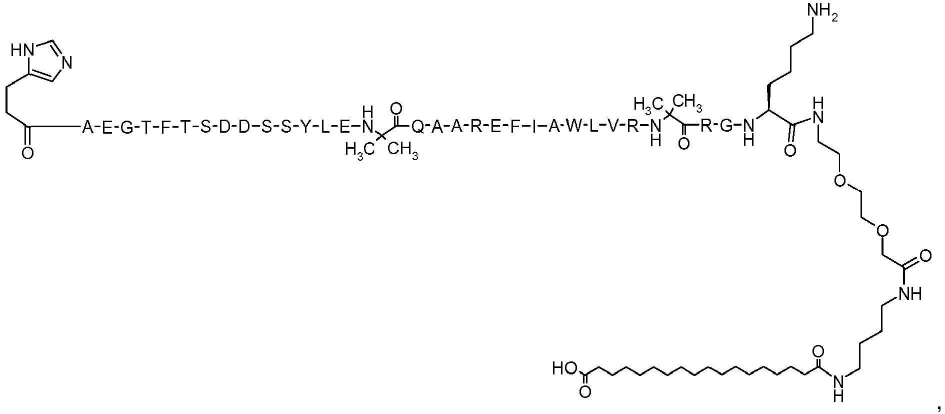
[Imidazolylpropionic acid⁷, Aib^{22,35} ]GLP1(7-37)Lys NH( (2-{[4-(17-carboxyheptadecanoylamino)butylcarbamoyl]methoxy}ethoxy)ethoxy)), 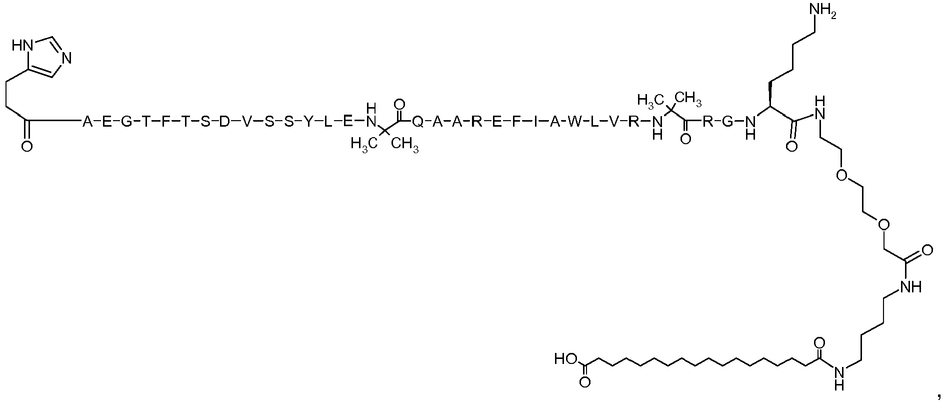 and
[3-(5-Imidazoyl)propionyl⁷, Aib⁸, Arg^{26,34} ] GLP-1 (7-37)Lys{2-(2-(2-(2-[2-(2-(17-carboxyheptanoylamino)ethoxy)ethoxy]acetylamino)ethoxy)ethoxy)acetyl)}-OH,

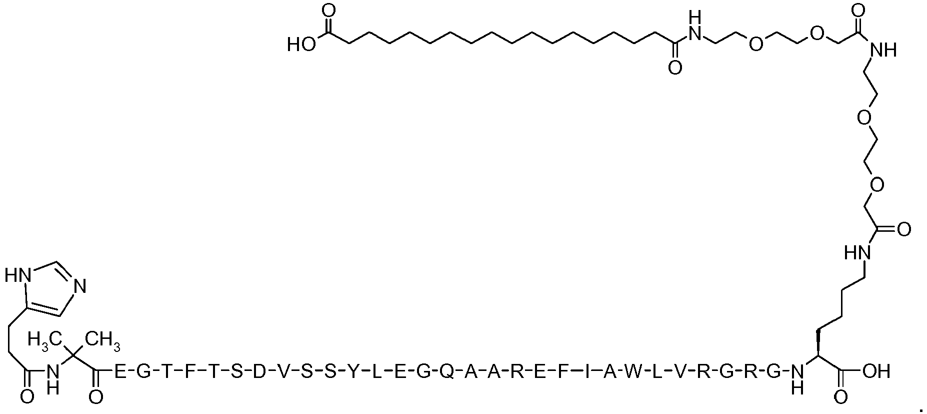

13. A pharmaceutical composition comprising a compound according to any one of claims 1-12, and a pharmaceutically acceptable excipient.

14. A compound according to any of claims 1-12 for use as a medicament.

15. Use of a compound according to any one of the claims 1-12 for the preparation of a medicament; preferably i) for the treatment or prevention of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, stroke, inflammatory bowel syndrome, dyspepsia and gastric ulcers; ii) for the preparation of a medicament for delaying or preventing disease progression in type 2 diabetes; and/or iii) for the preparation of a medicament for decreasing food intake, decreasing β-cell apoptosis, increasing β-cell funtion and β-cell mass, and/or for restoring glucose sensitivity to β-cells.

## Patentansprüche

1. Verbindung mit der Formel (I):
A-W-B-Y- Therapeutisches Polypeptid (I)
wobei
das Polypeptid ein GLP-1-Peptid ist, das die Aminosäuresequenz der Formel (V) umfasst:
Xaa₇-Xaa₈-Glu-Gly-Thr-Phe-Thr-Ser-Asp-Val-Ser-Xaa₁₈-Tyr-Leu-Glu-Xaa₂₂-Xaa₂₃-Ala-Ala-Xaa₂₆₋Glu-Phe-Ile-Xaa₃₀-Trp-Leu-Val-Xaa₃₄-Xaa₃₅-Xaa₃₆-Xaa₃₇-Xaa₃₈
Formel (V) (SEQ ID NO: 3)
wobei
Xaa₇ L-Histidin, D-Histidin, Desamino-Histidin, 2-Amino-Histidin, β-Hydroxy-Histidin, Homohistidin, N^{α}-Acetyl-Histidin, α-Fluormethyl-Histidin, α-Methyl-Histidin, 3-Pyridylalanin, 2-Pyridylalanin oder 4-Pyridylalanin ist;
Xaa₈ Ala, Gly, Val, Leu, Ile, Lys, Aib, (1-Aminocyclopropyl)carbonsäure, (1-Aminocyclobutyl)carbonsäure, (1-Aminocyclopentyl)carbonsäure, (1-Aminocyclohexyl)carbonsäure, (1-Aminocycloheptyl)carbonsäure oder (1-Aminocyclooctyl)carbonsäure ist;
Xaa₁₈ Ser, Lys oder Arg ist;
Xaa₂₂ Gly, Glu oder Aib ist;
Xaa23 Gln, Glu, Lys oder Arg ist;
Xaa26 Lys, Glu oder Arg ist;
Xaa₃₀ Ala, Glu oder Arg ist;
Xaa34 Lys, Glu oder Arg ist;
Xaa35 Gly oder Aib ist;
Xaa36 Arg oder Lys ist;
Xaa₃₇ Gly, Ala, Glu oder Lys ist;
Xaa₃₈ Lys, Amid oder nicht vorhanden ist;
A ein albuminbindender Rest ist, ausgewählt aus der Gruppe, bestehend aus 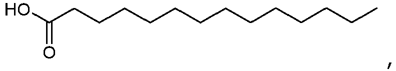 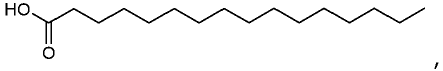 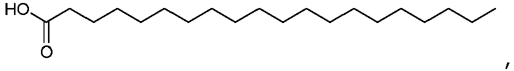 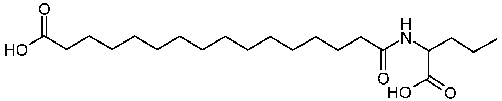 wobei das chirale Kohlenstoffatom entweder R oder S ist, 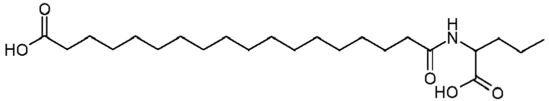 wobei das chirale Kohlenstoffatom entweder R oder S ist, 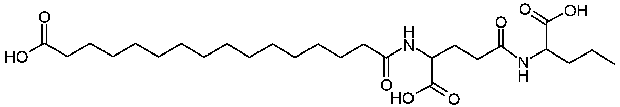 wobei die beiden chiralen Kohlenstoffatome unabhängig voneinander entweder R oder S sind, 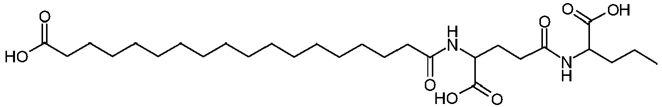 wobei die beiden chiralen Kohlenstoffatome unabhängig voneinander entweder R oder S sind, 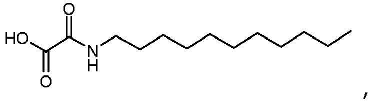 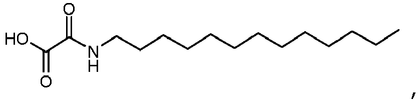 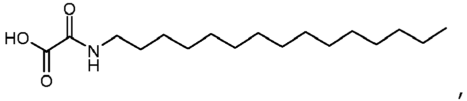 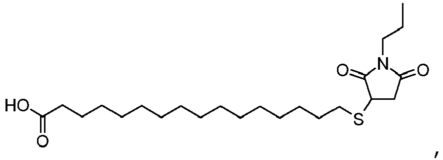 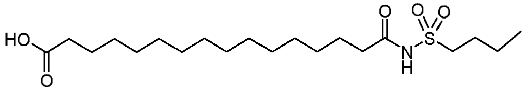 und 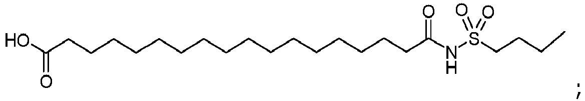
B -(CH₂)ₗ-O-[(CH₂)ₙ-O]ₘ-(CH₂)ₚ-[C(O)NH-(CH₂)ₗ-O-[(CH₂)ₙ-O]ₘ-(CH₂)ₚ]_{q}- ist, wobei l, m, n und p unabhängig voneinander 1-5 sind und q 0-5 ist;
Y eine chemische Gruppe ist, die B und das therapeutische Mittel verbindet, ausgewählt aus der Gruppe, bestehend aus -C(O)NH-, -NHC(O)-, -C(O)NHCH₂-, -CH₂NHC(O)-, -OC(O)NH -, -NHC(O)O-, -C(O)NHCH₂-, CH₂NHC(O)-, -C(O)CH₂-, -CH₂C(O)-, -C(O)CH=CH-, - CH=CHC(O)-, -(CH₂)ₛ-, -C(O)-, -C(O)O-, -OC(O)-, -NHC(O)- und -C(O)NH-, wobei s 0 oder 1 ist;
und
W eine chemische Gruppe ist, die A und B verbindet, ausgewählt aus der Gruppe, bestehend aus -C(O)NH-, -NHC(O)-, -C(O)NHCH₂-, -CH₂NHC(O)-, -OC(O)NH -, -NHC(O)O-, -C(O)CH₂-, -CH₂C(O)-, -C(O)CH=CH-, -CH=CHC(O)-, -(CH₂)ₛ-, -C(O)-, -C(O)O-, -OC(O)-, -NHC(O)- und -C(O)NH-, wobei s 0 oder 1 ist;
oder ein pharmazeutisch unbedenkliches Salz davon.

2. Verbindung nach Anspruch 1, wobei i) q 0 oder 1 ist; ii) q 1 ist; oder iii) q 0 ist.

3. Verbindung nach einem der vorhergehenden Ansprüche, wobei I 2 ist; und/oder wobei n 2 ist.

4. Verbindung nach einem der vorhergehenden Ansprüche, wobei -W-B-Y- ausgewählt ist aus der Gruppe, bestehend aus 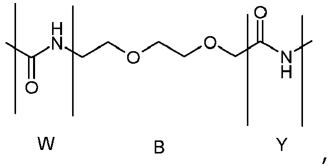 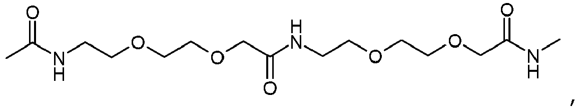 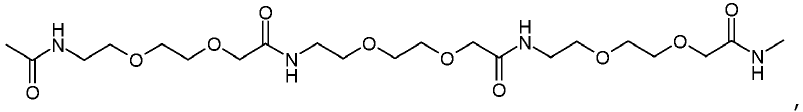 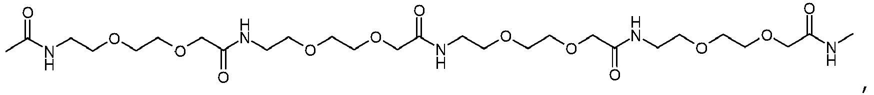 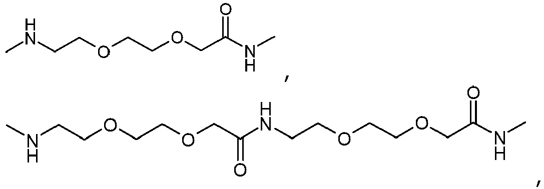 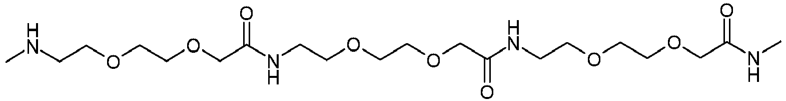 und

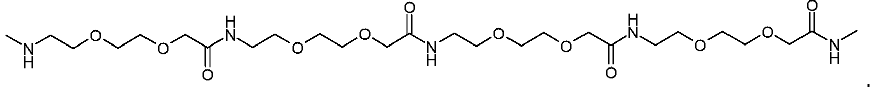

5. Verbindung nach einem der vorhergehenden Ansprüche, wobei der albuminbindende Rest i) über Spacer und Linker an das therapeutische Polypeptid über die ε-Aminogruppe eines Lysinrestes gebunden ist; und/oder ii) über einen Linker an einen Aminosäurerest, ausgewählt aus Cystein, Glutamat und Aspartat.

6. Verbindung nach einem der vorhergehenden Ansprüche, wobei das GLP-1-Peptid umfasst: i) nicht mehr als zehn Aminosäurereste, die im Vergleich zu GLP-1(7-37) (SEQ ID Nr. 1) ausgetauscht, hinzugefügt oder entfernt wurden; ii) bevorzugt nicht mehr als sechs Aminosäurereste, die im Vergleich zu GLP-1(7-37) (SEQ ID Nr. 1) ausgetauscht, hinzugefügt oder entfernt wurden; und/oder iii) nicht mehr als 4 Aminosäurereste, die nicht durch den genetischen Code kodiert sind.

7. Verbindung nach einem der vorhergehenden Ansprüche, wobei i) das GLP-1-Peptid einen Aib-Rest in Position 8 umfasst; und/oder ii) wobei der Aminosäurerest in Position 7 des GLP-1-Peptids ausgewählt ist aus der Gruppe, bestehend aus D-Histidin, Desamino-Histidin, 2-Amino-Histidin, β-Hydroxy-Histidin, Homohistidin, N^{α}-Acetyl-Histidin , α-Fluormethyl-Histidin, α-Methyl-Histidin, 3-Pyridylalanin, 2-Pyridylalanin und 4-Pyridylalanin.

8. Verbindung nach einem der vorhergehenden Ansprüche, wobei das GLP-1-Peptid ausgewählt ist aus der Gruppe, bestehend aus Arg³⁴GLP-1(7-37),
Lys³⁸Arg^{26,34}GLP-1(7-38), Lys³⁸Arg^{26,34}GLP-1(7-38)-OH, Lys³⁶Arg^{26,34}GLP-1(7-36), Aib^{8,22,35}GLP-1(7-37), Aib^{8,35} GLP-1(7-37), Aib^{8,22} GLP-1(7-37), Aib^{8,22,35}Arg^{26,34}Lys³⁸GLP-1(7-38),Aib^{8,35}Arg^{26,34}Lys³⁸GLP-1(7-38), Aib^{8,22}Arg^{26,34}Lys³⁸GLP-1(7-38),Aib^{8,22,35}Arg^{26,34}LyS³⁸GLP-1(7-38), Aib^{8,35}Arg^{26,34}Lys³⁸GLP-1(7-38),Aib^{8,22,35}Arg²⁶Lys³⁸GLP-1(7-38), Aib^{8,35}Arg²⁶Lys³⁸GLP-1(7-38), Aib^{8,22} Arg²⁶Lys³⁸GLP-1(7-38), Aib^{8,22,35}Arg³⁴Lys³⁸GLP-1(7-38), Aib^{8,35}Arg³⁴Lys³⁸GLP-1(7-38), Aib^{8,22}Arg³⁴Lys³⁸GLP-1(7-38), Aib^{8,22,35}Ala³⁷Lys³⁸GLP-1(7-38), Aib^{8,35}Ala³⁷Lys³⁸GLP-1(7-38), Aib^{8,22}Ala³⁷Lys³⁸GLP-1(7-38), Aib^{8,22,35}Lys³⁷GLP-1(7-37), Aib^{8,35}Lys³⁷GLP-1(7-37) und Aib^{8,22}Lys³⁷GLP-1(7-38).

9. Verbindung nach einem der vorhergehenden Ansprüche, wobei das GLP-1-Peptid an den hydrophilen Spacer über den Aminosäurerest in Position 23, 26, 34, 36 oder 38, bezogen auf die Aminosäuresequenz SEQ ID No:1, gebunden ist.

10. Verbindung nach einem der vorhergehenden Ansprüche, wobei ein albuminbindender Rest über den hydrophilen Spacer an den C-terminalen Aminosäurerest des GLP-1-Peptids gebunden ist.

11. Verbindung nach Anspruch 10, wobei ein zweiter albuminbindender Rest an einen Aminosäurerest gebunden ist, der nicht der C-terminale Aminosäurerest ist.

12. Verbindung nach einem der vorhergehenden Ansprüche, wobei die Verbindung ausgewählt ist aus der Gruppe, bestehend aus
N^{ε37}-{2-[2-(2-(15-Carboxypentadecanoylamino)ethoxy)ethoxy]acetyl}-[Aib^{8,22,35},Lys³⁷] GLP-1(7-37)amid 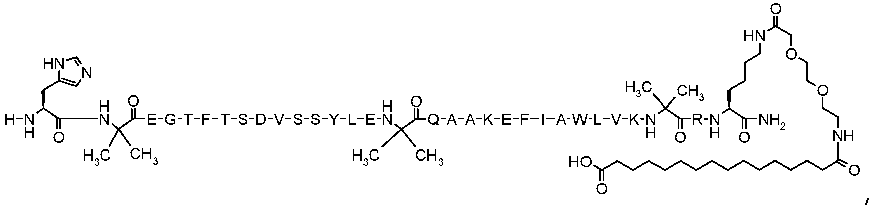
N^{ε37}-(2-(2-(2-(17-Carboxyheptadecanoylamino)ethoxy)ethoxy)acetyl)[Aib^{8,22,35},Lys³⁷]GLP-1(7-37)amid, 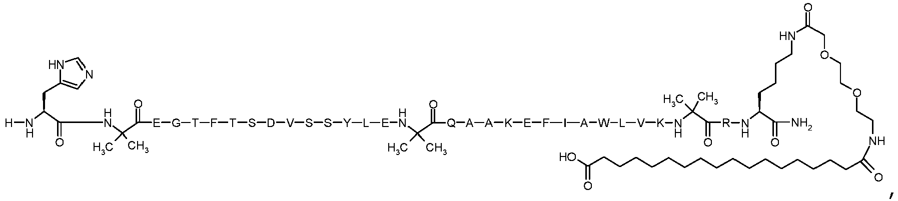
N^{ε37-}(2-(2-(2-(19-Carboxynonadecanoylamino)ethoxy)ethoxy)acetyl)[Aib^{8,22,35},Lys³⁷]GLP-1(7-37)amid, 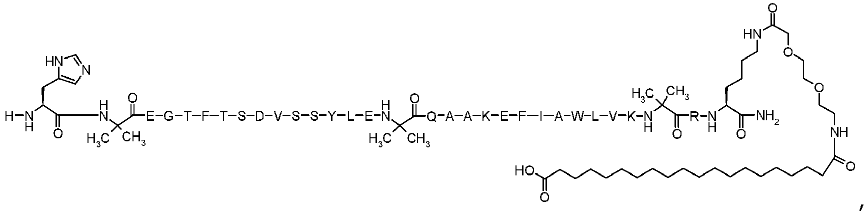
[Aib^{8,22,35}]GLP-1(7-37)Lys(({2-[2-(2-{2-[2-(2-{2-[2-(15-Carboxypentadecanoylamino)-ethoxy]ethoxy}acetylamino)ethoxy]ethoxy}acetyl amino)ethoxy]ethoxy}acetyl))amid, 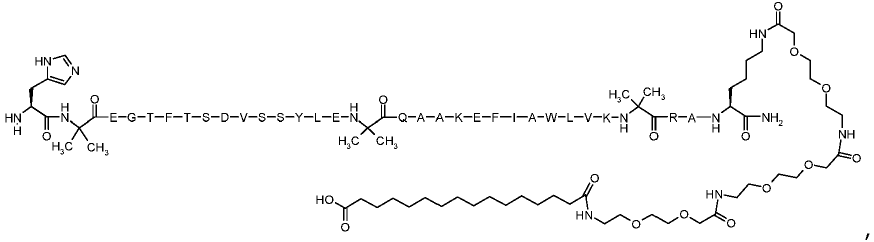
N^{ε37}-([2-(2-{3-[2,5-Dioxo-3-(15-Carboxypentadecylsulfanyl)-Pyrrolidin-1-yl]-Propionylamino}ethoxy)ethoxy)acetyl]-[D-Ala⁸,Lys³⁷]-GLP-1-[7-37]amid, 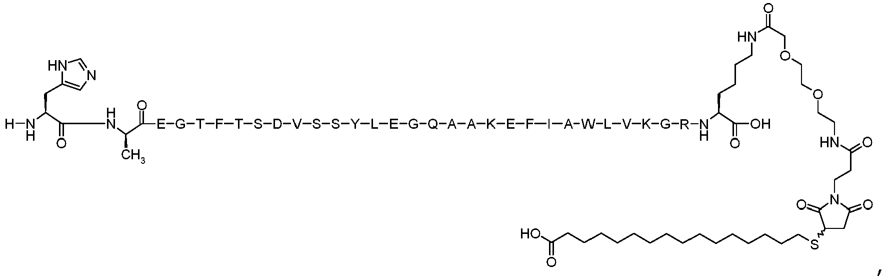
[Aib^{8,22,35}Ala³⁷]GLP-1(7-37)Lys((2-(2-(2-(11-(Oxalylamino)undecanoylamino)ethoxy)ethoxy)acetyl-)))amid, 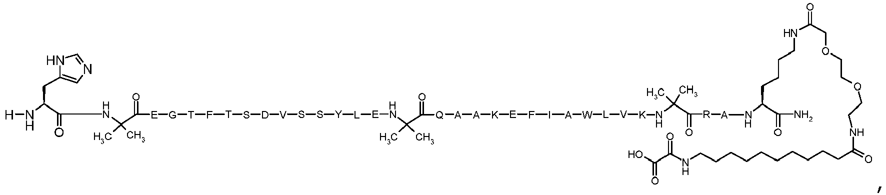
[Aib^{8,22,35},Ala³⁷]-GLP-1(7-37)Lys({2-[2-(2-{2-[2-(2-(15-Carboxy-Pentadecanoylamino)-Ethoxy]ethoxy}acetylamino)ethoxy]ethoxy}acetyl)amid, 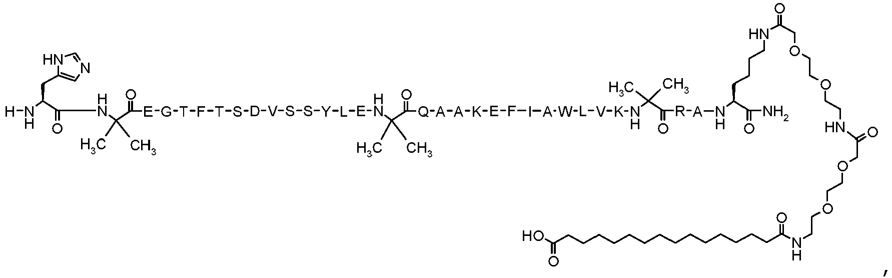
[Aib⁸,Arg^{26,34}] GLP-1 (7-37)Lys{2-(2-(2-(2-[2-(2-(17-Carboxyheptanoylamino)ethoxy)ethoxy]acetylamino)ethoxy)ethoxy)acetyl)}-OH, 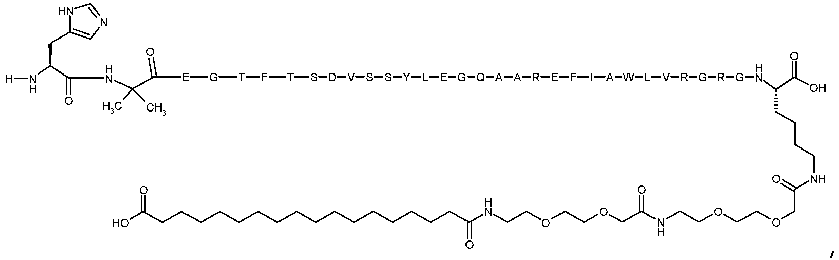
[Gly⁸, Arg^{26,34}] GLP1-(7-37) Lys{2-(2-(2-(2-[2-(2-(17-Carboxyheptadecanoylamino)ethoxy)ethoxy]acetyl)ethoxy)ethoxy)acetyl)}-OH, 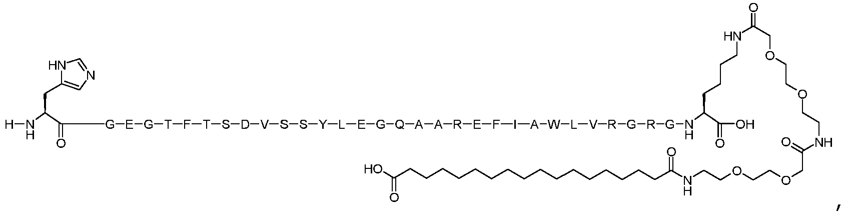 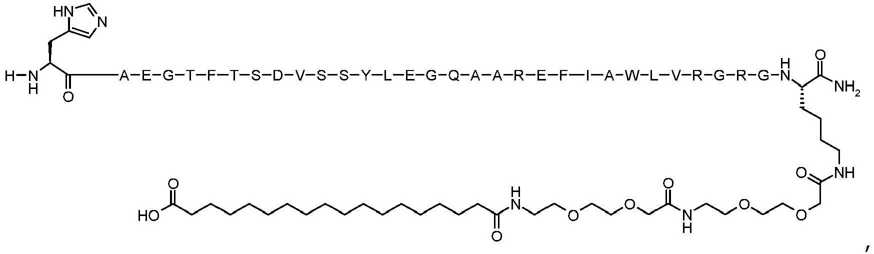
[Gly⁸,Arg^{26,34}]GLP-1(7-37)Lys(2-(2-(19-(Carboxy)nonadecanoylamino)ethoxy)ethoxy)acetyl)-OH, 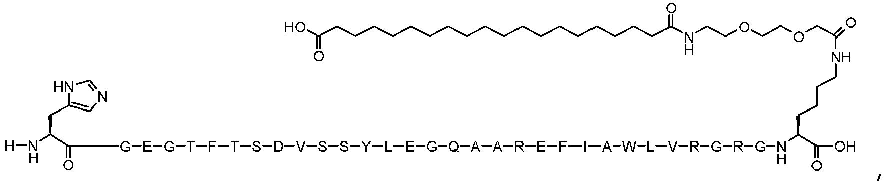
[Gly⁸,Arg²⁶,³⁴]GLP-1(7-37)Lys((2-(2-(17-(Carboxy)heptadecanoylamino)ethoxy)ethoxy)acetyl))-OH, 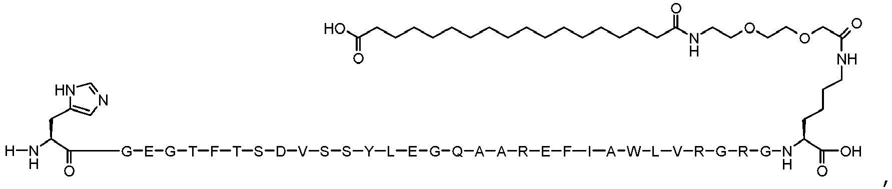
[Gly⁸,Arg^{26,34}]GLP-1(7-37)Lys(2-(2-(2-(4-(19-(Carboxy)nonadecanoylamino)-4-Carboxybutyrylamino)ethoxy)ethoxy)acetyl)-OH, 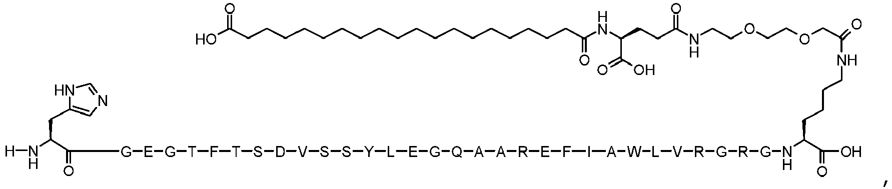 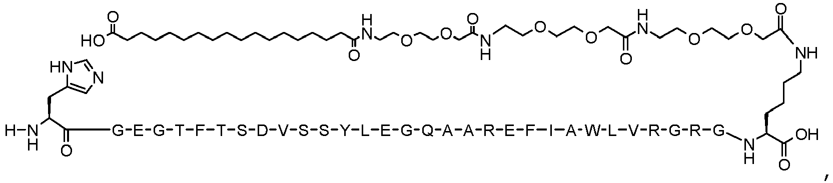
N^{ε20}(2-(2-(2-(2-(2-(2-(2-(2-(2-(2-(2-(2-(4-(17-(Carboxy)heptadecanoylamino)-4-Carboxybutyrylamino)ethoxy)ethoxy)acetylamino)ethoxy)ethoxy)acetylamino) ethoxy)ethoxy)acetylamino)ethoxy)ethoxy)acetyl) [Lys²⁰]exendin-4 (1-39)-NH₂, 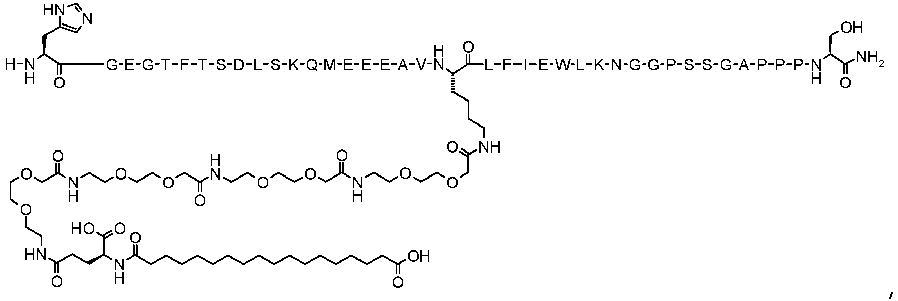
N^{ε36}-(2-(2-(2-(2-(2-(2-(17-Carboxyheptadecanoylamino)ethoxy)ethoxy) acetylamino)ethoxy)ethoxy)acetyl) [Aib⁸,Arg^{26,34}, Lys³⁶] GLP-1 (7-37), 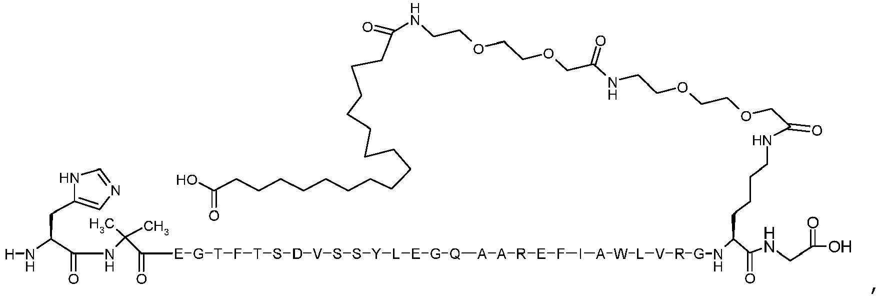
N-^{ε36}-(2-(2-(2-(2-(2-(2-(17-Carboxyheptadecanoylamino)ethoxy)ethoxy) acetylamino)ethoxy)ethoxy)acetyl) [Arg^{26,34}, Lys³⁶] GLP-1 (7-37), 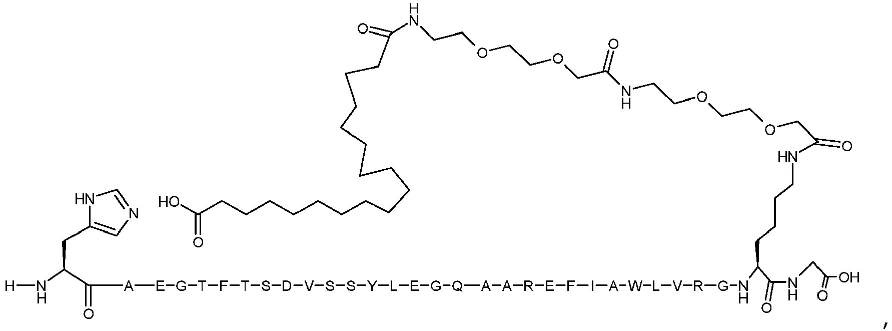
17-Carboxyheptadecanoylamino)ethoxy)ethoxy) acetylamino)ethoxy)ethoxy)acetyl) [Gly⁸,Arg^{26,34},Lys³⁶] GLP-1 (7-37), 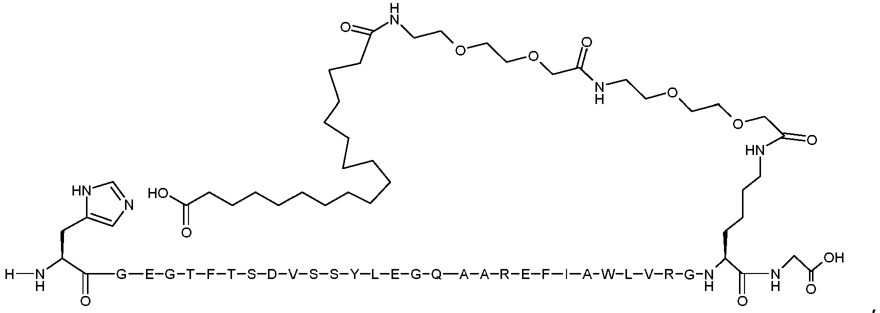
N^{ε26}-(2-[2-(2-[2-(2-[2-(17-Carboxyheptadecanoylamino)ethoxy] ethoxy)acetylamino]ethoxy)ethoxy]acetyl)[Arg³⁴]GLP-1-(7-37)-OH, 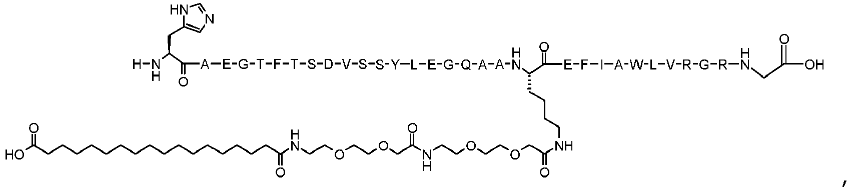
N^{ε26}-[2-(2-[2-(2-[2-(2-[4-(17-Carboxyheptadecanoylamino)-4(S)-Carboxybutyrylamino]ethoxy)ethoxy]acetylamino)ethoxy]ethoxy)acetyl][Arg³⁴]GLP-1-(7-37)-OH, 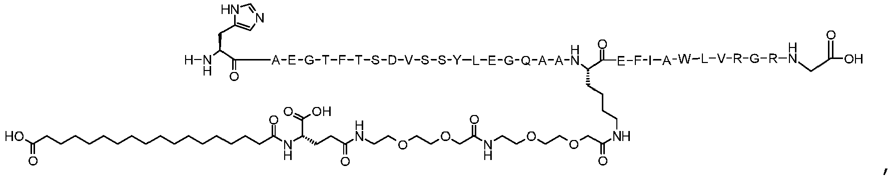
N^{ε20}-(2-(2-(2-(2-(2-(2-(2-(2-(2-(17-Carboxyheptadecanoylamino)ethoxy)ethoxy)acetylamino)ethoxy)ethoxy)acetylamino)ethoxy)ethoxy)acetyl)[Lys²⁰] Exendin-4 (1-39)amid, 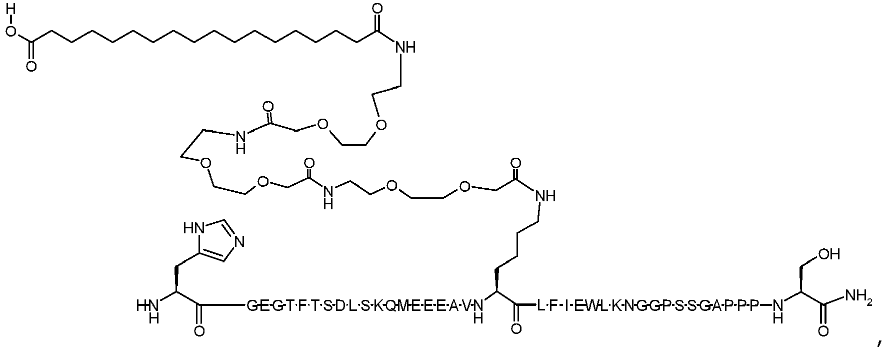
[Gly⁸, Glu^{22,23,30}, Arg^{18,26,34}]GLP1 (7-37) Lys(2-(2-(2-(2-(2-(2-(17-Carboxyheptadecanoylamino)ethoxy)ethoxy)acetylamino)ethoxy))ethoxy)acetyl)-NH₂, 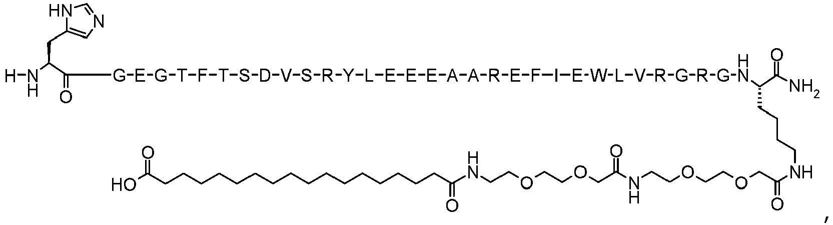
[Imidazolylpropionsäure⁷, Asp¹⁶, Aib^{22,35} ]GLP1(7-37)Lys NH((2-{[4-(17-Carboxyheptadecanoylamino)butylcarbamoyl]methoxy}ethoxy)ethoxy)), 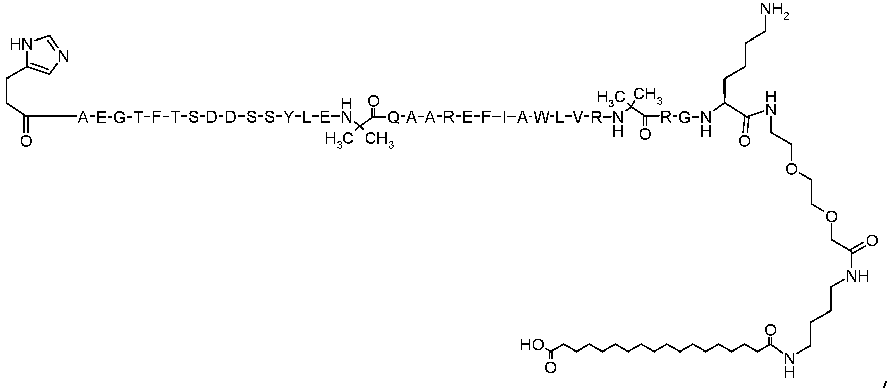
[Imidazolylpropionsure⁷, Aib^{22,35} ]GLP1(7-37)Lys NH( (2-{[4-(17-Carboxyheptadecanoylamino)butylcarbamoyl]methoxy}ethoxy)ethoxy)), 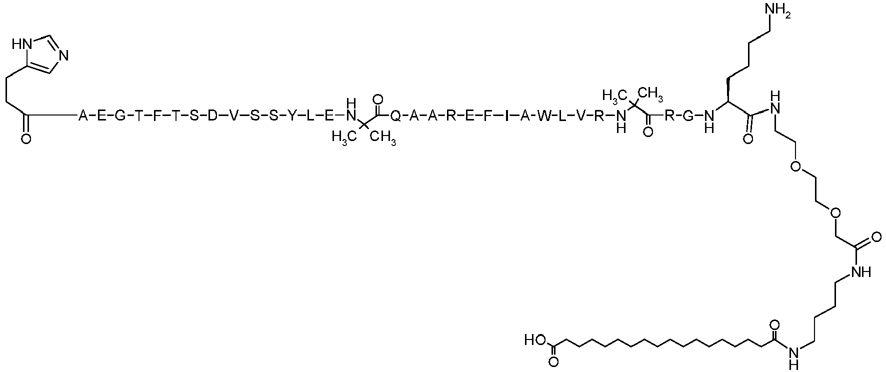 und
[3-(5-Imidazoyl)propionyl⁷, Aib⁸, Arg^{26,34} ] GLP-1 (7-37)Lys{2-(2-(2-(2-[2-(2-(17-Carboxyheptanoylamino)ethoxy)ethoxy]acetylamino)ethoxy)ethoxy)acetyl)}-OH,

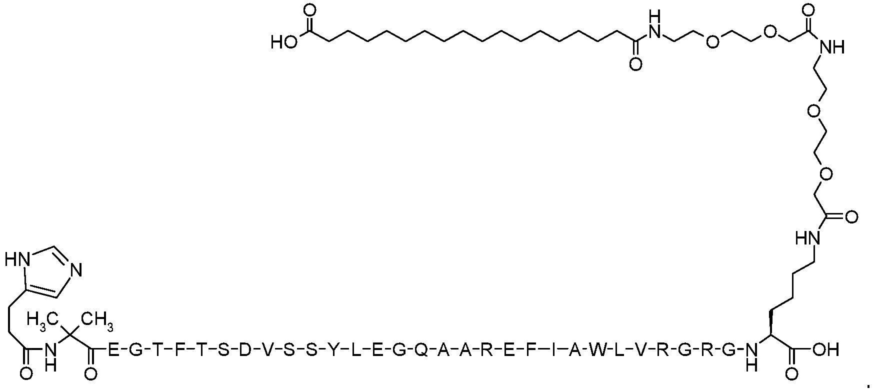

13. Pharmazeutische Zusammensetzung, eine Verbindung nach einem der Ansprüche 1-12 und einen pharmazeutisch unbedenklichen Hilfsstoff umfassend.

14. Verbindung nach einem der Ansprüche 1-12 zur Verwendung als Medikament.

15. Verwendung einer Verbindung nach einem der Ansprüche 1-12 zur Herstellung eines Arzneimittels; bevorzugt i) zur Behandlung oder Vorbeugung von Hyperglykämie, Typ-2-Diabetes, gestörter Glukosetoleranz, Typ-1-Diabetes, Fettleibigkeit, Bluthochdruck, Syndrom X, Dyslipidämie, kognitiven Störungen, Atherosklerose, Myokardinfarkt, koronarer Herzkrankheit und anderen kardiovaskulären Störungen, Schlaganfall, entzündlichem Darmsyndrom, Dyspepsie und Magengeschwüren; ii) zur Herstellung eines Medikaments zur Verzögerung oder Verhinderung des Fortschreitens der Krankheit bei Typ-2-Diabetes; und/oder iii) zur Herstellung eines Medikaments zur Verringerung der Nahrungsaufnahme, zur Verringerung der β-Zell-Apoptose, zur Erhöhung der β-Zell-Funktion und der β-Zell-Masse und/oder zur Wiederherstellung der Glukoseempfindlichkeit der β-Zellen.

## Revendications

1. Un composé qui a la formule (I) :
A-W-B-Y- polypeptide thérapeutique (I)
dans lequel
ledit polypeptide est un peptide GLP-1 comprenant la séquence d'acides aminés de formule (V) :
Xaa₇-Xaa₈-Glu-Gly-Thr-Phe-Thr-Ser-Asp-Val-Ser-Xaa₁₈-Tyr-Leu-Glu-Xaa₂₂-Xaa₂₃-Ala-Ala-Xaa₂₆-Glu-Phe-Ile-Xaa₃₀-Trp-Leu-Val-Xaa₃₄-Xaa₃₅-Xaa₃₆-Xaa₃₇-Xaa₃₈
Formule (V) (SEQ ID N° : 3)
dans lequel
Xaa₇ est de la L-histidine, de la D-histidine, de la désamino-histidine, de la 2-amino-histidine, de la β-hydroxy-histidine, de l'homohistidine, de la N°-acétyl-histidine, de l'α-fluorométhyl-histidine, de l'α-méthyl-histidine, de la 3-pyridylalanine, de la 2-pyridylalanine ou de la 4-pyridylalanine ;
Xaa₈ est Ala, Gly, Val, Leu, Ile, Lys, Aib, l'acide (1-aminocyclopropyl) carboxylique, l'acide (1-aminocyclobutyl) carboxylique, l'acide (1-aminocyclopentyl) carboxylique, l'acide (1-aminocyclohexyl) carboxylique, l'acide (1-aminocycloheptyl) carboxylique ou l'acide (1-aminocyclooctyl) carboxylique ;
Xaa₁₈ est Ser, Lys ou Arg ;
Xaa22 est Gly, Glu ou Aib ;
Xaa23 est Gln, Glu, Lys ou Arg ;
Xaa26 est Lys, Glu ou Arg ;
Xaa₃₀ est Ala, Glu ou Arg ;
Xaa34 est Lys, Glu ou Arg ;
Xaa35 est Gly ou Aib ;
Xaa36 est Arg ou Lys ;
Xaa37 est Gly, Ala, Glu ou Lys ;
Xaa38 est Lys, un amide ou est absent ;
A est un résidu de liaison à l'albumine choisi dans le groupe constitué de 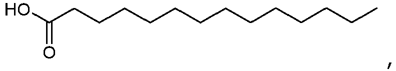 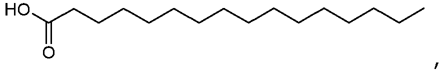 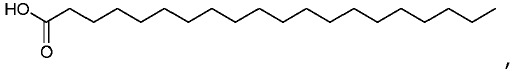 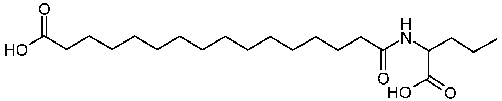 où l'atome de carbone chiral est soit R soit S, 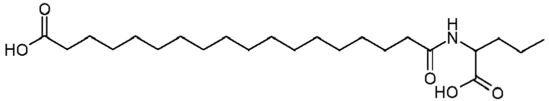 où l'atome de carbone chiral est soit R soit S, 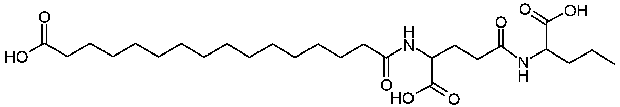 où les deux atomes de carbone chiraux sont indépendamment soit R soit S, 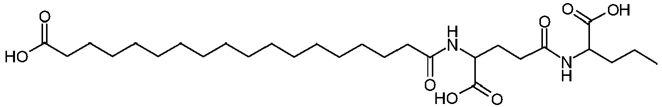 où les deux atomes de carbone chiraux sont indépendamment soit R soit S, 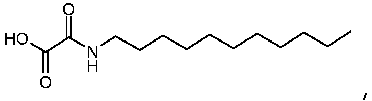 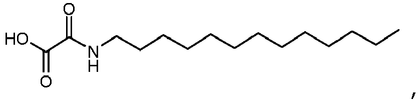 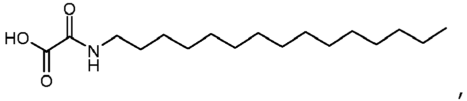 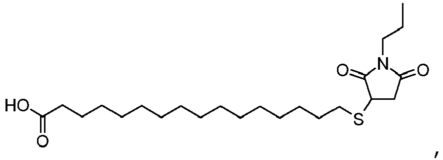 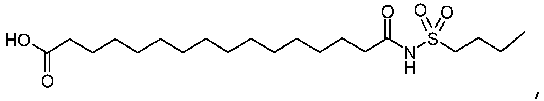 et 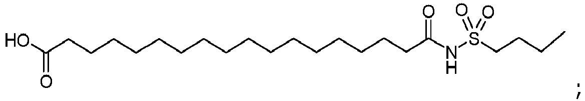
B est -(CH₂)ₗ-O-[(CH₂)ₙ-O]ₘ-(CH₂)ₚ-[C(O)NH-(CH₂)ₗ-O-[(CH₂)ₙ-O]ₘ-(CH₂)ₚ]_{q}-, où l, m, n et p sont indépendamment 1-5, et q est 0-5 ;
y est un groupe chimique liant B et l'agent thérapeutique, choisi dans le groupe constitué de -C(O)NH-, -NHC(O)-, -C(O)NHCH₂-, -CH₂NHC(O)-, -OC(O)NH-, -NHC(O)O-, -C(O)NHCH₂-, CH₂NHC(O)-, -C(O)CH₂-, -CH₂C(O)-, -C(O)CH=CH-, -CH=CHC(O)-, -(CH₂)ₛ-, -C(O)-, -C(O)O-, -OC(O)-, -NHC(O)- et -C(O)NH-, dans lequel s est 0 ou 1 ;
et
W est un groupe chimique liant A et B, choisi dans le groupe constitué de -C(O)NH-, - NHC(O)-, -C(O)NHCH₂-, -CH₂NHC(O)-, -OC(O)NH -, -NHC(O)O-, -C(O)CH₂-, -CH₂C(O)-, -C(O)CH=CH-, -CH=CHC(O)-, -(CH₂)ₛ-, -C(O)-, -C(O)O-, -OC(O)-, -NHC(O)- et -C(O)NH-, dans lequel s est 0 ou 1 ;
ou un sel pharmaceutiquement acceptable de ceux-ci.

2. Un composé selon la revendication 1, dans lequel i) q est 0 ou 1 ; ii) q est 1 ; ou iii) q est 0.

3. Un composé selon l'une quelconque des revendications précédentes, dans lequel l est 2 ; et/ou n est 2.

4. Un composé selon l'une quelconque des revendications précédentes, dans lequel -W-B-Y-est choisi dans le groupe constitué de 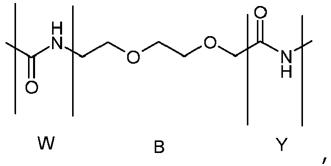 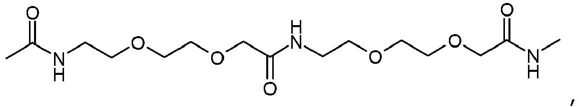 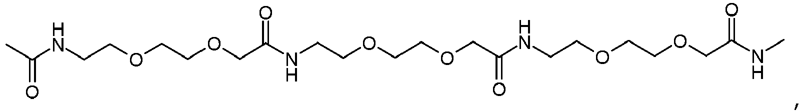 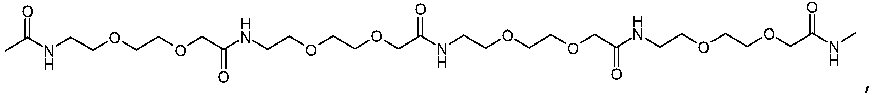 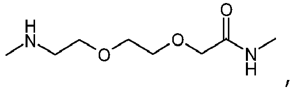 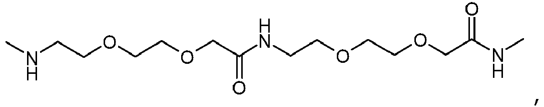 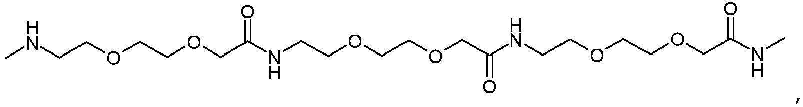 et

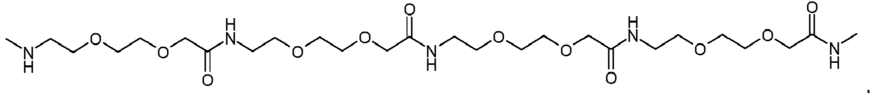

5. Un composé selon l'une quelconque des revendications précédentes, dans lequel le résidu de liaison à l'albumine i) par l'intermédiaire d'un espaceur et de lieurs est fixé audit polypeptide thérapeutique par l'intermédiaire du groupe ε-amino d'un résidu de lysine ; et/ou ii) par l'intermédiaire d'un lieur à un résidu d'acide aminé choisi parmi la cystéine, le glutamate et l'aspartate.

6. Un composé selon l'une quelconque des revendications précédentes, dans lequel ledit peptide GLP-1 comprend i) pas plus de dix résidus d'acides aminés qui ont été échangés, ajoutés ou supprimés par rapport à GLP-1(7-37) (SEQ ID N° 1) ; ii) de préférence pas plus de six résidus d'acides aminés qui n'ont pas été échangés, ajoutés ou supprimés par rapport à GLP-1(7-37) (SEQ ID N° 1) ; et/ou iii) pas plus de 4 résidus d'acides aminés qui ne sont pas codés par le code génétique.

7. Un composé selon l'une quelconque des revendications précédentes, dans lequel i) ledit peptide GLP-1 comprend un résidu Aib en position 8 ; et/ou ii) dans lequel le résidu d'acide aminé en position 7 dudit peptide GLP-1 est choisi dans le groupe constitué de la D-histidine, de la désamino-histidine, de la 2-amino-histidine, de la β-hydroxy-histidine, de l'homohistidine, de la N ^{α}-acétyl-histidine, de l'α-fluorométhyl-histidine, de l'α-méthyl-histidine, de la 3-pyridylalanine, de la 2-pyridylalanine et de la 4-pyridylalanine.

8. Un composé selon l'une quelconque des revendications précédentes, dans lequel ledit peptide GLP-1 est choisi dans le groupe constitué de Arg³⁴GLP-1(7-37),
Lys³⁸Arg^{26,34}GLP-1(7-38), Lys³⁸Arg^{26,34}GLP-1(7-38)-OH, Lys³⁶Arg^{26,34}GLP-1(7-36), Aib^{8,22,35}GLP-1(7-37), Aib^{8,35}GLP-1(7-37), Aib^{8,22}GLP-1(7-37), Aib^{8,22,35}Arg^{26,34}Lys³⁸GLP-1(7-38), Aib^{8,35}Arg^{26,34}Lys³⁸GLP-1(7-38), Aib^{8,22}Arg^{26,34}Lys³⁸GLP-1(7-38), Aib^{8,22,35}Arg^{26,34}Lys³⁸GLP-1(7-38), Aib^{8,35}Arg^{26,34}Lys³⁸GLP-1(7-38), Aib^{8,22,35}Arg²⁶Lys³⁸GLP-1(7-38), Aib^{8,35}Arg²⁶Lys³⁸GLP-1(7-38), Aib^{8,22}Arg²⁶Lys³⁸GLP-1(7-38), Aib^{8,22,35}Arg³⁴Lys³⁸GLP-1(7-38), Aib^{8,35}Arg³⁴Lys³⁸GLP-1(7-38), Aib^{8,22}Arg³⁴Lys³⁸GLP-1(7-38), Aib^{8,22,35}Ala³⁷Lys³⁸GLP-1(7-38), Aib^{8,35}Ala³⁷Lys³⁸GLP-1(7-38), Aib^{8,22}Ala³⁷Lys³⁸GLP-1(7-38), Aib^{8,22,35}Lys³⁷GLP-1(7-37), Aib^{8,35}Lys³⁷GLP-1(7-37) et Aib^{8,22}Lys³⁷GLP-1(7-38).

9. Un composé selon l'une quelconque des revendications précédentes, dans lequel ledit peptide GLP-1 est fixé audit espaceur hydrophile par l'intermédiaire du résidu d'acide aminé en position 23, 26, 34, 36 ou 38 par rapport à la séquence d'acides aminés SEQ ID N° 1.

10. Un composé selon l'une quelconque des revendications précédentes, dans lequel un résidu de liaison à l'albumine par l'intermédiaire dudit espaceur hydrophile est fixé au résidu d'acide aminé C-terminal dudit peptide GLP-1.

11. Un composé selon la revendication 10, dans lequel un second résidu de liaison à l'albumine est fixé à un résidu d'acide aminé qui n'est pas le résidu d'acide aminé C-terminal.

12. Un composé selon l'une quelconque des revendications précédentes, dans lequel ledit composé est choisi dans le groupe constitué de
N^{ε37}-{2-[2-(2-(15-carboxypentadécanoylamino)éthoxy)éthoxy]acétyl}-[Aib^{8,22,35},Lys³⁷] GLP-1(7-37)amide 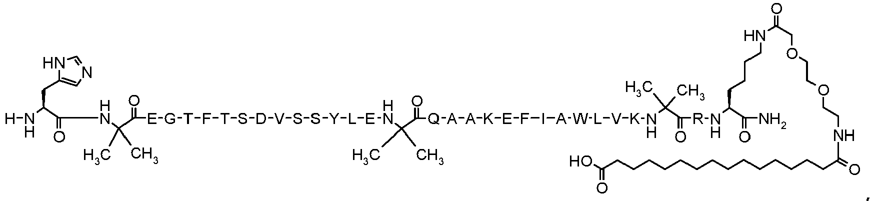
N^{ε37}-(2-(2-(2-(17-carboxyheptadécanoylamino)éthoxy)éthoxy)acétyl)[Aib^{8,22,35},Lys³⁷]GLP-1(7-37)amide, 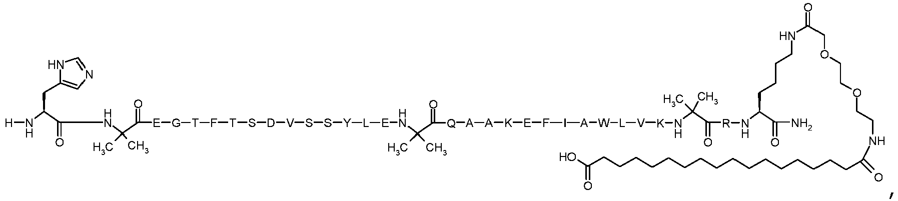
N^{ε37}-(2-(2-(2-(19-carboxynonadécanoylamino)éthoxy)éthoxy)acétyl)[Aib^{8,22,35},Lys³⁷]GLP-1(7-37)amide, 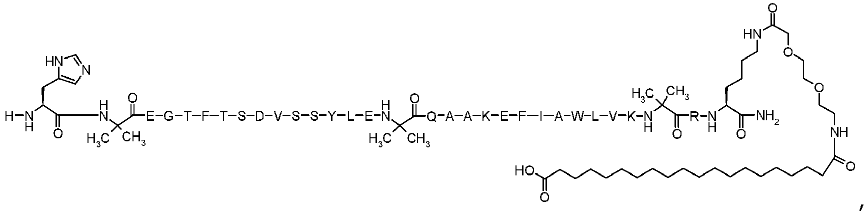
[Aib^{8,22,35}]GLP-1(7-37)Lys(({2-[2-(2-{2-[2-(2-{2-[2-(15-carboxypentadécanoylamino)-éthoxy]éthoxy}acétylamino)éthoxy]éthoxy}acétylamino)éthoxy]éthoxy}acétyl))amide, 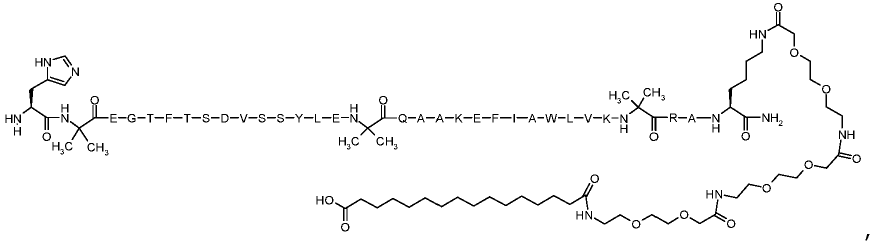
N^{ε37}-([2-(2-{3-[2,5-dioxo-3-(15-carboxypentadécylsulfanyl)-pyrrolidin-1-yl]-propionylamino}éthoxy)éthoxy)acétyl]-[D-Ala⁸,Lys³⁷]-GLP-1-[7-37]amide, 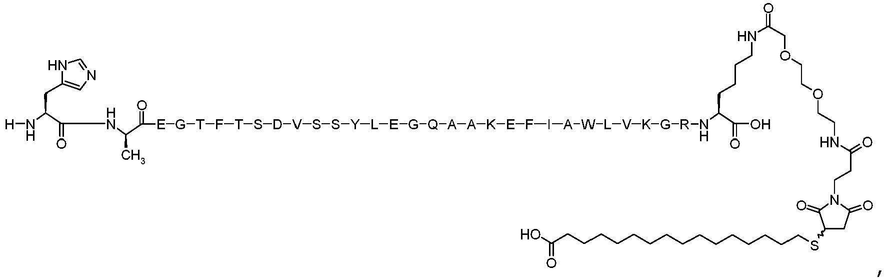
[Aib^{8,22,35}Ala³⁷]GLP-1(7-37)Lys((2-(2-(2-(11-(oxalylamino)undécanoylamino)éthoxy)éthoxy)acétyl-)))amide, 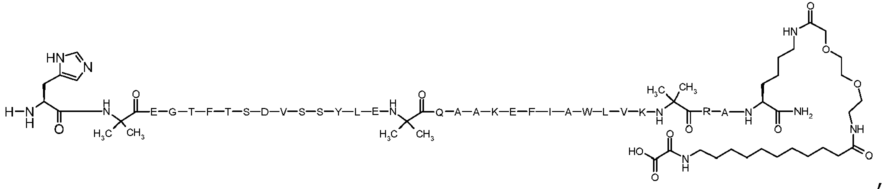
[Aib^{8,22,35}, Ala³⁷]-GLP-1(7-37)Lys({2-[2-(2-{2-[2-(2-(15-carboxy-pentadécanoylamino)-éthoxy]éthoxy}acétylamino)éthoxy]éthoxy}acétyl)amide, 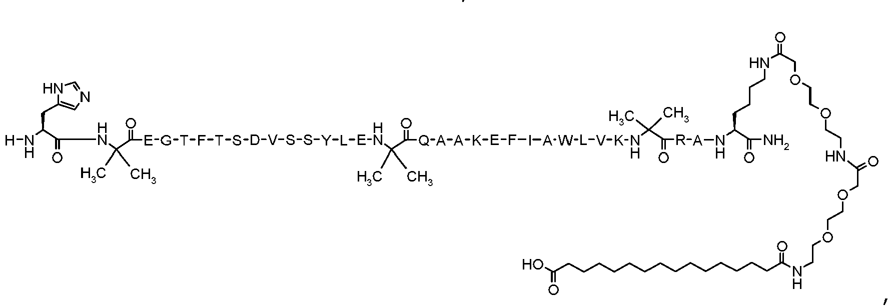
[Aib⁸, Arg^{26,34}] GLP-1 (7-37)Lys{2-(2-(2-(2-[2-(2-(17-carboxyheptanoylamino)éthoxy)éthoxy]acétylamino)éthoxy)éthoxy)acétyl)}-OH, 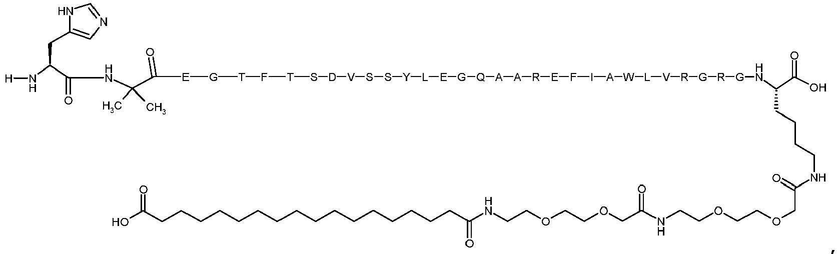
[Gly⁸, Arg^{26,34}] GLP1-(7-37) Lys{2-(2-(2-(2-[2-(2-(17-carboxyheptadécanoylamino)éthoxy)éthoxy]acétyl)éthoxy)éthoxy)acétyl)}-OH, 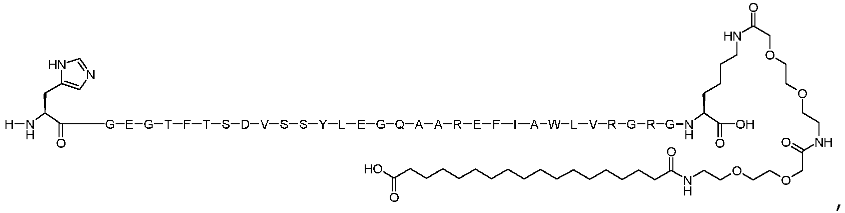 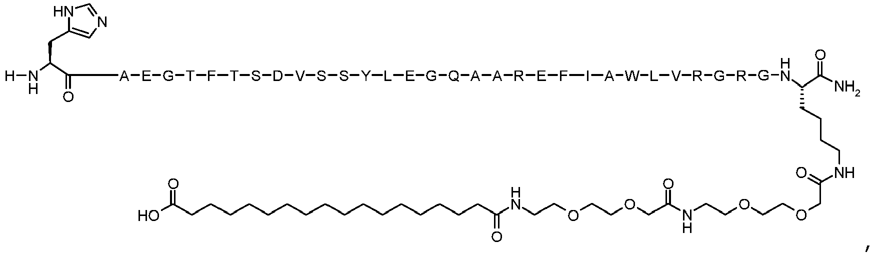
[Gly⁸, Arg^{26,34}]GLP-1(7-37)Lys(2-(2-(19-(carboxy)nonadécanoylamino)éthoxy)éthoxy)acétyl)-OH, 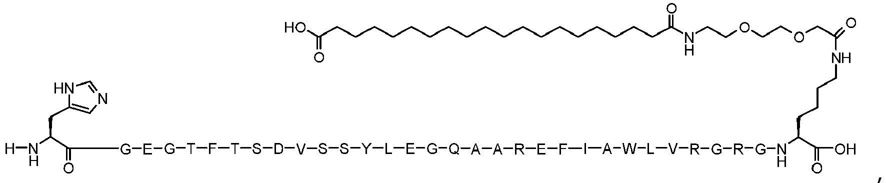
[Gly⁸, Arg^{26,34}]GLP-1(7-37)Lys((2-(2-(17-(carboxy)heptadécanoylamino)éthoxy)éthoxy)acétyl))-OH, 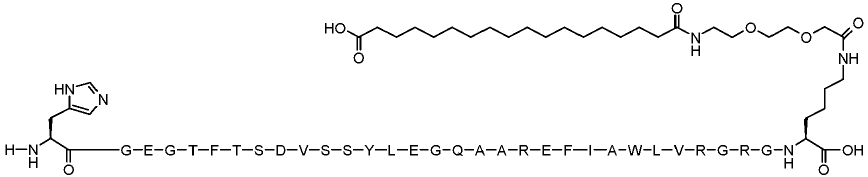
[Gly⁸,Arg^{26,34}]GLP-1(7-37)Lys(2-(2-(2-(4-(19-(carboxy)nonadécanoylamino)-4-carboxybutyrylamino)éthoxy)éthoxy)acétyl)-OH, 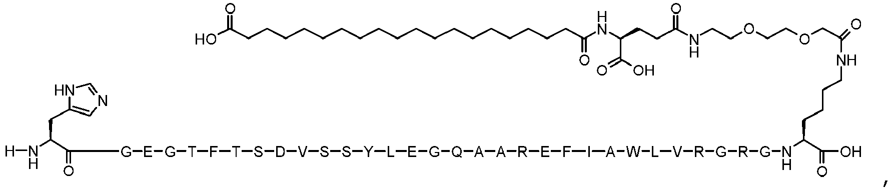 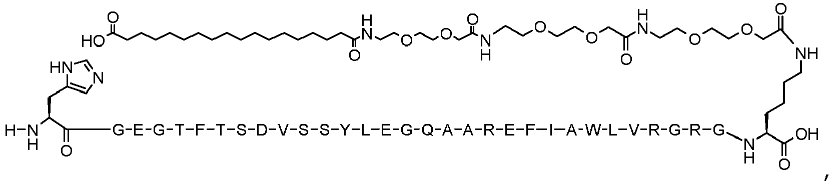
N^{ε20}(2-(2-(2-(2-(2-(2-(2-(2-(2-(2-(2-(2-(4-(17-(carboxy)heptadécanoylamino)-4-carboxybutyrylamino)éthoxy)éthoxy)acétylamino)éthoxy)éthoxy)acétylamino) éthoxy)éthoxy)acétylamino)éthoxy)éthoxy)acétyl) [Lys²⁰]exendin-4 (1-39)-NH₂, 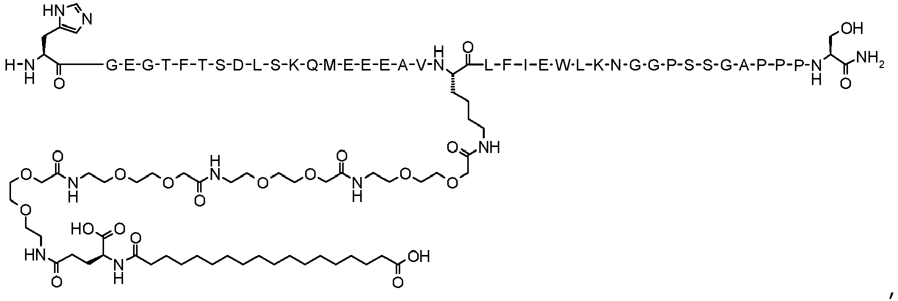
N^{ε36}-(2-(2-(2-(2-(2-(2-(17-Carboxyheptadécanoylamino)éthoxy)éthoxy) acétylamino)éthoxy)éthoxy)acétyl) [Aib⁸,Arg^{26,34},Lys³⁶] GLP-1 (7-37), 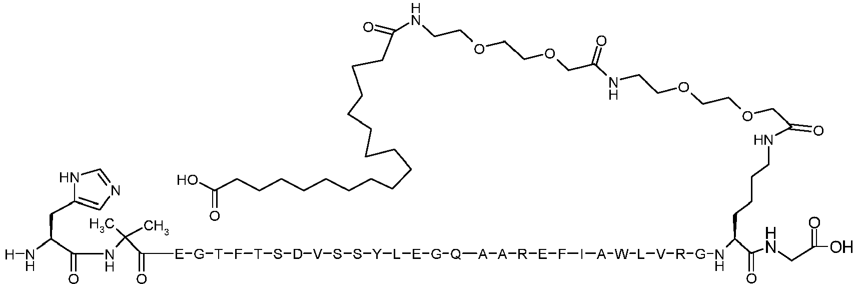
N-^{ε36}-(2-(2-(2-(2-(2-(2-(17-Carboxyheptadécanoylamino)éthoxy)éthoxy) acétylamino)éthoxy)éthoxy)acétyl) [Arg^{26,34}, Lys³⁶] GLP-1 (7-37), 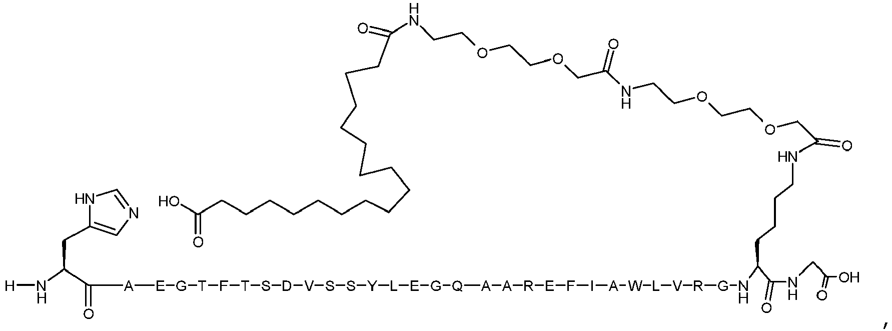
N^{ε36}-(2-(2-(2-(2-(2-(2-(17-Carboxyheptadécanoylamino)éthoxy)éthoxy) acétylamino)éthoxy)éthoxy)acétyl) [Gly⁸,Arg^{26,34},Lys³⁶] GLP-1 (7-37), 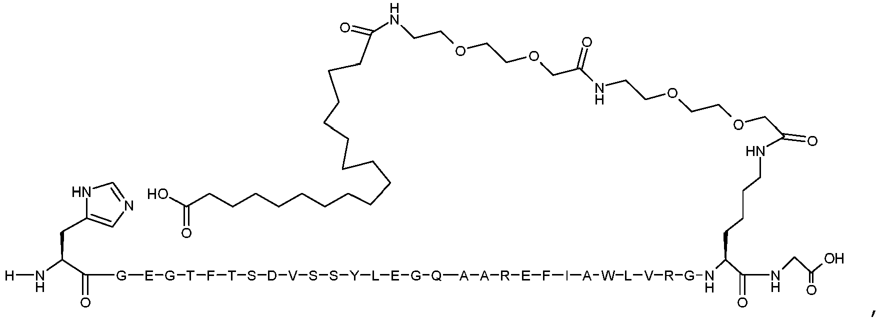
N^{ε26-}(2-[2-(2-[2-(2-[2-(17-Carboxyheptadécanoylamino)éthoxy] éthoxy)acétylamino]éthoxy)éthoxy]acétyl)[Arg³⁴]GLP-1-(7-37)-OH, 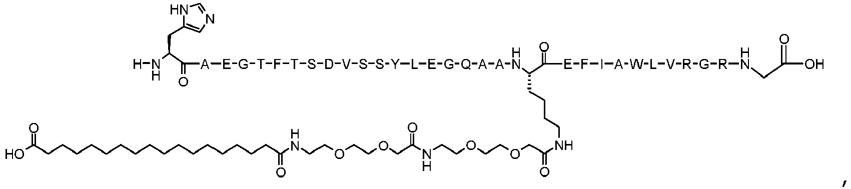
N^{ε26}-[2-(2-[2-(2-[2-(2-[4-(17-Carboxyheptadécanoylamino)-4(S)-carboxybutyrylamino]éthoxy)éthoxy]acétylamino)éthoxy]éthoxy)acétyl][Arg³⁴]GLP-1-(7-37)-OH, 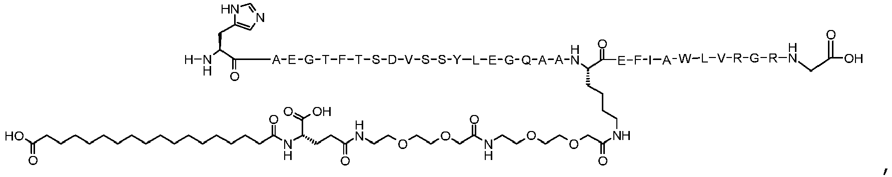
N^{ε20}-(2-(2-(2-(2-(2-(2-(2-(2-(2-(17-Carboxyheptadécanoylamino)éthoxy)éthoxy)acétylamino)éthoxy)éthoxy)acétyl-amino)éthoxy)éthoxy)acétyl)[Lys²⁰] Exendin-4 (1-39) amide, 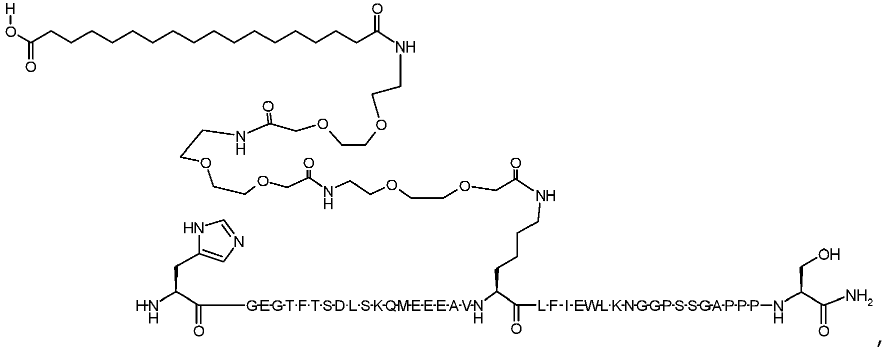
[Gly⁸, Glu^{22,23,30}, Arg^{18,26,34}]GLP1 (7-37) Lys(2-(2-(2-(2-(2-(2-(17-carboxyheptadécanoylamino)éthoxy)éthoxy)acétylamino)éthoxy))éthoxy)acétyl)-NH₂, 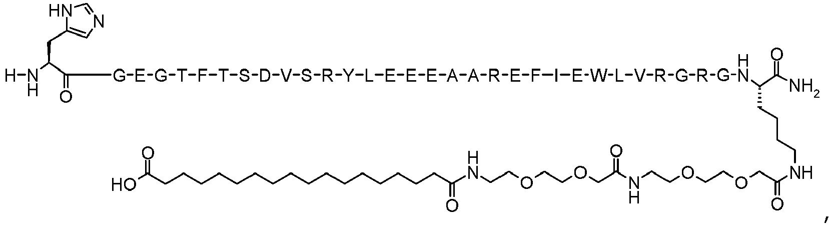
[Acide imidazolylpropionique⁷, Asp¹⁶, Aib^{22,35}]GLP1(7-37)Lys NH((2-{[4-(17-carboxyheptadécanoylamino)butylcarbamoyl]méthoxy}éthoxy)éthoxy)), 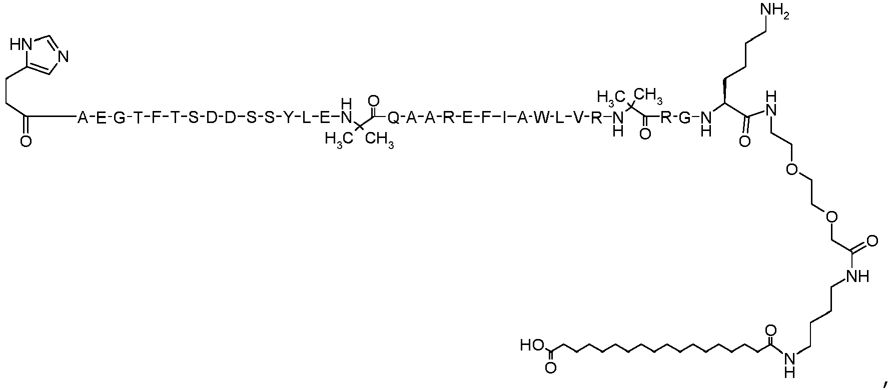
[Acide imidazolylpropionique⁷, Aib^{22,35} ]GLP1(7-37)Lys NH((2-{[4-(17-carboxyheptadécanoylamino)butylcarbamoyl]méthoxy}éthoxy)éthoxy)), 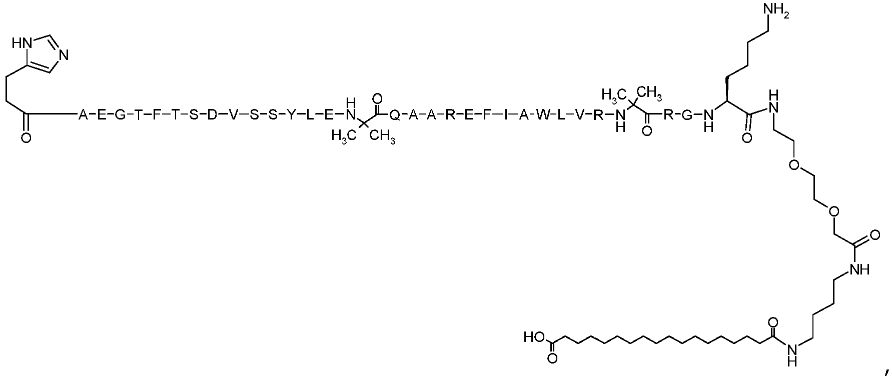 et
[3-(5-Imidazoyl)propionyl⁷, Aib⁸, Arg^{26,34}]GLP-1(7-37)Lys{2-(2-(2-(2-[2-(2-(17-carboxyheptanoylamino)éthoxy)éthoxy]acétylamino)éthoxy)éthoxy)acétyl)}-OH,

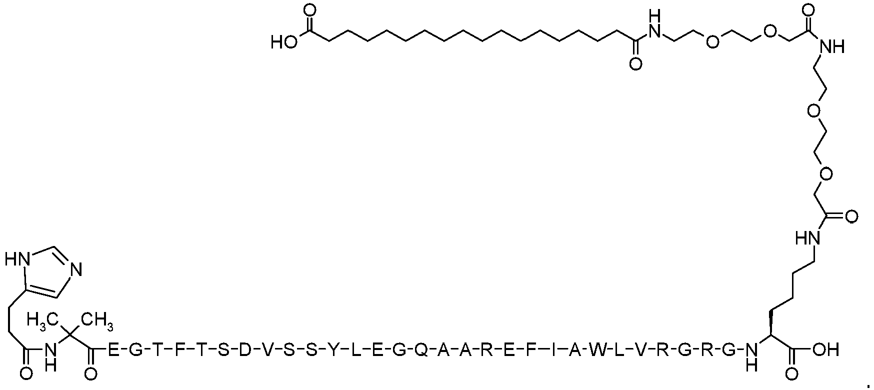

13. Une Composition pharmaceutique comprenant un composé selon l'une quelconque des revendications 1 à 12, et un excipient pharmaceutiquement acceptable.

14. Un composé selon l'une quelconque des revendications 1 à 12, pour utilisation en tant que médicament.

15. Utilisation d'un composé selon l'une quelconque des revendications 1 à 12 pour la préparation d'un médicament ; de préférence i) pour le traitement ou la prévention de l'hyperglycémie, du diabète de type 2, de l'intolérance au glucose, du diabète de type 1, de l'obésité, de l'hypertension, du syndrome X, de la dyslipidémie, de troubles cognitifs, de l'athérosclérose, de l'infarctus du myocarde, de la maladie des artères coronaires et d'autres troubles cardio-vasculaires, de l'accident vasculaire cérébral, du syndrome inflammatoire de l'intestin, de la dyspepsie et des ulcères gastriques ; ii) pour la préparation d'un médicament pour retarder ou prévenir la progression de la maladie dans le diabète de type 2 ; et/ou iii) pour la préparation d'un médicament pour diminuer la prise de nourriture, diminuer l'apoptose des cellules β, augmenter la fonction des cellules β et la masse des cellules β, et/ou pour restaurer la sensibilité du glucose aux cellules β.


### SEQUENCE LISTING

<110> Novo Nordisk A/S
<120> Novel GLP-1 derivatives
<130> 6692-WO
<160> 5
<170> Patentln version 3.1
<210> 1
   <211> 31
   <212> PRT
   <213> Homo sapiens
<400> 1
   His Ala Glu Gly Thr Phe Thr Ser Asp Val Ser Ser Tyr Leu Glu Gly
   1 5 10 15
   Gln Ala Ala Lys Glu Phe Ile Ala Trp Leu Val Lys Gly Arg Gly
   20 25 30
<210> 2
   <211> 40
   <212> PRT
   <213> Synthetic construct
<220>
   <221> MISC_FEATURE
   <222> (1)..(1)
   <223> Xaa at position 1 is L-histidine, D-histidine, desamino-histidine , 2-amino-histidine, beta-hydroxy-histidine, homohistidine, N-alp ha-acetyl-histidine, alpha-fluoromethyl-histidine, alpha-methyl-h istidine, 3-pyridylalanine, 2-pyridylalanine, or 4-pyridylalanine
<220>
   <221> MISC_FEATURE
   <222> (2)..(2)
   <223> Xaa at position 2 is Ala, Gly, Val, Leu, Ile, Lys, Aib, (1-aminoc yclopropyl) carboxylic acid, (1-aminocyclobutyl) carboxylic acid, (1-aminocyclopentyl) carboxylic acid, (1-aminocyclohexyl) carboxy lic acid, (1-aminocycloheptyl) carboxylic acid or (1-aminocyclooc tyl) carboxylic acid.
<220>
   <221> MISC_FEATURE
   <222> (10)..(10)
   <223> Xaa at position 10 is Val or Leu.
<220>
   <221> MISC_FEATURE
   <222> (12)..(12)
   <223> Xaa at position 12 is Ser, Lys or Arg.
<220>
   <221> MISC_FEATURE
   <222> (13)..(13)
   <223> Xaa at position 13 is Tyr or Gln.
<220>
   <221> MISC_FEATURE
   <222> (14)..(14)
   <223> Xaa at position 14 is Leu or Met.
<220>
   <221> MISC_FEATURE
   <222> (16)..(16)
   <223> Xaa at position 16 is Gly, Glu or Aib.
<220>
   <221> MISC_FEATURE
   <222> (17)..(17)
   <223> Xaa at position 17 is Gln, Glu, Lys or Arg.
<220>
   <221> MISC_FEATURE
   <222> (19)..(19)
   <223> Xaa at position 19 is Ala or Val.
<220>
   <221> MISC_FEATURE
   <222> (20)..(20)
   <223> Xaa at position 20 is Lys, Glu or Arg.
<220>
   <221> MISC_FEATURE
   <222> (21)..(21)
   <223> Xaa at position 21 is Glu or Leu.
<220>
   <221> MISC_FEATURE
   <222> (24)..(24)
   <223> Xaa at position 24 is Ala, Glu or Arg.
<220>
   <221> MISC_FEATURE
   <222> (27)..(27)
   <223> Xaa at position 27 is Val or Lys.
<220>
   <221> MISC_FEATURE
   <222> (28)..(28)
   <223> Xaa at position 28 is Lys, Glu, Asn or Arg.
<220>
   <221> MISC_FEATURE
   <222> (29)..(29)
   <223> Xaa at position 29 is Gly or Aib.
<220>
   <221> MISC_FEATURE
   <222> (30)..(30)
   <223> Xaa at position 30 is Arg, Gly or Lys.
<220>
   <221> MISC_FEATURE
   <222> (31)..(31)
   <223> Xaa at position 31 is Gly, Ala, Glu, Pro, Lys, amide or is absent
<220>
   <221> MISC_FEATURE
   <222> (32)..(32)
   <223> Xaa at position 32 is Lys, Ser, amide or is absent.
<220>
   <221> MISC_FEATURE
   <222> (33)..(33)
   <223> Xaa at position 33 is Ser, Lys, amide or is absent.
<220>
   <221> MISC_FEATURE
   <222> (34)..(34)
   <223> Xaa at position 34 is Gly, amide or is absent.
<220>
   <221> MISC_FEATURE
   <222> (35)..(35)
   <223> Xaa at position 35 is Ala, amide or is absent.
<220>
   <221> MISC_FEATURE
   <222> (36)..(36)
   <223> Xaa at position 36 is Pro, amide or is absent.
<220>
   <221> MISC_FEATURE
   <222> (37)..(37)
   <223> Xaa at position 37 is Pro, amide or is absent.
<220>
   <221> MISC_FEATURE
   <222> (38)..(38)
   <223> Xaa at position 38 is Pro, amide or is absent.
<220>
   <221> MISC_FEATURE
   <222> (39)..(39)
   <223> Xaa at position 39 is Ser, amide or is absent.
<220>
   <221> MISC_FEATURE
   <222> (40)..(40)
   <223> Xaa at position 40 is amide or is absent.
<400> 2
   Xaa Xaa Glu Gly Thr Phe Thr Ser Asp Xaa Ser Xaa Xaa Xaa Glu Xaa
   1 5 10 15
   Xaa Ala Xaa Xaa Xaa Phe Ile Xaa Trp Leu Xaa Xaa Xaa Xaa Xaa Xaa
   20 25 30
   Xaa Xaa Xaa Xaa Xaa Xaa Xaa Xaa
   35 40
<210> 3
   <211> 32
   <212> PRT
   <213> Synthetic construct
<220>
   <221> MISC_FEATURE
   <222> (1)..(1)
   <223> Xaa at position 1 is L-histidine, D-histidine, desamino-histidine , 2-amino-histidine, beta-hydroxy-histidine, homohistidine, N-alp ha-acetyl-histidine, alpha-fluoromethyl-histidine, alpha-methyl-h istidine, 3-pyridylalanine, 2-pyridylalanine, or 4-pyridylalanine
<220>
   <221> MISC_FEATURE
   <222> (2)..(2)
   <223> Xaa at position 2 is Ala, Gly, Val, Leu, Ile, Lys, Aib, (1-aminoc yclopropyl) carboxylic acid, (1-aminocyclobutyl) carboxylic acid, (1-aminocyclopentyl) carboxylic acid, (1-aminocyclohexyl) carboxy lic acid, (1-aminocycloheptyl) carboxylic acid or (1-aminocyclooc tyl) carboxylic acid.
<220>
   <221> MISC_FEATURE
   <222> (12)..(12)
   <223> Xaa at position 12 is Ser, Lys or Arg.
<220>
   <221> MISC_FEATURE
   <222> (16)..(16)
   <223> Xaa at position 16 is Gly, Glu or Aib.
<220>
   <221> MISC_FEATURE
   <222> (17)..(17)
   <223> Xaa at position 17 is Gln, Gly, Lys or Arg.
<220>
   <221> MISC_FEATURE
   <222> (20)..(20)
   <223> Xaa at position 20 is Lys, Glu or Arg.
<220>
   <221> MISC_FEATURE
   <222> (24)..(24)
   <223> Xaa at position 24 is Ala, Glu or Arg.
<220>
   <221> MISC_FEATURE
   <222> (28)..(28)
   <223> Xaa at position 28 is Lys, Glu or Arg.
<220>
   <221> MISC_FEATURE
   <222> (29)..(29)
   <223> Xaa at position 29 is Gly or Aib.
<220>
   <221> MISC_FEATURE
   <222> (30)..(30)
   <223> Xaa at position 30 is Arg or Lys..
<220>
   <221> MISC_FEATURE
   <222> (31)..(31)
   <223> Xaa at position 31 is Gly, Ala, Glu or Lys.
<220>
   <221> MISC_FEATURE
   <222> (32)..(32)
   <223> Xaa at position 32 is Lys, amide or is absent.
<400> 3
   Xaa Xaa Glu Gly Thr Phe Thr Ser Asp Val Ser Xaa Tyr Leu Glu Xaa
   1 5 10 15
   Xaa Ala Ala Xaa Glu Phe Ile Xaa Trp Leu Val Xaa Xaa Xaa Xaa Xaa
   20 25 30
<210> 4
   <211> 39
   <212> PRT
   <213> Gila monster
<220>
   <221> MISC_FEATURE
   <222> (39)..(39)
   <223> Amidation of carboxy group.
<400> 4
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 5
   <211> 44
   <212> PRT
   <213> Synthetic construct
<220>
   <221> MISC_FEATURE
   <222> (44)..(44)
   <223> Amidation of carboxy group.
<400> 5
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Ser Lys Lys Lys Lys Lys Lys
   35 40